# EUROPEAN PATENT SPECIFICATION

(11) **EP 2 354 155 B1**
(45) Date of publication and mention of the grant of the patent: **03.05.2017**
(21) Application number: 10184697.0
(22) Date of filing: 17.05.2004
(51) Int. Cl.: C07K 14/475, C12N 9/88, A61K 38/51

(54) **Fusion Proteins for Treatment of CNS**
Fusionsproteine zur Behandlung des ZNS
Protéines de fusion pour le traitement du SNC

(30) Priority: 16.05.2003 US 471236; 16.05.2003 US 471239; 16.05.2003 US 471240; 16.05.2003 US 471300; 29.05.2003 US 474372
(43) Date of publication of application: 10.08.2011
(62) Divisional of application: 04776038.4
(73) Proprietor: Acorda Therapeutics, Inc., Ardsley, NY 10502 (US)
(72) Inventor: Gruskin, Elliott A, Malvern, PA 19355 (US); Caggiano, Anthony O, Larchmont 10538 (US); Iaci, Jennifer, Montville 07005 (US); Zimber, Michael P, Encinitas, CA 92024 (US); Roy, Gargi, Boyds, MD 20841 (US)
(74) Representative: Fyfe, Fiona Allison Watson

(56) References cited:
- WO-A-00/62067
- WO-A-02/055684
- WO-A-02/083179
- WO-A-2004/017044
- WO-A1-00/64482
- WO-A1-95/13091
- WO-A1-03/074080
- ROY G ET AL: "Generation of substantially smaller deletion mutants of chondroitinases AC and B those are biologically active", SOCIETY FOR NEUROSCIENCE ABSTRACT VIEWER AND ITINERARY PLANNER, & 33RD ANNUAL MEETING OF THE SOCIETY OF NEUROSCIENCE; NEW ORLEANS, LA, USA; NOVEMBER 08-12, 2003, vol. 2003, 1 January 2003 (2003-01-01), XP002550406,
- BRADBURY E J ET AL: "Chondroitinase ABC promotes functional recovery after spinal cord injury", NATURE, NATURE PUBLISHING GROUP, LONDON, GB, vol. 416, 11 April 2002 (2002-04-11), pages 636-640, XP002245003, ISSN: 0028-0836
- SCHWARZE STEVEN R ET AL: "In vivo protein transduction: Delivery of a biologically active protein into the mouse", SCIENCE, AMERICAN ASSOCIATION FOR THE ADVANCEMENT OF SCIENCE, WASHINGTON, DC; US, vol. 285, no. 5433, 3 September 1999 (1999-09-03), pages 1569-1572, XP002537718, ISSN: 0036-8075, DOI: DOI:10.1126/SCIENCE.285.5433.1569
- DIMAYUGA FILOMENA O ET AL: "The neuregulin GGF2 attenuates free radical release from activated microglial cells.", JOURNAL OF NEUROIMMUNOLOGY, vol. 136, no. 1-2, March 2003 (2003-03), pages 67-74, XP002651543, ISSN: 0165-5728
- MCGEE A W ET AL: "The Nogo-66 receptor: focusing myelin inhibition of axon regeneration", TRENDS IN NEUROSCIENCE, ELSEVIER, AMSTERDAM, NL, vol. 26, no. 4, April 2003 (2003-04), pages 193-198, XP004418152, ISSN: 0166-2236
- GRANDPRE TADZIA ET AL: "Nogo-66 receptor antagonist peptide promotes axonal regeneration", NATURE (LONDON), vol. 417, no. 6888, 30 May 2002 (2002-05-30), pages 547-551, XP002651544, ISSN: 0028-0836
- HUNT D ET AL: "The Nogo receptor, its ligands and axonal regeneration in the spinal cord; A review.", JOURNAL OF NEUROCYTOLOGY, vol. 31, no. 2, February 2002 (2002-02), pages 93-120, XP002651545, ISSN: 0300-4864

### SEQUENCE LISTING

<110> Acorda Therapeutics, Inc.
<120> FUSION PROTEINS FOR TREATMENT OF CNS
<130> P43717.EP.02
<150> 60/471,239
   <151> 2003-05-16
<150> 60/471,300
   <151> 2003-05-16
<150> 60/471,236
   <151> 2003-05-16
<150> 60/474,372
   <151> 2003-05-29
<150> 60/471,240
   <151> 2003-05-16
<160> 57
<170> PatentIn version 3.2
<210> 1
   <211> 997
   <212> PRT
   <213> Artificial
<220>
   <223> Synthetic polypeptide, chondroitinase ABC I protein
<400> 1
   Ala Thr Ser Asn Pro Ala Phe Asp Pro Lys Asn Leu Met Gln Ser Glu
   1               5                   10                  15
   Ile Tyr His Phe Ala Gln Asn Asn Pro Leu Ala Asp Phe Ser Ser Asp
               20                  25                  30
   Lys Asn Ser Ile Leu Thr Leu Ser Asp Lys Arg Ser Ile Met Gly Asn
           35                  40                  45
   Gln Ser Leu Leu Trp Lys Trp Lys Gly Gly Ser Ser Phe Thr Leu His
       50                  55                  60
   Lys Lys Leu Ile Val Pro Thr Asp Lys Glu Ala Ser Lys Ala Trp Gly
   65                  70                  75                  80
   Arg Ser Ser Thr Pro Val Phe Ser Phe Trp Leu Tyr Asn Glu Lys Pro
                   85                  90                  95
   Ile Asp Gly Tyr Leu Thr Ile Asp Phe Gly Glu Lys Leu Ile Ser Thr
               100                 105                 110
   Ser Glu Ala Gln Ala Gly Phe Lys Val Lys Leu Asp Phe Thr Gly Trp
   115 120 125
   Arg Thr Val Gly Val Ser Leu Asn Asn Asp Leu Glu Asn Arg Glu Met
   130 135 140
   Thr Leu Asn Ala Thr Asn Thr Ser Ser Asp Gly Thr Gln Asp Ser Ile
   145 150 155 160
   Gly Arg Ser Leu Gly Ala Lys Val Asp Ser Ile Arg Phe Lys Ala Pro
   165 170 175
   Ser Asn Val Ser Gln Gly Glu Ile Tyr Ile Asp Arg Ile Met Phe Ser
   180 185 190
   Val Asp Asp Ala Arg Tyr Gln Trp Ser Asp Tyr Gln Val Lys Thr Arg
   195 200 205
   Leu Ser Glu Pro Glu Ile Gln Phe His Asn Val Lys Pro Gln Leu Pro
   210 215 220
   Val Thr Pro Glu Asn Leu Ala Ala Ile Asp Leu Ile Arg Gln Arg Leu
   225 230 235 240
   Ile Asn Glu Phe Val Gly Gly Glu Lys Glu Thr Asn Leu Ala Leu Glu
   245 250 255
   Glu Asn Ile Ser Lys Leu Lys Ser Asp Phe Asp Ala Leu Asn Thr His
   260 265 270
   Thr Leu Ala Asn Gly Gly Thr Gln Gly Arg His Leu Ile Thr Asp Lys
   275 280 285
   Gln Ile Ile Ile Tyr Gln Pro Glu Asn Leu Asn Ser Gln Asp Lys Gln
   290 295 300
   Leu Phe Asp Asn Tyr Val Ile Leu Gly Asn Tyr Thr Thr Leu Met Phe
   305 310 315 320
   Asn Ile Ser Arg Ala Tyr Val Leu Glu Lys Asp Pro Thr Gln Lys Ala
   325 330 335
   Gln Leu Lys Gln Met Tyr Leu Leu Met Thr Lys His Leu Leu Asp Gln
   340 345 350
   Gly Phe Val Lys Gly Ser Ala Leu Val Thr Thr His His Trp Gly Tyr
   355 360 365
   Ser Ser Arg Trp Trp Tyr Ile Ser Thr Leu Leu Met Ser Asp Ala Leu
   370 375 380
   Lys Glu Ala Asn Leu Gln Thr Gln Val Tyr Asp Ser Leu Leu Trp Tyr
   385 390 395 400
   Ser Arg Glu Phe Lys Ser Ser Phe Asp Met Lys Val Ser Ala Asp Ser
                   405                 410                 415
   Ser Asp Leu Asp Tyr Phe Asn Thr Leu Ser Arg Gln His Leu Ala Leu
               420                 425                 430
   Leu Leu Leu Glu Pro Asp Asp Gln Lys Arg Ile Asn Leu Val Asn Thr
           435                 440                 445
   Phe Ser His Tyr Ile Thr Gly Ala Leu Thr Gln Val Pro Pro Gly Gly
       450                 455                 460
   Lys Asp Gly Leu Arg Pro Asp Gly Thr Ala Trp Arg His Glu Gly Asn
   465                 470                 475                 480
   Tyr Pro Gly Tyr Ser Phe Pro Ala Phe Lys Asn Ala Ser Gln Leu Ile
                   485                 490                 495
   Tyr Leu Leu Arg Asp Thr Pro Phe Ser Val Gly Glu Ser Gly Trp Asn
               500                 505                 510
   Asn Leu Lys Lys Ala Met Val Ser Ala Trp Ile Tyr Ser Asn Pro Glu
           515                 520                 525
   Val Gly Leu Pro Leu Ala Gly Arg His Pro Phe Asn Ser Pro Ser Leu
       530                 535                 540
   Lys Ser Val Ala Gln Gly Tyr Tyr Trp Leu Ala Met Ser Ala Lys Ser
   545                 550                 555                 560
   Ser Pro Asp Lys Thr Leu Ala Ser Ile Tyr Leu Ala Ile Ser Asp Lys
                   565                 570                 575
   Thr Gln Asn Glu Ser Thr Ala Ile Phe Gly Glu Thr Ile Thr Pro Ala
               580                 585                 590
   Ser Leu Pro Gln Gly Phe Tyr Ala Phe Asn Gly Gly Ala Phe Gly Ile
           595                 600                 605
   His Arg Trp Gln Asp Lys Met Val Thr Leu Lys Ala Tyr Asn Thr Asn
       610                 615                 620
   Val Trp Ser Ser Glu Ile Tyr Asn Lys Asp Asn Arg Tyr Gly Arg Tyr
   625                 630                 635                 640
   Gln Ser His Gly Val Ala Gln Ile Val Ser Asn Gly Ser Gln Leu Ser
                   645                 650                 655
   Gln Gly Tyr Gln Gln Glu Gly Trp Asp Trp Asn Arg Met Glu Gly Ala
               660                 665                 670
   Thr Thr Ile His Leu Pro Leu Lys Asp Leu Asp Ser Pro Lys Pro His
           675                 680                 685
   Thr Leu Met Gln Arg Gly Glu Arg Gly Phe Ser Gly Thr Ser Ser Leu
       690                 695                 700
   Glu Gly Gln Tyr Gly Met Met Ala Phe Asn Leu Ile Tyr Pro Ala Asn
   705                 710                 715                 720
   Leu Glu Arg Phe Asp Pro Asn Phe Thr Ala Lys Lys Ser Val Leu Ala
                   725                 730                 735
   Ala Asp Asn His Leu Ile Phe Ile Gly Ser Asn Ile Asn Ser Ser Asp
               740                 745                 750
   Lys Asn Lys Asn Val Glu Thr Thr Leu Phe Gln His Ala Ile Thr Pro
           755                 760                 765
   Thr Leu Asn Thr Leu Trp Ile Asn Gly Gln Lys Ile Glu Asn Met Pro
       770                 775                 780
   Tyr Gln Thr Thr Leu Gln Gln Gly Asp Trp Leu Ile Asp Ser Asn Gly
   785                 790                 795                 800
   Asn Gly Tyr Leu Ile Thr Gln Ala Glu Lys Val Asn Val Ser Arg Gln
                   805                 810                 815
   His Gln Val Ser Ala Glu Asn Lys Asn Arg Gln Pro Thr Glu Gly Asn
               820                 825                 830
   Phe Ser Ser Ala Trp Ile Asp His Ser Thr Arg Pro Lys Asp Ala Ser
           835                 840                 845
   Tyr Glu Tyr Met Val Phe Leu Asp Ala Thr Pro Glu Lys Met Gly Glu
       850                 855                 860
   Met Ala Gln Lys Phe Arg Glu Asn Asn Gly Leu Tyr Gln Val Leu Arg
   865                 870                 875                 880
   Lys Asp Lys Asp Val His Ile Ile Leu Asp Lys Leu Ser Asn Val Thr
                   885                 890                 895
   Gly Tyr Ala Phe Tyr Gln Pro Ala Ser Ile Glu Asp Lys Trp Ile Lys
               900                 905                 910
   Lys Val Asn Lys Pro Ala Ile Val Met Thr His Arg Gln Lys Asp Thr
           915                 920                 925
   Leu Ile Val Ser Ala Val Thr Pro Asp Leu Asn Met Thr Arg Gln Lys
       930                 935                 940
   Ala Ala Thr Pro Val Thr Ile Asn Val Thr Ile Asn Gly Lys Trp Gln
   945                 950                 955                 960
   Ser Ala Asp Lys Asn Ser Glu Val Lys Tyr Gln Val Ser Gly Asp Asn
                   965                 970                 975
   Thr Glu Leu Thr Phe Thr Ser Tyr Phe Gly Ile Pro Gln Glu Ile Lys
               980                 985                 990
   Leu Ser Pro Leu Pro
           995
<210> 2
   <211> 977
   <212> PRT
   <213> Artificial
<220>
   <223> Synthetic polypeptide N(delta)20 ABCI protein
<400> 2
   Ala Gln Asn Asn Pro Leu Ala Asp Phe Ser Ser Asp Lys Asn Ser Ile
   1               5                   10                  15
   Leu Thr Leu Ser Asp Lys Arg Ser Ile Met Gly Asn Gln Ser Leu Leu
               20                  25                  30
   Trp Lys Trp Lys Gly Gly Ser Ser Phe Thr Leu His Lys Lys Leu Ile
           35                  40                  45
   Val Pro Thr Asp Lys Glu Ala Ser Lys Ala Trp Gly Arg Ser Ser Thr
       50                  55                  60
   Pro Val Phe Ser Phe Trp Leu Tyr Asn Glu Lys Pro Ile Asp Gly Tyr
   65                  70                  75                  80
   Leu Thr Ile Asp Phe Gly Glu Lys Leu Ile Ser Thr Ser Glu Ala Gln
                   85                  90                  95
   Ala Gly Phe Lys Val Lys Leu Asp Phe Thr Gly Trp Arg Thr Val Gly
               100                 105                 110
   Val Ser Leu Asn Asn Asp Leu Glu Asn Arg Glu Met Thr Leu Asn Ala
           115                 120                 125
   Thr Asn Thr Ser Ser Asp Gly Thr Gln Asp Ser Ile Gly Arg Ser Leu
       130                 135                 140
   Gly Ala Lys Val Asp Ser Ile Arg Phe Lys Ala Pro Ser Asn Val Ser
   145                 150                 155                 160
   Gln Gly Glu Ile Tyr Ile Asp Arg Ile Met Phe Ser Val Asp Asp Ala
                   165                 170                 175
   Arg Tyr Gln Trp Ser Asp Tyr Gln Val Lys Thr Arg Leu Ser Glu Pro
   180 185 190
   Glu Ile Gln Phe His Asn Val Lys Pro Gln Leu Pro Val Thr Pro Glu
   195 200 205
   Asn Leu Ala Ala Ile Asp Leu Ile Arg Gln Arg Leu Ile Asn Glu Phe
   210 215 220
   Val Gly Gly Glu Lys Glu Thr Asn Leu Ala Leu Glu Glu Asn Ile Ser
   225 230 235 240
   Lys Leu Lys Ser Asp Phe Asp Ala Leu Asn Thr His Thr Leu Ala Asn
   245 250 255
   Gly Gly Thr Gln Gly Arg His Leu Ile Thr Asp Lys Gln Ile Ile Ile
   260 265 270
   Tyr Gln Pro Glu Asn Leu Asn Ser Gln Asp Lys Gln Leu Phe Asp Asn
   275 280 285
   Tyr Val Ile Leu Gly Asn Tyr Thr Thr Leu Met Phe Asn Ile Ser Arg
   290 295 300
   Ala Tyr Val Leu Glu Lys Asp Pro Thr Gln Lys Ala Gln Leu Lys Gln
   305 310 315 320
   Met Tyr Leu Leu Met Thr Lys His Leu Leu Asp Gln Gly Phe Val Lys
   325 330 335
   Gly Ser Ala Leu Val Thr Thr His His Trp Gly Tyr Ser Ser Arg Trp
   340 345 350
   Trp Tyr Ile Ser Thr Leu Leu Met Ser Asp Ala Leu Lys Glu Ala Asn
   355 360 365
   Leu Gln Thr Gln Val Tyr Asp Ser Leu Leu Trp Tyr Ser Arg Glu Phe
   370 375 380
   Lys Ser Ser Phe Asp Met Lys Val Ser Ala Asp Ser Ser Asp Leu Asp
   385 390 395 400
   Tyr Phe Asn Thr Leu Ser Arg Gln His Leu Ala Leu Leu Leu Leu Glu
   405 410 415
   Pro Asp Asp Gln Lys Arg Ile Asn Leu Val Asn Thr Phe Ser His Tyr
   420 425 430
   Ile Thr Gly Ala Leu Thr Gln Val Pro Pro Gly Gly Lys Asp Gly Leu
   435 440 445
   Arg Pro Asp Gly Thr Ala Trp Arg His Glu Gly Asn Tyr Pro Gly Tyr
       450                 455                 460
   Ser Phe Pro Ala Phe Lys Asn Ala Ser Gln Leu Ile Tyr Leu Leu Arg
   465                 470                 475                 480
   Asp Thr Pro Phe Ser Val Gly Glu Ser Gly Trp Asn Asn Leu Lys Lys
                   485                 490                 495
   Ala Met Val Ser Ala Trp Ile Tyr Ser Asn Pro Glu Val Gly Leu Pro
               500                 505                 510
   Leu Ala Gly Arg His Pro Phe Asn Ser Pro Ser Leu Lys Ser Val Ala
           515                 520                 525
   Gln Gly Tyr Tyr Trp Leu Ala Met Ser Ala Lys Ser Ser Pro Asp Lys
       530                 535                 540
   Thr Leu Ala Ser Ile Tyr Leu Ala Ile Ser Asp Lys Thr Gln Asn Glu
   545                 550                 555                 560
   Ser Thr Ala Ile Phe Gly Glu Thr Ile Thr Pro Ala Ser Leu Pro Gln
                   565                 570                 575
   Gly Phe Tyr Ala Phe Asn Gly Gly Ala Phe Gly Ile His Arg Trp Gln
               580                 585                 590
   Asp Lys Met Val Thr Leu Lys Ala Tyr Asn Thr Asn Val Trp Ser Ser
           595                 600                 605
   Glu Ile Tyr Asn Lys Asp Asn Arg Tyr Gly Arg Tyr Gln Ser His Gly
       610                 615                 620
   Val Ala Gln Ile Val Ser Asn Gly Ser Gln Leu Ser Gln Gly Tyr Gln
   625                 630                 635                 640
   Gln Glu Gly Trp Asp Trp Asn Arg Met Glu Gly Ala Thr Thr Ile His
                   645                 650                 655
   Leu Pro Leu Lys Asp Leu Asp Ser Pro Lys Pro His Thr Leu Met Gln
               660                 665                 670
   Arg Gly Glu Arg Gly Phe Ser Gly Thr Ser Ser Leu Glu Gly Gln Tyr
           675                 680                 685
   Gly Met Met Ala Phe Asn Leu Ile Tyr Pro Ala Asn Leu Glu Arg Phe
       690                 695                 700
   Asp Pro Asn Phe Thr Ala Lys Lys Ser Val Leu Ala Ala Asp Asn His
   705                 710                 715                 720
   Leu Ile Phe Ile Gly Ser Asn Ile Asn Ser Ser Asp Lys Asn Lys Asn
   725 730 735
   Val Glu Thr Thr Leu Phe Gln His Ala Ile Thr Pro Thr Leu Asn Thr
   740 745 750
   Leu Trp Ile Asn Gly Gln Lys Ile Glu Asn Met Pro Tyr Gln Thr Thr
   755 760 765
   Leu Gln Gln Gly Asp Trp Leu Ile Asp Ser Asn Gly Asn Gly Tyr Leu
   770 775 780
   Ile Thr Gln Ala Glu Lys Val Asn Val Ser Arg Gln His Gln Val Ser
   785 790 795 800
   Ala Glu Asn Lys Asn Arg Gln Pro Thr Glu Gly Asn Phe Ser Ser Ala
   805 810 815
   Trp Ile Asp His Ser Thr Arg Pro Lys Asp Ala Ser Tyr Glu Tyr Met
   820 825 830
   Val Phe Leu Asp Ala Thr Pro Glu Lys Met Gly Glu Met Ala Gln Lys
   835 840 845
   Phe Arg Glu Asn Asn Gly Leu Tyr Gln Val Leu Arg Lys Asp Lys Asp
   850 855 860
   Val His Ile Ile Leu Asp Lys Leu Ser Asn Val Thr Gly Tyr Ala Phe
   865 870 875 880
   Tyr Gln Pro Ala Ser Ile Glu Asp Lys Trp Ile Lys Lys Val Asn Lys
   885 890 895
   Pro Ala Ile Val Met Thr His Arg Gln Lys Asp Thr Leu Ile Val Ser
   900 905 910
   Ala Val Thr Pro Asp Leu Asn Met Thr Arg Gln Lys Ala Ala Thr Pro
   915 920 925
   Val Thr Ile Asn Val Thr Ile Asn Gly Lys Trp Gln Ser Ala Asp Lys
   930 935 940
   Asn Ser Glu Val Lys Tyr Gln Val Ser Gly Asp Asn Thr Glu Leu Thr
   945 950 955 960
   Phe Thr Ser Tyr Phe Gly Ile Pro Gln Glu Ile Lys Leu Ser Pro Leu
   965 970 975
   Pro
<210> 3
   <211> 937
   <212> PRT
   <213> Artificial
<220>
   <223> Synthetic polypeptide N(delta)60 ABCI
<400> 3
   Phe Thr Leu His Lys Lys Leu Ile Val Pro Thr Asp Lys Glu Ala Ser
   1               5                   10                  15
   Lys Ala Trp Gly Arg Ser Ser Thr Pro Val Phe Ser Phe Trp Leu Tyr
               20                  25                  30
   Asn Glu Lys Pro Ile Asp Gly Tyr Leu Thr Ile Asp Phe Gly Glu Lys
           35                  40                  45
   Leu Ile Ser Thr Ser Glu Ala Gln Ala Gly Phe Lys Val Lys Leu Asp
       50                  55                  60
   Phe Thr Gly Trp Arg Thr Val Gly Val Ser Leu Asn Asn Asp Leu Glu
   65                  70                  75                  80
   Asn Arg Glu Met Thr Leu Asn Ala Thr Asn Thr Ser Ser Asp Gly Thr
                   85                  90                  95
   Gln Asp Ser Ile Gly Arg Ser Leu Gly Ala Lys Val Asp Ser Ile Arg
               100                 105                 110
   Phe Lys Ala Pro Ser Asn Val Ser Gln Gly Glu Ile Tyr Ile Asp Arg
           115                 120                 125
   Ile Met Phe Ser Val Asp Asp Ala Arg Tyr Gln Trp Ser Asp Tyr Gln
       130                 135                 140
   Val Lys Thr Arg Leu Ser Glu Pro Glu Ile Gln Phe His Asn Val Lys
   145                 150                 155                 160
   Pro Gln Leu Pro Val Thr Pro Glu Asn Leu Ala Ala Ile Asp Leu Ile
                   165                 170                 175
   Arg Gln Arg Leu Ile Asn Glu Phe Val Gly Gly Glu Lys Glu Thr Asn
               180                 185                 190
   Leu Ala Leu Glu Glu Asn Ile Ser Lys Leu Lys Ser Asp Phe Asp Ala
           195                 200                 205
   Leu Asn Thr His Thr Leu Ala Asn Gly Gly Thr Gln Gly Arg His Leu
       210                 215                 220
   Ile Thr Asp Lys Gln Ile Ile Ile Tyr Gln Pro Glu Asn Leu Asn Ser
   225                 230                 235                 240
   Gln Asp Lys Gln Leu Phe Asp Asn Tyr Val Ile Leu Gly Asn Tyr Thr
                   245                 250                 255
   Thr Leu Met Phe Asn Ile Ser Arg Ala Tyr Val Leu Glu Lys Asp Pro
   260 265 270
   Thr Gln Lys Ala Gln Leu Lys Gln Met Tyr Leu Leu Met Thr Lys His
   275 280 285
   Leu Leu Asp Gln Gly Phe Val Lys Gly Ser Ala Leu Val Thr Thr His
   290 295 300
   His Trp Gly Tyr Ser Ser Arg Trp Trp Tyr Ile Ser Thr Leu Leu Met
   305 310 315 320
   Ser Asp Ala Leu Lys Glu Ala Asn Leu Gln Thr Gln Val Tyr Asp Ser
   325 330 335
   Leu Leu Trp Tyr Ser Arg Glu Phe Lys Ser Ser Phe Asp Met Lys Val
   340 345 350
   Ser Ala Asp Ser Ser Asp Leu Asp Tyr Phe Asn Thr Leu Ser Arg Gln
   355 360 365
   His Leu Ala Leu Leu Leu Leu Glu Pro Asp Asp Gln Lys Arg Ile Asn
   370 375 380
   Leu Val Asn Thr Phe Ser His Tyr Ile Thr Gly Ala Leu Thr Gln Val
   385 390 395 400
   Pro Pro Gly Gly Lys Asp Gly Leu Arg Pro Asp Gly Thr Ala Trp Arg
   405 410 415
   His Glu Gly Asn Tyr Pro Gly Tyr Ser Phe Pro Ala Phe Lys Asn Ala
   420 425 430
   Ser Gln Leu Ile Tyr Leu Leu Arg Asp Thr Pro Phe Ser Val Gly Glu
   435 440 445
   Ser Gly Trp Asn Asn Leu Lys Lys Ala Met Val Ser Ala Trp Ile Tyr
   450 455 460
   Ser Asn Pro Glu Val Gly Leu Pro Leu Ala Gly Arg His Pro Phe Asn
   465 470 475 480
   Ser Pro Ser Leu Lys Ser Val Ala Gln Gly Tyr Tyr Trp Leu Ala Met
   485 490 495
   Ser Ala Lys Ser Ser Pro Asp Lys Thr Leu Ala Ser Ile Tyr Leu Ala
   500 505 510
   Ile Ser Asp Lys Thr Gln Asn Glu Ser Thr Ala Ile Phe Gly Glu Thr
   515 520 525
   Ile Thr Pro Ala Ser Leu Pro Gln Gly Phe Tyr Ala Phe Asn Gly Gly
       530                 535                 540
   Ala Phe Gly Ile His Arg Trp Gln Asp Lys Met Val Thr Leu Lys Ala
   545                 550                 555                 560
   Tyr Asn Thr Asn Val Trp Ser Ser Glu Ile Tyr Asn Lys Asp Asn Arg
                   565                 570                 575
   Tyr Gly Arg Tyr Gln Ser His Gly Val Ala Gln Ile Val Ser Asn Gly
               580                 585                 590
   Ser Gln Leu Ser Gln Gly Tyr Gln Gln Glu Gly Trp Asp Trp Asn Arg
           595                 600                 605
   Met Glu Gly Ala Thr Thr Ile His Leu Pro Leu Lys Asp Leu Asp Ser
       610                 615                 620
   Pro Lys Pro His Thr Leu Met Gln Arg Gly Glu Arg Gly Phe Ser Gly
   625                 630                 635                 640
   Thr Ser Ser Leu Glu Gly Gln Tyr Gly Met Met Ala Phe Asn Leu Ile
                   645                 650                 655
   Tyr Pro Ala Asn Leu Glu Arg Phe Asp Pro Asn Phe Thr Ala Lys Lys
               660                 665                 670
   Ser Val Leu Ala Ala Asp Asn His Leu Ile Phe Ile Gly Ser Asn Ile
           675                 680                 685
   Asn Ser Ser Asp Lys Asn Lys Asn Val Glu Thr Thr Leu Phe Gln His
       690                 695                 700
   Ala Ile Thr Pro Thr Leu Asn Thr Leu Trp Ile Asn Gly Gln Lys Ile
   705                 710                 715                 720
   Glu Asn Met Pro Tyr Gln Thr Thr Leu Gln Gln Gly Asp Trp Leu Ile
                   725                 730                 735
   Asp Ser Asn Gly Asn Gly Tyr Leu Ile Thr Gln Ala Glu Lys Val Asn
               740                 745                 750
   Val Ser Arg Gln His Gln Val Ser Ala Glu Asn Lys Asn Arg Gln Pro
           755                 760                 765
   Thr Glu Gly Asn Phe Ser Ser Ala Trp Ile Asp His Ser Thr Arg Pro
       770                 775                 780
   Lys Asp Ala Ser Tyr Glu Tyr Met Val Phe Leu Asp Ala Thr Pro Glu
   785                 790                 795                 800
   Lys Met Gly Glu Met Ala Gln Lys Phe Arg Glu Asn Asn Gly Leu Tyr
                   805                 810                 815
   Gln Val Leu Arg Lys Asp Lys Asp Val His Ile Ile Leu Asp Lys Leu
               820                 825                 830
   Ser Asn Val Thr Gly Tyr Ala Phe Tyr Gln Pro Ala Ser Ile Glu Asp
           835                 840                 845
   Lys Trp Ile Lys Lys Val Asn Lys Pro Ala Ile Val Met Thr His Arg
       850                 855                 860
   Gln Lys Asp Thr Leu Ile Val Ser Ala Val Thr Pro Asp Leu Asn Met
   865                 870                 875                 880
   Thr Arg Gln Lys Ala Ala Thr Pro Val Thr Ile Asn Val Thr Ile Asn
                   885                 890                 895
   Gly Lys Trp Gln Ser Ala Asp Lys Asn Ser Glu Val Lys Tyr Gln Val
               900                 905                 910
   Ser Gly Asp Asn Thr Glu Leu Thr Phe Thr Ser Tyr Phe Gly Ile Pro
           915                 920                 925
   Gln Glu Ile Lys Leu Ser Pro Leu Pro
       930                 935
<210> 4
   <211> 858
   <212> PRT
   <213> Artificial
<220>
   <223> Synthetic polypeptide, N(delta) 60 C(delta)80 ABCI (F[sub]85 - A[sub]942)
<400> 4
   Phe Thr Leu His Lys Lys Leu Ile Val Pro Thr Asp Lys Glu Ala Ser
   1               5                   10                  15
   Lys Ala Trp Gly Arg Ser Ser Thr Pro Val Phe Ser Phe Trp Leu Tyr
               20                  25                  30
   Asn Glu Lys Pro Ile Asp Gly Tyr Leu Thr Ile Asp Phe Gly Glu Lys
           35                  40                  45
   Leu Ile Ser Thr Ser Glu Ala Gln Ala Gly Phe Lys Val Lys Leu Asp
       50                  55                  60
   Phe Thr Gly Trp Arg Thr Val Gly Val Ser Leu Asn Asn Asp Leu Glu
   65                  70                  75                  80
   Asn Arg Glu Met Thr Leu Asn Ala Thr Asn Thr Ser Ser Asp Gly Thr
                   85                  90                  95
   Gln Asp Ser Ile Gly Arg Ser Leu Gly Ala Lys Val Asp Ser Ile Arg
               100                 105                 110
   Phe Lys Ala Pro Ser Asn Val Ser Gln Gly Glu Ile Tyr Ile Asp Arg
           115                 120                 125
   Ile Met Phe Ser Val Asp Asp Ala Arg Tyr Gln Trp Ser Asp Tyr Gln
       130                 135                 140
   Val Lys Thr Arg Leu Ser Glu Pro Glu Ile Gln Phe His Asn Val Lys
   145                 150                 155                 160
   Pro Gln Leu Pro Val Thr Pro Glu Asn Leu Ala Ala Ile Asp Leu Ile
                   165                 170                 175
   Arg Gln Arg Leu Ile Asn Glu Phe Val Gly Gly Glu Lys Glu Thr Asn
               180                 185                 190
   Leu Ala Leu Glu Glu Asn Ile Ser Lys Leu Lys Ser Asp Phe Asp Ala
           195                 200                 205
   Leu Asn Thr His Thr Leu Ala Asn Gly Gly Thr Gln Gly Arg His Leu
       210                 215                 220
   Ile Thr Asp Lys Gln Ile Ile Ile Tyr Gln Pro Glu Asn Leu Asn Ser
   225                 230                 235                 240
   Gln Asp Lys Gln Leu Phe Asp Asn Tyr Val Ile Leu Gly Asn Tyr Thr
                   245                 250                 255
   Thr Leu Met Phe Asn Ile Ser Arg Ala Tyr Val Leu Glu Lys Asp Pro
               260                 265                 270
   Thr Gln Lys Ala Gln Leu Lys Gln Met Tyr Leu Leu Met Thr Lys His
           275                 280                 285
   Leu Leu Asp Gln Gly Phe Val Lys Gly Ser Ala Leu Val Thr Thr His
       290                 295                 300
   His Trp Gly Tyr Ser Ser Arg Trp Trp Tyr Ile Ser Thr Leu Leu Met
   305                 310                 315                 320
   Ser Asp Ala Leu Lys Glu Ala Asn Leu Gln Thr Gln Val Tyr Asp Ser
                   325                 330                 335
   Leu Leu Trp Tyr Ser Arg Glu Phe Lys Ser Ser Phe Asp Met Lys Val
               340                 345                 350
   Ser Ala Asp Ser Ser Asp Leu Asp Tyr Phe Asn Thr Leu Ser Arg Gln
           355                 360                 365
   His Leu Ala Leu Leu Leu Leu Glu Pro Asp Asp Gln Lys Arg Ile Asn
       370                 375                 380
   Leu Val Asn Thr Phe Ser His Tyr Ile Thr Gly Ala Leu Thr Gln Val
   385                 390                 395                 400
   Pro Pro Gly Gly Lys Asp Gly Leu Arg Pro Asp Gly Thr Ala Trp Arg
                   405                 410                 415
   His Glu Gly Asn Tyr Pro Gly Tyr Ser Phe Pro Ala Phe Lys Asn Ala
               420                 425                 430
   Ser Gln Leu Ile Tyr Leu Leu Arg Asp Thr Pro Phe Ser Val Gly Glu
           435                 440                 445
   Ser Gly Trp Asn Asn Leu Lys Lys Ala Met Val Ser Ala Trp Ile Tyr
       450                 455                 460
   Ser Asn Pro Glu Val Gly Leu Pro Leu Ala Gly Arg His Pro Phe Asn
   465                 470                 475                 480
   Ser Pro Ser Leu Lys Ser Val Ala Gln Gly Tyr Tyr Trp Leu Ala Met
                   485                 490                 495
   Ser Ala Lys Ser Ser Pro Asp Lys Thr Leu Ala Ser Ile Tyr Leu Ala
               500                 505                 510
   Ile Ser Asp Lys Thr Gln Asn Glu Ser Thr Ala Ile Phe Gly Glu Thr
           515                 520                 525
   Ile Thr Pro Ala Ser Leu Pro Gln Gly Phe Tyr Ala Phe Asn Gly Gly
       530                 535                 540
   Ala Phe Gly Ile His Arg Trp Gln Asp Lys Met Val Thr Leu Lys Ala
   545                 550                 555                 560
   Tyr Asn Thr Asn Val Trp Ser Ser Glu Ile Tyr Asn Lys Asp Asn Arg
                   565                 570                 575
   Tyr Gly Arg Tyr Gln Ser His Gly Val Ala Gln Ile Val Ser Asn Gly
               580                 585                 590
   Ser Gln Leu Ser Gln Gly Tyr Gln Gln Glu Gly Trp Asp Trp Asn Arg
           595                 600                 605
   Met Glu Gly Ala Thr Thr Ile His Leu Pro Leu Lys Asp Leu Asp Ser
       610                 615                 620
   Pro Lys Pro His Thr Leu Met Gln Arg Gly Glu Arg Gly Phe Ser Gly
   625                 630                 635                 640
   Thr Ser Ser Leu Glu Gly Gln Tyr Gly Met Met Ala Phe Asn Leu Ile
   645 650 655
   Tyr Pro Ala Asn Leu Glu Arg Phe Asp Pro Asn Phe Thr Ala Lys Lys
   660 665 670
   Ser Val Leu Ala Ala Asp Asn His Leu Ile Phe Ile Gly Ser Asn Ile
   675 680 685
   Asn Ser Ser Asp Lys Asn Lys Asn Val Glu Thr Thr Leu Phe Gln His
   690 695 700
   Ala Ile Thr Pro Thr Leu Asn Thr Leu Trp Ile Asn Gly Gln Lys Ile
   705 710 715 720
   Glu Asn Met Pro Tyr Gln Thr Thr Leu Gln Gln Gly Asp Trp Leu Ile
   725 730 735
   Asp Ser Asn Gly Asn Gly Tyr Leu Ile Thr Gln Ala Glu Lys Val Asn
   740 745 750
   Val Ser Arg Gln His Gln Val Ser Ala Glu Asn Lys Asn Arg Gln Pro
   755 760 765
   Thr Glu Gly Asn Phe Ser Ser Ala Trp Ile Asp His Ser Thr Arg Pro
   770 775 780
   Lys Asp Ala Ser Tyr Glu Tyr Met Val Phe Leu Asp Ala Thr Pro Glu
   785 790 795 800
   Lys Met Gly Glu Met Ala Gln Lys Phe Arg Glu Asn Asn Gly Leu Tyr
   805 810 815
   Gln Val Leu Arg Lys Asp Lys Asp Val His Ile Ile Leu Asp Lys Leu
   820 825 830
   Ser Asn Val Thr Gly Tyr Ala Phe Tyr Gln Pro Ala Ser Ile Glu Asp
   835 840 845
   Lys Trp Ile Lys Lys Val Asn Lys Pro Ala
   850 855
<210> 5
   <211> 700
   <212> PRT
   <213> Pedobacter heparinus
<220>
   <223> LOCUS (1HMW_A), Chain A, Active site of Chondroitinase AC Lyase
<400> 5
   Met Lys Lys Leu Phe Val Thr Cys Ile Val Phe Phe Ser Ile Leu Ser
   1               5                   10                  15
   Pro Ala Leu Leu Ile Ala Gln Gln Thr Gly Thr Ala Glu Leu Ile Met
   20 25 30
   Lys Arg Val Met Leu Asp Leu Lys Lys Pro Leu Arg Asn Met Asp Lys
   35 40 45
   Val Ala Glu Lys Asn Leu Asn Thr Leu Gln Pro Asp Gly Ser Trp Lys
   50 55 60
   Asp Val Pro Tyr Lys Asp Asp Ala Met Thr Asn Trp Leu Pro Asn Asn
   65 70 75 80
   His Leu Leu Gln Leu Glu Thr Ile Ile Gln Ala Tyr Ile Glu Lys Asp
   85 90 95
   Ser His Tyr Tyr Gly Asp Asp Lys Val Phe Asp Gln Ile Ser Lys Ala
   100 105 110
   Phe Lys Tyr Trp Tyr Asp Ser Asp Pro Lys Ser Arg Asn Trp Trp His
   115 120 125
   Asn Glu Ile Ala Thr Pro Gln Ala Leu Gly Glu Met Leu Ile Leu Met
   130 135 140
   Arg Tyr Gly Lys Lys Pro Leu Asp Glu Ala Leu Val His Lys Leu Thr
   145 150 155 160
   Glu Arg Met Lys Arg Gly Glu Pro Glu Lys Lys Thr Gly Ala Asn Lys
   165 170 175
   Thr Asp Ile Ala Leu His Tyr Phe Tyr Arg Ala Leu Leu Thr Ser Asp
   180 185 190
   Glu Ala Leu Leu Ser Phe Ala Val Lys Glu Leu Phe Tyr Pro Val Gln
   195 200 205
   Phe Val His Tyr Glu Glu Gly Leu Gln Tyr Asp Tyr Ser Tyr Leu Gln
   210 215 220
   His Gly Pro Gln Leu Gln Ile Ser Ser Tyr Gly Ala Val Phe Ile Thr
   225 230 235 240
   Gly Val Leu Lys Leu Ala Asn Tyr Val Arg Asp Thr Pro Tyr Ala Leu
   245 250 255
   Ser Thr Glu Lys Leu Ala Ile Phe Ser Lys Tyr Tyr Arg Asp Ser Tyr
   260 265 270
   Leu Lys Ala Ile Arg Gly Ser Tyr Met Asp Phe Asn Val Glu Gly Arg
   275 280 285
   Gly Val Ser Arg Pro Asp Ile Leu Asn Lys Lys Ala Glu Lys Lys Arg
       290                 295                 300
   Leu Leu Val Ala Lys Met Ile Asp Leu Lys His Thr Glu Glu Trp Ala
   305                 310                 315                 320
   Asp Ala Ile Ala Arg Thr Asp Ser Thr Val Ala Ala Gly Tyr Lys Ile
                   325                 330                 335
   Glu Pro Tyr His His Gln Phe Trp Asn Gly Asp Tyr Val Gln His Leu
               340                 345                 350
   Arg Pro Ala Tyr Ser Phe Asn Val Arg Met Val Ser Lys Arg Thr Arg
           355                 360                 365
   Arg Ser Glu Ser Gly Asn Lys Glu Asn Leu Leu Gly Arg Tyr Leu Ser
       370                 375                 380
   Asp Gly Ala Thr Asn Ile Gln Leu Arg Gly Pro Glu Tyr Tyr Asn Ile
   385                 390                 395                 400
   Met Pro Val Trp Glu Trp Asp Lys Ile Pro Gly Ile Thr Ser Arg Asp
                   405                 410                 415
   Tyr Leu Thr Asp Arg Pro Leu Thr Lys Leu Trp Gly Glu Gln Gly Ser
               420                 425                 430
   Asn Asp Phe Ala Gly Gly Val Ser Asp Gly Val Tyr Gly Ala Ser Ala
           435                 440                 445
   Tyr Ala Leu Asp Tyr Asp Ser Leu Gln Ala Lys Lys Ala Trp Phe Phe
       450                 455                 460
   Phe Asp Lys Glu Ile Val Cys Leu Gly Ala Gly Ile Asn Ser Asn Ala
   465                 470                 475                 480
   Pro Glu Asn Ile Thr Thr Thr Leu Asn Gln Ser Trp Leu Asn Gly Pro
                   485                 490                 495
   Val Ile Ser Thr Ala Gly Lys Thr Gly Arg Gly Lys Ile Thr Thr Phe
               500                 505                 510
   Lys Ala Gln Gly Gln Phe Trp Leu Leu His Asp Ala Ile Gly Tyr Tyr
           515                 520                 525
   Phe Pro Glu Gly Ala Asn Leu Ser Leu Ser Thr Gln Ser Gln Lys Gly
       530                 535                 540
   Asn Trp Phe His Ile Asn Asn Ser His Ser Lys Asp Glu Val Ser Gly
   545                 550                 555                 560
   Asp Val Phe Lys Leu Trp Ile Asn His Gly Ala Arg Pro Glu Asn Ala
                       565                 570                 575
   Gln Tyr Ala Tyr Ile Val Leu Pro Gly Ile Asn Lys Pro Glu Glu Ile
                   580                 585                 590
   Lys Lys Tyr Asn Gly Thr Ala Pro Lys Val Leu Ala Asn Thr Asn Gln
               595                 600                 605
   Leu Gln Ala Val Tyr His Gln Gln Leu Asp Met Val Gln Ala Ile Phe
       610                 615                 620
   Tyr Thr Ala Gly Lys Leu Ser Val Ala Gly Ile Glu Ile Glu Thr Asp
   625                 630                 635                 640
   Lys Pro Cys Ala Val Leu Ile Lys His Ile Asn Gly Lys Gln Val Ile
                   645                 650                 655
   Trp Ala Ala Asp Pro Leu Gln Lys Glu Lys Thr Ala Val Leu Ser Ile
               660                 665                 670
   Arg Asp Leu Lys Thr Gly Lys Thr Asn Arg Val Lys Ile Asp Phe Pro
           675                 680                 685
   Gln Gln Glu Phe Ala Gly Ala Thr Val Glu Leu Lys
       690                 695                 700
<210> 6
   <211> 478
   <212> PRT
   <213> Artificial
<220>
   <223> Synthetic polypeptide C(delta)200 chondroitinase AC (Q[sub]23 - T[sub]500)
<400> 6
   Gln Gln Thr Gly Thr Ala Glu Leu Ile Met Lys Arg Val Met Leu Asp
   1               5                   10                  15
   Leu Lys Lys Pro Leu Arg Asn Met Asp Lys Val Ala Glu Lys Asn Leu
               20                  25                  30
   Asn Thr Leu Gln Pro Asp Gly Ser Trp Lys Asp Val Pro Tyr Lys Asp
           35                  40                  45
   Asp Ala Met Thr Asn Trp Leu Pro Asn Asn His Leu Leu Gln Leu Glu
       50                  55                  60
   Thr Ile Ile Gln Ala Tyr Ile Glu Lys Asp Ser His Tyr Tyr Gly Asp
   65                  70                  75                  80
   Asp Lys Val Phe Asp Gln Ile Ser Lys Ala Phe Lys Tyr Trp Tyr Asp
                   85                  90                  95
   Ser Asp Pro Lys Ser Arg Asn Trp Trp His Asn Glu Ile Ala Thr Pro
               100                 105                 110
   Gln Ala Leu Gly Glu Met Leu Ile Leu Met Arg Tyr Gly Lys Lys Pro
           115                 120                 125
   Leu Asp Glu Ala Leu Val His Lys Leu Thr Glu Arg Met Lys Arg Gly
       130                 135                 140
   Glu Pro Glu Lys Lys Thr Gly Ala Asn Lys Thr Asp Ile Ala Leu His
   145                 150                 155                 160
   Tyr Phe Tyr Arg Ala Leu Leu Thr Ser Asp Glu Ala Leu Leu Ser Phe
                   165                 170                 175
   Ala Val Lys Glu Leu Phe Tyr Pro Val Gln Phe Val His Tyr Glu Glu
               180                 185                 190
   Gly Leu Gln Tyr Asp Tyr Ser Tyr Leu Gln His Gly Pro Gln Leu Gln
           195                 200                 205
   Ile Ser Ser Tyr Gly Ala Val Phe Ile Thr Gly Val Leu Lys Leu Ala
       210                 215                 220
   Asn Tyr Val Arg Asp Thr Pro Tyr Ala Leu Ser Thr Glu Lys Leu Ala
   225                 230                 235                 240
   Ile Phe Ser Lys Tyr Tyr Arg Asp Ser Tyr Leu Lys Ala Ile Arg Gly
                   245                 250                 255
   Ser Tyr Met Asp Phe Asn Val Glu Gly Arg Gly Val Ser Arg Pro Asp
               260                 265                 270
   Ile Leu Asn Lys Lys Ala Glu Lys Lys Arg Leu Leu Val Ala Lys Met
           275                 280                 285
   Ile Asp Leu Lys His Thr Glu Glu Trp Ala Asp Ala Ile Ala Arg Thr
       290                 295                 300
   Asp Ser Thr Val Ala Ala Gly Tyr Lys Ile Glu Pro Tyr His His Gln
   305                 310                 315                 320
   Phe Trp Asn Gly Asp Tyr Val Gln His Leu Arg Pro Ala Tyr Ser Phe
                   325                 330                 335
   Asn Val Arg Met Val Ser Lys Arg Thr Arg Arg Ser Glu Ser Gly Asn
               340                 345                 350
   Lys Glu Asn Leu Leu Gly Arg Tyr Leu Ser Asp Gly Ala Thr Asn Ile
           355                 360                 365
   Gln Leu Arg Gly Pro Glu Tyr Tyr Asn Ile Met Pro Val Trp Glu Trp
       370                 375                 380
   Asp Lys Ile Pro Gly Ile Thr Ser Arg Asp Tyr Leu Thr Asp Arg Pro
   385                 390                 395                 400
   Leu Thr Lys Leu Trp Gly Glu Gln Gly Ser Asn Asp Phe Ala Gly Gly
                   405                 410                 415
   Val Ser Asp Gly Val Tyr Gly Ala Ser Ala Tyr Ala Leu Asp Tyr Asp
               420                 425                 430
   Ser Leu Gln Ala Lys Lys Ala Trp Phe Phe Phe Asp Lys Glu Ile Val
           435                 440                 445
   Cys Leu Gly Ala Gly Ile Asn Ser Asn Ala Pro Glu Asn Ile Thr Thr
       450                 455                 460
   Thr Leu Asn Gln Ser Trp Leu Asn Gly Pro Val Ile Ser Thr
   465                 470                 475
<210> 7
   <211> 458
   <212> PRT
   <213> Artificial
<220>
   <223> Synthetic polypeptide C(delta)220 chondroitinase AC (Q[sub]23 - A[sub]480)
<400> 7
   Gln Gln Thr Gly Thr Ala Glu Leu Ile Met Lys Arg Val Met Leu Asp
   1               5                   10                  15
   Leu Lys Lys Pro Leu Arg Asn Met Asp Lys Val Ala Glu Lys Asn Leu
               20                  25                  30
   Asn Thr Leu Gln Pro Asp Gly Ser Trp Lys Asp Val Pro Tyr Lys Asp
           35                  40                  45
   Asp Ala Met Thr Asn Trp Leu Pro Asn Asn His Leu Leu Gln Leu Glu
       50                  55                  60
   Thr Ile Ile Gln Ala Tyr Ile Glu Lys Asp Ser His Tyr Tyr Gly Asp
   65                  70                  75                  80
   Asp Lys Val Phe Asp Gln Ile Ser Lys Ala Phe Lys Tyr Trp Tyr Asp
                   85                  90                  95
   Ser Asp Pro Lys Ser Arg Asn Trp Trp His Asn Glu Ile Ala Thr Pro
               100                 105                 110
   Gln Ala Leu Gly Glu Met Leu Ile Leu Met Arg Tyr Gly Lys Lys Pro
           115                 120                 125
   Leu Asp Glu Ala Leu Val His Lys Leu Thr Glu Arg Met Lys Arg Gly
       130                 135                 140
   Glu Pro Glu Lys Lys Thr Gly Ala Asn Lys Thr Asp Ile Ala Leu His
   145                 150                 155                 160
   Tyr Phe Tyr Arg Ala Leu Leu Thr Ser Asp Glu Ala Leu Leu Ser Phe
                   165                 170                 175
   Ala Val Lys Glu Leu Phe Tyr Pro Val Gln Phe Val His Tyr Glu Glu
               180                 185                 190
   Gly Leu Gln Tyr Asp Tyr Ser Tyr Leu Gln His Gly Pro Gln Leu Gln
           195                 200                 205
   Ile Ser Ser Tyr Gly Ala Val Phe Ile Thr Gly Val Leu Lys Leu Ala
       210                 215                 220
   Asn Tyr Val Arg Asp Thr Pro Tyr Ala Leu Ser Thr Glu Lys Leu Ala
   225                 230                 235                 240
   Ile Phe Ser Lys Tyr Tyr Arg Asp Ser Tyr Leu Lys Ala Ile Arg Gly
                   245                 250                 255
   Ser Tyr Met Asp Phe Asn Val Glu Gly Arg Gly Val Ser Arg Pro Asp
               260                 265                 270
   Ile Leu Asn Lys Lys Ala Glu Lys Lys Arg Leu Leu Val Ala Lys Met
           275                 280                 285
   Ile Asp Leu Lys His Thr Glu Glu Trp Ala Asp Ala Ile Ala Arg Thr
       290                 295                 300
   Asp Ser Thr Val Ala Ala Gly Tyr Lys Ile Glu Pro Tyr His His Gln
   305                 310                 315                 320
   Phe Trp Asn Gly Asp Tyr Val Gln His Leu Arg Pro Ala Tyr Ser Phe
                   325                 330                 335
   Asn Val Arg Met Val Ser Lys Arg Thr Arg Arg Ser Glu Ser Gly Asn
               340                 345                 350
   Lys Glu Asn Leu Leu Gly Arg Tyr Leu Ser Asp Gly Ala Thr Asn Ile
           355                 360                 365
   Gln Leu Arg Gly Pro Glu Tyr Tyr Asn Ile Met Pro Val Trp Glu Trp
       370                 375                 380
   Asp Lys Ile Pro Gly Ile Thr Ser Arg Asp Tyr Leu Thr Asp Arg Pro
   385                 390                 395                 400
   Leu Thr Lys Leu Trp Gly Glu Gln Gly Ser Asn Asp Phe Ala Gly Gly
   405 410 415
   Val Ser Asp Gly Val Tyr Gly Ala Ser Ala Tyr Ala Leu Asp Tyr Asp
   420 425 430
   Ser Leu Gln Ala Lys Lys Ala Trp Phe Phe Phe Asp Lys Glu Ile Val
   435 440 445
   Cys Leu Gly Ala Gly Ile Asn Ser Asn Ala
   450 455
<210> 8
   <211> 458
   <212> PRT
   <213> Artificial
<220>
   <223> Synthetic polypeptide, N(delta)20 C(delta)200 chondroitinase AC (L[sub]43 -T[sub]500)
<400> 8
   Leu Arg Asn Met Asp Lys Val Ala Glu Lys Asn Leu Asn Thr Leu Gln
   1 5 10 15
   Pro Asp Gly Ser Trp Lys Asp Val Pro Tyr Lys Asp Asp Ala Met Thr
   20 25 30
   Asn Trp Leu Pro Asn Asn His Leu Leu Gln Leu Glu Thr Ile Ile Gln
   35 40 45
   Ala Tyr Ile Glu Lys Asp Ser His Tyr Tyr Gly Asp Asp Lys Val Phe
   50 55 60
   Asp Gln Ile Ser Lys Ala Phe Lys Tyr Trp Tyr Asp Ser Asp Pro Lys
   65 70 75 80
   Ser Arg Asn Trp Trp His Asn Glu Ile Ala Thr Pro Gln Ala Leu Gly
   85 90 95
   Glu Met Leu Ile Leu Met Arg Tyr Gly Lys Lys Pro Leu Asp Glu Ala
   100 105 110
   Leu Val His Lys Leu Thr Glu Arg Met Lys Arg Gly Glu Pro Glu Lys
   115 120 125
   Lys Thr Gly Ala Asn Lys Thr Asp Ile Ala Leu His Tyr Phe Tyr Arg
   130 135 140
   Ala Leu Leu Thr Ser Asp Glu Ala Leu Leu Ser Phe Ala Val Lys Glu
   145 150 155 160
   Leu Phe Tyr Pro Val Gln Phe Val His Tyr Glu Glu Gly Leu Gln Tyr
   165 170 175
   Asp Tyr Ser Tyr Leu Gln His Gly Pro Gln Leu Gln Ile Ser Ser Tyr
   180 185 190
   Gly Ala Val Phe Ile Thr Gly Val Leu Lys Leu Ala Asn Tyr Val Arg
   195 200 205
   Asp Thr Pro Tyr Ala Leu Ser Thr Glu Lys Leu Ala Ile Phe Ser Lys
   210 215 220
   Tyr Tyr Arg Asp Ser Tyr Leu Lys Ala Ile Arg Gly Ser Tyr Met Asp
   225 230 235 240
   Phe Asn Val Glu Gly Arg Gly Val Ser Arg Pro Asp Ile Leu Asn Lys
   245 250 255
   Lys Ala Glu Lys Lys Arg Leu Leu Val Ala Lys Met Ile Asp Leu Lys
   260 265 270
   His Thr Glu Glu Trp Ala Asp Ala Ile Ala Arg Thr Asp Ser Thr Val
   275 280 285
   Ala Ala Gly Tyr Lys Ile Glu Pro Tyr His His Gln Phe Trp Asn Gly
   290 295 300
   Asp Tyr Val Gln His Leu Arg Pro Ala Tyr Ser Phe Asn Val Arg Met
   305 310 315 320
   Val Ser Lys Arg Thr Arg Arg Ser Glu Ser Gly Asn Lys Glu Asn Leu
   325 330 335
   Leu Gly Arg Tyr Leu Ser Asp Gly Ala Thr Asn Ile Gln Leu Arg Gly
   340 345 350
   Pro Glu Tyr Tyr Asn Ile Met Pro Val Trp Glu Trp Asp Lys Ile Pro
   355 360 365
   Gly Ile Thr Ser Arg Asp Tyr Leu Thr Asp Arg Pro Leu Thr Lys Leu
   370 375 380
   Trp Gly Glu Gln Gly Ser Asn Asp Phe Ala Gly Gly Val Ser Asp Gly
   385 390 395 400
   Val Tyr Gly Ala Ser Ala Tyr Ala Leu Asp Tyr Asp Ser Leu Gln Ala
   405 410 415
   Lys Lys Ala Trp Phe Phe Phe Asp Lys Glu Ile Val Cys Leu Gly Ala
   420 425 430
   Gly Ile Asn Ser Asn Ala Pro Glu Asn Ile Thr Thr Thr Leu Asn Gln
   435 440 445
   Ser Trp Leu Asn Gly Pro Val Ile Ser Thr
   450 455
<210> 9
   <211> 427
   <212> PRT
   <213> Artificial
<220>
   <223> Synthetic polypeptide, N(delta)50 C(delta)200 of chondroitinase AC (T[sub]74 -T[sub]500)
<400> 9
   Thr Asn Trp Leu Pro Asn Asn His Leu Leu Gln Leu Glu Thr Ile Ile
   1 5 10 15
   Gln Ala Tyr Ile Glu Lys Asp Ser His Tyr Tyr Gly Asp Asp Lys Val
   20 25 30
   Phe Asp Gln Ile Ser Lys Ala Phe Lys Tyr Trp Tyr Asp Ser Asp Pro
   35 40 45
   Lys Ser Arg Asn Trp Trp His Asn Glu Ile Ala Thr Pro Gln Ala Leu
   50 55 60
   Gly Glu Met Leu Ile Leu Met Arg Tyr Gly Lys Lys Pro Leu Asp Glu
   65 70 75 80
   Ala Leu Val His Lys Leu Thr Glu Arg Met Lys Arg Gly Glu Pro Glu
   85 90 95
   Lys Lys Thr Gly Ala Asn Lys Thr Asp Ile Ala Leu His Tyr Phe Tyr
   100 105 110
   Arg Ala Leu Leu Thr Ser Asp Glu Ala Leu Leu Ser Phe Ala Val Lys
   115 120 125
   Glu Leu Phe Tyr Pro Val Gln Phe Val His Tyr Glu Glu Gly Leu Gln
   130 135 140
   Tyr Asp Tyr Ser Tyr Leu Gln His Gly Pro Gln Leu Gln Ile Ser Ser
   145 150 155 160
   Tyr Gly Ala Val Phe Ile Thr Gly Val Leu Lys Leu Ala Asn Tyr Val
   165 170 175
   Arg Asp Thr Pro Tyr Ala Leu Ser Thr Glu Lys Leu Ala Ile Phe Ser
   180 185 190
   Lys Tyr Tyr Arg Asp Ser Tyr Leu Lys Ala Ile Arg Gly Ser Tyr Met
   195 200 205
   Asp Phe Asn Val Glu Gly Arg Gly Val Ser Arg Pro Asp Ile Leu Asn
       210                 215                 220
   Lys Lys Ala Glu Lys Lys Arg Leu Leu Val Ala Lys Met Ile Asp Leu
   225                 230                 235                 240
   Lys His Thr Glu Glu Trp Ala Asp Ala Ile Ala Arg Thr Asp Ser Thr
                   245                 250                 255
   Val Ala Ala Gly Tyr Lys Ile Glu Pro Tyr His His Gln Phe Trp Asn
               260                 265                 270
   Gly Asp Tyr Val Gln His Leu Arg Pro Ala Tyr Ser Phe Asn Val Arg
           275                 280                 285
   Met Val Ser Lys Arg Thr Arg Arg Ser Glu Ser Gly Asn Lys Glu Asn
       290                 295                 300
   Leu Leu Gly Arg Tyr Leu Ser Asp Gly Ala Thr Asn Ile Gln Leu Arg
   305                 310                 315                 320
   Gly Pro Glu Tyr Tyr Asn Ile Met Pro Val Trp Glu Trp Asp Lys Ile
                   325                 330                 335
   Pro Gly Ile Thr Ser Arg Asp Tyr Leu Thr Asp Arg Pro Leu Thr Lys
               340                 345                 350
   Leu Trp Gly Glu Gln Gly Ser Asn Asp Phe Ala Gly Gly Val Ser Asp
           355                 360                 365
   Gly Val Tyr Gly Ala Ser Ala Tyr Ala Leu Asp Tyr Asp Ser Leu Gln
       370                 375                 380
   Ala Lys Lys Ala Trp Phe Phe Phe Asp Lys Glu Ile Val Cys Leu Gly
   385                 390                 395                 400
   Ala Gly Ile Asn Ser Asn Ala Pro Glu Asn Ile Thr Thr Thr Leu Asn
                   405                 410                 415
   Gln Ser Trp Leu Asn Gly Pro Val Ile Ser Thr
               420                 425
<210> 10
   <211> 378
   <212> PRT
   <213> Artificial
<220>
   <223> Synthetic polypeptide, N(delta)100 C(delta)200 of chondroitinase AC (S[sub]123 - T[sub] 500)
<400> 10
   Ser Arg Asn Trp Trp His Asn Glu Ile Ala Thr Pro Gln Ala Leu Gly
   1 5 10 15
   Glu Met Leu Ile Leu Met Arg Tyr Gly Lys Lys Pro Leu Asp Glu Ala
               20                  25                  30
   Leu Val His Lys Leu Thr Glu Arg Met Lys Arg Gly Glu Pro Glu Lys
           35                  40                  45
   Lys Thr Gly Ala Asn Lys Thr Asp Ile Ala Leu His Tyr Phe Tyr Arg
       50                  55                  60
   Ala Leu Leu Thr Ser Asp Glu Ala Leu Leu Ser Phe Ala Val Lys Glu
   65                  70                  75                  80
   Leu Phe Tyr Pro Val Gln Phe Val His Tyr Glu Glu Gly Leu Gln Tyr
                   85                  90                  95
   Asp Tyr Ser Tyr Leu Gln His Gly Pro Gln Leu Gln Ile Ser Ser Tyr
               100                 105                 110
   Gly Ala Val Phe Ile Thr Gly Val Leu Lys Leu Ala Asn Tyr Val Arg
           115                 120                 125
   Asp Thr Pro Tyr Ala Leu Ser Thr Glu Lys Leu Ala Ile Phe Ser Lys
       130                 135                 140
   Tyr Tyr Arg Asp Ser Tyr Leu Lys Ala Ile Arg Gly Ser Tyr Met Asp
   145                 150                 155                 160
   Phe Asn Val Glu Gly Arg Gly Val Ser Arg Pro Asp Ile Leu Asn Lys
                   165                 170                 175
   Lys Ala Glu Lys Lys Arg Leu Leu Val Ala Lys Met Ile Asp Leu Lys
               180                 185                 190
   His Thr Glu Glu Trp Ala Asp Ala Ile Ala Arg Thr Asp Ser Thr Val
           195                 200                 205
   Ala Ala Gly Tyr Lys Ile Glu Pro Tyr His His Gln Phe Trp Asn Gly
       210                 215                 220
   Asp Tyr Val Gln His Leu Arg Pro Ala Tyr Ser Phe Asn Val Arg Met
   225                 230                 235                 240
   Val Ser Lys Arg Thr Arg Arg Ser Glu Ser Gly Asn Lys Glu Asn Leu
                   245                 250                 255
   Leu Gly Arg Tyr Leu Ser Asp Gly Ala Thr Asn Ile Gln Leu Arg Gly
               260                 265                 270
   Pro Glu Tyr Tyr Asn Ile Met Pro Val Trp Glu Trp Asp Lys Ile Pro
           275                 280                 285
   Gly Ile Thr Ser Arg Asp Tyr Leu Thr Asp Arg Pro Leu Thr Lys Leu
       290                 295                 300
   Trp Gly Glu Gln Gly Ser Asn Asp Phe Ala Gly Gly Val Ser Asp Gly
   305                 310                 315                 320
   Val Tyr Gly Ala Ser Ala Tyr Ala Leu Asp Tyr Asp Ser Leu Gln Ala
                   325                 330                 335
   Lys Lys Ala Trp Phe Phe Phe Asp Lys Glu Ile Val Cys Leu Gly Ala
               340                 345                 350
   Gly Ile Asn Ser Asn Ala Pro Glu Asn Ile Thr Thr Thr Leu Asn Gln
           355                 360                 365
   Ser Trp Leu Asn Gly Pro Val Ile Ser Thr
       370                 375
<210> 11
   <211> 353
   <212> PRT
   <213> Artificial
<220>
   <223> Synthetic polypeptide, N(delta)50 C(delta)275 of chondroitinase AC (T[sub]74 - L[sub]426)
<400> 11
   Thr Asn Trp Leu Pro Asn Asn His Leu Leu Gln Leu Glu Thr Ile Ile
   1               5                   10                  15
   Gln Ala Tyr Ile Glu Lys Asp Ser His Tyr Tyr Gly Asp Asp Lys Val
               20                  25                  30
   Phe Asp Gln Ile Ser Lys Ala Phe Lys Tyr Trp Tyr Asp Ser Asp Pro
           35                  40                  45
   Lys Ser Arg Asn Trp Trp His Asn Glu Ile Ala Thr Pro Gln Ala Leu
       50                  55                  60
   Gly Glu Met Leu Ile Leu Met Arg Tyr Gly Lys Lys Pro Leu Asp Glu
   65                  70                  75                  80
   Ala Leu Val His Lys Leu Thr Glu Arg Met Lys Arg Gly Glu Pro Glu
                   85                  90                  95
   Lys Lys Thr Gly Ala Asn Lys Thr Asp Ile Ala Leu His Tyr Phe Tyr
               100                 105                 110
   Arg Ala Leu Leu Thr Ser Asp Glu Ala Leu Leu Ser Phe Ala Val Lys
           115                 120                 125
   Glu Leu Phe Tyr Pro Val Gln Phe Val His Tyr Glu Glu Gly Leu Gln
   130 135 140
   Tyr Asp Tyr Ser Tyr Leu Gln His Gly Pro Gln Leu Gln Ile Ser Ser
   145 150 155 160
   Tyr Gly Ala Val Phe Ile Thr Gly Val Leu Lys Leu Ala Asn Tyr Val
   165 170 175
   Arg Asp Thr Pro Tyr Ala Leu Ser Thr Glu Lys Leu Ala Ile Phe Ser
   180 185 190
   Lys Tyr Tyr Arg Asp Ser Tyr Leu Lys Ala Ile Arg Gly Ser Tyr Met
   195 200 205
   Asp Phe Asn Val Glu Gly Arg Gly Val Ser Arg Pro Asp Ile Leu Asn
   210 215 220
   Lys Lys Ala Glu Lys Lys Arg Leu Leu Val Ala Lys Met Ile Asp Leu
   225 230 235 240
   Lys His Thr Glu Glu Trp Ala Asp Ala Ile Ala Arg Thr Asp Ser Thr
   245 250 255
   Val Ala Ala Gly Tyr Lys Ile Glu Pro Tyr His His Gln Phe Trp Asn
   260 265 270
   Gly Asp Tyr Val Gln His Leu Arg Pro Ala Tyr Ser Phe Asn Val Arg
   275 280 285
   Met Val Ser Lys Arg Thr Arg Arg Ser Glu Ser Gly Asn Lys Glu Asn
   290 295 300
   Leu Leu Gly Arg Tyr Leu Ser Asp Gly Ala Thr Asn Ile Gln Leu Arg
   305 310 315 320
   Gly Pro Glu Tyr Tyr Asn Ile Met Pro Val Trp Glu Trp Asp Lys Ile
   325 330 335
   Pro Gly Ile Thr Ser Arg Asp Tyr Leu Thr Asp Arg Pro Leu Thr Lys
   340 345 350
   Leu
<210> 12
   <211> 506
   <212> PRT
   <213> Pedobacter heparinus
<220>
   <223> LOCUS (Q46079), Chondroitinase B precursor (Chondroitinase B lyase) (chondroitin sulfate B lyase)
<400> 12
   Met Lys Met Leu Asn Lys Leu Ala Gly Tyr Leu Leu Pro Ile Met Val
   1               5                   10                  15
   Leu Leu Asn Val Ala Pro Cys Leu Gly Gln Val Val Ala Ser Asn Glu
               20                  25                  30
   Thr Leu Tyr Gln Val Val Lys Glu Val Lys Pro Gly Gly Leu Val Gln
           35                  40                  45
   Ile Ala Asp Gly Thr Tyr Lys Asp Val Gln Leu Ile Val Ser Asn Ser
       50                  55                  60
   Gly Lys Ser Gly Leu Pro Ile Thr Ile Lys Ala Leu Asn Pro Gly Lys
   65                  70                  75                  80
   Val Phe Phe Thr Gly Asp Ala Lys Val Glu Leu Arg Gly Glu His Leu
                   85                  90                  95
   Ile Leu Glu Gly Ile Trp Phe Lys Asp Gly Asn Arg Ala Ile Gln Ala
               100                 105                 110
   Trp Lys Ser His Gly Pro Gly Leu Val Ala Ile Tyr Gly Ser Tyr Asn
           115                 120                 125
   Arg Ile Thr Ala Cys Val Phe Asp Cys Phe Asp Glu Ala Asn Ser Ala
       130                 135                 140
   Tyr Ile Thr Thr Ser Leu Thr Glu Asp Gly Lys Val Pro Gln His Cys
   145                 150                 155                 160
   Arg Ile Asp His Cys Ser Phe Thr Asp Lys Ile Thr Phe Asp Gln Val
                   165                 170                 175
   Ile Asn Leu Asn Asn Thr Ala Arg Ala Ile Lys Asp Gly Ser Val Gly
               180                 185                 190
   Gly Pro Gly Met Tyr His Arg Val Asp His Cys Phe Phe Ser Asn Pro
           195                 200                 205
   Gln Lys Pro Gly Asn Ala Gly Gly Gly Ile Arg Ile Gly Tyr Tyr Arg
       210                 215                 220
   Asn Asp Ile Gly Arg Cys Leu Val Asp Ser Asn Leu Phe Met Arg Gln
   225                 230                 235                 240
   Asp Ser Glu Ala Glu Ile Ile Thr Ser Lys Ser Gln Glu Asn Val Tyr
                   245                 250                 255
   Tyr Gly Asn Thr Tyr Leu Asn Cys Gln Gly Thr Met Asn Phe Arg His
               260                 265                 270
   Gly Asp His Gln Val Ala Ile Asn Asn Phe Tyr Ile Gly Asn Asp Gln
   275 280 285
   Arg Phe Gly Tyr Gly Gly Met Phe Val Trp Gly Ser Arg His Val Ile
   290 295 300
   Ala Cys Asn Tyr Phe Glu Leu Ser Glu Thr Ile Lys Ser Arg Gly Asn
   305 310 315 320
   Ala Ala Leu Tyr Leu Asn Pro Gly Ala Met Ala Ser Glu His Ala Leu
   325 330 335
   Ala Phe Asp Met Leu Ile Ala Asn Asn Ala Phe Ile Asn Val Asn Gly
   340 345 350
   Tyr Ala Ile His Phe Asn Pro Leu Asp Glu Arg Arg Lys Glu Tyr Cys
   355 360 365
   Ala Ala Asn Arg Leu Lys Phe Glu Thr Pro His Gln Leu Met Leu Lys
   370 375 380
   Gly Asn Leu Phe Phe Lys Asp Lys Pro Tyr Val Tyr Pro Phe Phe Lys
   385 390 395 400
   Asp Asp Tyr Phe Ile Ala Gly Lys Asn Ser Trp Thr Gly Asn Val Ala
   405 410 415
   Leu Gly Val Glu Lys Gly Ile Pro Val Asn Ile Ser Ala Asn Arg Ser
   420 425 430
   Ala Tyr Lys Pro Val Lys Ile Lys Asp Ile Gln Pro Ile Glu Gly Ile
   435 440 445
   Ala Leu Asp Leu Asn Ala Leu Ile Ser Lys Gly Ile Thr Gly Lys Pro
   450 455 460
   Leu Ser Trp Asp Glu Val Arg Pro Tyr Trp Leu Lys Glu Met Pro Gly
   465 470 475 480
   Thr Tyr Ala Leu Thr Ala Arg Leu Ser Ala Asp Arg Ala Ala Lys Phe
   485 490 495
   Lys Ala Val Ile Lys Arg Asn Lys Glu His
   500 505
<210> 13
   <211> 401
   <212> PRT
   <213> Artificial
<220>
   <223> Synthetic polypeptide, N(delta)80 chondroitinase B (G[sub]106 - H[sub]506)
<400> 13
   Gly Asn Arg Ala Ile Gln Ala Trp Lys Ser His Gly Pro Gly Leu Val
   1               5                   10                  15
   Ala Ile Tyr Gly Ser Tyr Asn Arg Ile Thr Ala Cys Val Phe Asp Cys
               20                  25                  30
   Phe Asp Glu Ala Asn Ser Ala Tyr Ile Thr Thr Ser Leu Thr Glu Asp
           35                  40                  45
   Gly Lys Val Pro Gln His Cys Arg Ile Asp His Cys Ser Phe Thr Asp
       50                  55                  60
   Lys Ile Thr Phe Asp Gln Val Ile Asn Leu Asn Asn Thr Ala Arg Ala
   65                  70                  75                  80
   Ile Lys Asp Gly Ser Val Gly Gly Pro Gly Met Tyr His Arg Val Asp
                   85                  90                  95
   His Cys Phe Phe Ser Asn Pro Gln Lys Pro Gly Asn Ala Gly Gly Gly
               100                 105                 110
   Ile Arg Ile Gly Tyr Tyr Arg Asn Asp Ile Gly Arg Cys Leu Val Asp
           115                 120                 125
   Ser Asn Leu Phe Met Arg Gln Asp Ser Glu Ala Glu Ile Ile Thr Ser
       130                 135                 140
   Lys Ser Gln Glu Asn Val Tyr Tyr Gly Asn Thr Tyr Leu Asn Cys Gln
   145                 150                 155                 160
   Gly Thr Met Asn Phe Arg His Gly Asp His Gln Val Ala Ile Asn Asn
                   165                 170                 175
   Phe Tyr Ile Gly Asn Asp Gln Arg Phe Gly Tyr Gly Gly Met Phe Val
               180                 185                 190
   Trp Gly Ser Arg His Val Ile Ala Cys Asn Tyr Phe Glu Leu Ser Glu
           195                 200                 205
   Thr Ile Lys Ser Arg Gly Asn Ala Ala Leu Tyr Leu Asn Pro Gly Ala
       210                 215                 220
   Met Ala Ser Glu His Ala Leu Ala Phe Asp Met Leu Ile Ala Asn Asn
   225                 230                 235                 240
   Ala Phe Ile Asn Val Asn Gly Tyr Ala Ile His Phe Asn Pro Leu Asp
                   245                 250                 255
   Glu Arg Arg Lys Glu Tyr Cys Ala Ala Asn Arg Leu Lys Phe Glu Thr
               260                 265                 270
   Pro His Gln Leu Met Leu Lys Gly Asn Leu Phe Phe Lys Asp Lys Pro
           275                 280                 285
   Tyr Val Tyr Pro Phe Phe Lys Asp Asp Tyr Phe Ile Ala Gly Lys Asn
       290                 295                 300
   Ser Trp Thr Gly Asn Val Ala Leu Gly Val Glu Lys Gly Ile Pro Val
   305                 310                 315                 320
   Asn Ile Ser Ala Asn Arg Ser Ala Tyr Lys Pro Val Lys Ile Lys Asp
                   325                 330                 335
   Ile Gln Pro Ile Glu Gly Ile Ala Leu Asp Leu Asn Ala Leu Ile Ser
               340                 345                 350
   Lys Gly Ile Thr Gly Lys Pro Leu Ser Trp Asp Glu Val Arg Pro Tyr
           355                 360                 365
   Trp Leu Lys Glu Met Pro Gly Thr Tyr Ala Leu Thr Ala Arg Leu Ser
       370                 375                 380
   Ala Asp Arg Ala Ala Lys Phe Lys Ala Val Ile Lys Arg Asn Lys Glu
   385                 390                 395                 400
   His
<210> 14
   <211> 361
   <212> PRT
   <213> Artificial
<220>
   <223> Synthetic polypeptide, N(delta)120 chondroitinase B (I[sub]146 - H[sub]506)
<400> 14
   Ile Thr Thr Ser Leu Thr Glu Asp Gly Lys Val Pro Gln His Cys Arg
   1               5                   10                  15
   Ile Asp His Cys Ser Phe Thr Asp Lys Ile Thr Phe Asp Gln Val Ile
               20                  25                  30
   Asn Leu Asn Asn Thr Ala Arg Ala Ile Lys Asp Gly Ser Val Gly Gly
           35                  40                  45
   Pro Gly Met Tyr His Arg Val Asp His Cys Phe Phe Ser Asn Pro Gln
       50                  55                  60
   Lys Pro Gly Asn Ala Gly Gly Gly Ile Arg Ile Gly Tyr Tyr Arg Asn
   65                  70                  75                  80
   Asp Ile Gly Arg Cys Leu Val Asp Ser Asn Leu Phe Met Arg Gln Asp
                   85                  90                  95
   Ser Glu Ala Glu Ile Ile Thr Ser Lys Ser Gln Glu Asn Val Tyr Tyr
               100                 105                 110
   Gly Asn Thr Tyr Leu Asn Cys Gln Gly Thr Met Asn Phe Arg His Gly
           115                 120                 125
   Asp His Gln Val Ala Ile Asn Asn Phe Tyr Ile Gly Asn Asp Gln Arg
       130                 135                 140
   Phe Gly Tyr Gly Gly Met Phe Val Trp Gly Ser Arg His Val Ile Ala
   145                 150                 155                 160
   Cys Asn Tyr Phe Glu Leu Ser Glu Thr Ile Lys Ser Arg Gly Asn Ala
                   165                 170                 175
   Ala Leu Tyr Leu Asn Pro Gly Ala Met Ala Ser Glu His Ala Leu Ala
               180                 185                 190
   Phe Asp Met Leu Ile Ala Asn Asn Ala Phe Ile Asn Val Asn Gly Tyr
           195                 200                 205
   Ala Ile His Phe Asn Pro Leu Asp Glu Arg Arg Lys Glu Tyr Cys Ala
       210                 215                 220
   Ala Asn Arg Leu Lys Phe Glu Thr Pro His Gln Leu Met Leu Lys Gly
   225                 230                 235                 240
   Asn Leu Phe Phe Lys Asp Lys Pro Tyr Val Tyr Pro Phe Phe Lys Asp
                   245                 250                 255
   Asp Tyr Phe Ile Ala Gly Lys Asn Ser Trp Thr Gly Asn Val Ala Leu
               260                 265                 270
   Gly Val Glu Lys Gly Ile Pro Val Asn Ile Ser Ala Asn Arg Ser Ala
           275                 280                 285
   Tyr Lys Pro Val Lys Ile Lys Asp Ile Gln Pro Ile Glu Gly Ile Ala
       290                 295                 300
   Leu Asp Leu Asn Ala Leu Ile Ser Lys Gly Ile Thr Gly Lys Pro Leu
   305                 310                 315                 320
   Ser Trp Asp Glu Val Arg Pro Tyr Trp Leu Lys Glu Met Pro Gly Thr
                   325                 330                 335
   Tyr Ala Leu Thr Ala Arg Leu Ser Ala Asp Arg Ala Ala Lys Phe Lys
               340                 345                 350
   Ala Val Ile Lys Arg Asn Lys Glu His
           355                 360
<210> 15
   <211> 463
   <212> PRT
   <213> Artificial
<220>
   <223> Synthetic polypeptide, C(delta)19 chondroitinase B (Q[sub]26 - L[sub]488)
<400> 15
   Gln Val Val Ala Ser Asn Glu Thr Leu Tyr Gln Val Val Lys Glu Val
   1               5                   10                  15
   Lys Pro Gly Gly Leu Val Gln Ile Ala Asp Gly Thr Tyr Lys Asp Val
               20                  25                  30
   Gln Leu Ile Val Ser Asn Ser Gly Lys Ser Gly Leu Pro Ile Thr Ile
           35                  40                  45
   Lys Ala Leu Asn Pro Gly Lys Val Phe Phe Thr Gly Asp Ala Lys Val
       50                  55                  60
   Glu Leu Arg Gly Glu His Leu Ile Leu Glu Gly Ile Trp Phe Lys Asp
   65                  70                  75                  80
   Gly Asn Arg Ala Ile Gln Ala Trp Lys Ser His Gly Pro Gly Leu Val
                   85                  90                  95
   Ala Ile Tyr Gly Ser Tyr Asn Arg Ile Thr Ala Cys Val Phe Asp Cys
               100                 105                 110
   Phe Asp Glu Ala Asn Ser Ala Tyr Ile Thr Thr Ser Leu Thr Glu Asp
           115                 120                 125
   Gly Lys Val Pro Gln His Cys Arg Ile Asp His Cys Ser Phe Thr Asp
       130                 135                 140
   Lys Ile Thr Phe Asp Gln Val Ile Asn Leu Asn Asn Thr Ala Arg Ala
   145                 150                 155                 160
   Ile Lys Asp Gly Ser Val Gly Gly Pro Gly Met Tyr His Arg Val Asp
                   165                 170                 175
   His Cys Phe Phe Ser Asn Pro Gln Lys Pro Gly Asn Ala Gly Gly Gly
               180                 185                 190
   Ile Arg Ile Gly Tyr Tyr Arg Asn Asp Ile Gly Arg Cys Leu Val Asp
           195                 200                 205
   Ser Asn Leu Phe Met Arg Gln Asp Ser Glu Ala Glu Ile Ile Thr Ser
       210                 215                 220
   Lys Ser Gln Glu Asn Val Tyr Tyr Gly Asn Thr Tyr Leu Asn Cys Gln
   225                 230                 235                 240
   Gly Thr Met Asn Phe Arg His Gly Asp His Gln Val Ala Ile Asn Asn
                   245                 250                 255
   Phe Tyr Ile Gly Asn Asp Gln Arg Phe Gly Tyr Gly Gly Met Phe Val
               260                 265                 270
   Trp Gly Ser Arg His Val Ile Ala Cys Asn Tyr Phe Glu Leu Ser Glu
           275                 280                 285
   Thr Ile Lys Ser Arg Gly Asn Ala Ala Leu Tyr Leu Asn Pro Gly Ala
       290                 295                 300
   Met Ala Ser Glu His Ala Leu Ala Phe Asp Met Leu Ile Ala Asn Asn
   305                 310                 315                 320
   Ala Phe Ile Asn Val Asn Gly Tyr Ala Ile His Phe Asn Pro Leu Asp
                   325                 330                 335
   Glu Arg Arg Lys Glu Tyr Cys Ala Ala Asn Arg Leu Lys Phe Glu Thr
               340                 345                 350
   Pro His Gln Leu Met Leu Lys Gly Asn Leu Phe Phe Lys Asp Lys Pro
           355                 360                 365
   Tyr Val Tyr Pro Phe Phe Lys Asp Asp Tyr Phe Ile Ala Gly Lys Asn
       370                 375                 380
   Ser Trp Thr Gly Asn Val Ala Leu Gly Val Glu Lys Gly Ile Pro Val
   385                 390                 395                 400
   Asn Ile Ser Ala Asn Arg Ser Ala Tyr Lys Pro Val Lys Ile Lys Asp
                   405                 410                 415
   Ile Gln Pro Ile Glu Gly Ile Ala Leu Asp Leu Asn Ala Leu Ile Ser
               420                 425                 430
   Lys Gly Ile Thr Gly Lys Pro Leu Ser Trp Asp Glu Val Arg Pro Tyr
           435                 440                 445
   Trp Leu Lys Glu Met Pro Gly Thr Tyr Ala Leu Thr Ala Arg Leu
       450                 455                 460
<210> 16
   <211> 365
   <212> PRT
   <213> Artificial
<220>
   <223> Synthetic polypeptide, C(delta)120 chondroitinase B (Q[sub]26 - K[sub]390)
<400> 16
   Gln Val Val Ala Ser Asn Glu Thr Leu Tyr Gln Val Val Lys Glu Val
   1               5                   10                  15
   Lys Pro Gly Gly Leu Val Gln Ile Ala Asp Gly Thr Tyr Lys Asp Val
               20                  25                  30
   Gln Leu Ile Val Ser Asn Ser Gly Lys Ser Gly Leu Pro Ile Thr Ile
           35                  40                  45
   Lys Ala Leu Asn Pro Gly Lys Val Phe Phe Thr Gly Asp Ala Lys Val
       50                  55                  60
   Glu Leu Arg Gly Glu His Leu Ile Leu Glu Gly Ile Trp Phe Lys Asp
   65                  70                  75                  80
   Gly Asn Arg Ala Ile Gln Ala Trp Lys Ser His Gly Pro Gly Leu Val
                   85                  90                  95
   Ala Ile Tyr Gly Ser Tyr Asn Arg Ile Thr Ala Cys Val Phe Asp Cys
               100                 105                 110
   Phe Asp Glu Ala Asn Ser Ala Tyr Ile Thr Thr Ser Leu Thr Glu Asp
           115                 120                 125
   Gly Lys Val Pro Gln His Cys Arg Ile Asp His Cys Ser Phe Thr Asp
       130                 135                 140
   Lys Ile Thr Phe Asp Gln Val Ile Asn Leu Asn Asn Thr Ala Arg Ala
   145                 150                 155                 160
   Ile Lys Asp Gly Ser Val Gly Gly Pro Gly Met Tyr His Arg Val Asp
                   165                 170                 175
   His Cys Phe Phe Ser Asn Pro Gln Lys Pro Gly Asn Ala Gly Gly Gly
               180                 185                 190
   Ile Arg Ile Gly Tyr Tyr Arg Asn Asp Ile Gly Arg Cys Leu Val Asp
           195                 200                 205
   Ser Asn Leu Phe Met Arg Gln Asp Ser Glu Ala Glu Ile Ile Thr Ser
       210                 215                 220
   Lys Ser Gln Glu Asn Val Tyr Tyr Gly Asn Thr Tyr Leu Asn Cys Gln
   225                 230                 235                 240
   Gly Thr Met Asn Phe Arg His Gly Asp His Gln Val Ala Ile Asn Asn
                   245                 250                 255
   Phe Tyr Ile Gly Asn Asp Gln Arg Phe Gly Tyr Gly Gly Met Phe Val
               260                 265                 270
   Trp Gly Ser Arg His Val Ile Ala Cys Asn Tyr Phe Glu Leu Ser Glu
               275                 280                 285
   Thr Ile Lys Ser Arg Gly Asn Ala Ala Leu Tyr Leu Asn Pro Gly Ala
       290                 295                 300
   Met Ala Ser Glu His Ala Leu Ala Phe Asp Met Leu Ile Ala Asn Asn
   305                 310                 315                 320
   Ala Phe Ile Asn Val Asn Gly Tyr Ala Ile His Phe Asn Pro Leu Asp
                   325                 330                 335
   Glu Arg Arg Lys Glu Tyr Cys Ala Ala Asn Arg Leu Lys Phe Glu Thr
               340                 345                 350
   Pro His Gln Leu Met Leu Lys Gly Asn Leu Phe Phe Lys
           355                 360                 365
<210> 17
   <211> 245
   <212> PRT
   <213> Artificial
<220>
   <223> Synthetic polypeptide, N(delta)120 C(delta)120 chondroitinase B (I[sub]146 - K[sub]390)
<400> 17
   Ile Thr Thr Ser Leu Thr Glu Asp Gly Lys Val Pro Gln His Cys Arg
   1               5                   10                  15
   Ile Asp His Cys Ser Phe Thr Asp Lys Ile Thr Phe Asp Gln Val Ile
               20                  25                  30
   Asn Leu Asn Asn Thr Ala Arg Ala Ile Lys Asp Gly Ser Val Gly Gly
           35                  40                  45
   Pro Gly Met Tyr His Arg Val Asp His Cys Phe Phe Ser Asn Pro Gln
       50                  55                  60
   Lys Pro Gly Asn Ala Gly Gly Gly Ile Arg Ile Gly Tyr Tyr Arg Asn
   65                  70                  75                  80
   Asp Ile Gly Arg Cys Leu Val Asp Ser Asn Leu Phe Met Arg Gln Asp
                   85                  90                  95
   Ser Glu Ala Glu Ile Ile Thr Ser Lys Ser Gln Glu Asn Val Tyr Tyr
               100                 105                 110
   Gly Asn Thr Tyr Leu Asn Cys Gln Gly Thr Met Asn Phe Arg His Gly
           115                 120                 125
   Asp His Gln Val Ala Ile Asn Asn Phe Tyr Ile Gly Asn Asp Gln Arg
       130                 135                 140
   Phe Gly Tyr Gly Gly Met Phe Val Trp Gly Ser Arg His Val Ile Ala
   145                 150                 155                 160
   Cys Asn Tyr Phe Glu Leu Ser Glu Thr Ile Lys Ser Arg Gly Asn Ala
                   165                 170                 175
   Ala Leu Tyr Leu Asn Pro Gly Ala Met Ala Ser Glu His Ala Leu Ala
               180                 185                 190
   Phe Asp Met Leu Ile Ala Asn Asn Ala Phe Ile Asn Val Asn Gly Tyr
           195                 200                 205
   Ala Ile His Phe Asn Pro Leu Asp Glu Arg Arg Lys Glu Tyr Cys Ala
       210                 215                 220
   Ala Asn Arg Leu Lys Phe Glu Thr Pro His Gln Leu Met Leu Lys Gly
   225                 230                 235                 240
   Asn Leu Phe Phe Lys
                   245
<210> 18
   <211> 2103
   <212> DNA
   <213> Pedobacter heparinus
<220>
   <223> LOCUS (CHU27583) Pedobacter heparinus chondroitinase AC precursor (cslA) gene
<400> 18
   atgaagaaat tatttgtaac ctgtatagtc tttttctcta ttttaagtcc tgctctgctt     60
   attgcacagc agaccggtac tgcagaactg attatgaagc gggtgatgct ggaccttaaa    120
   aagcctttgc gcaatatgga taaggtggcg gaaaagaacc tgaatacgct gcagcctgac    180
   ggtagctgga aggatgtgcc ttataaagat gatgccatga ccaattggtt gccaaacaac    240
   cacctgctac aattggaaac tattatacag gcttatattg aaaaagatag tcactattat    300
   ggcgacgata aagtgtttga ccagatttcc aaagctttta agtattggta tgacagcgac    360
   ccgaaaagcc gcaactggtg gcacaatgaa attgccactc cgcaggccct tggtgaaatg    420
   ctgatcctga tgcgttacgg taaaaagccg cttgatgaag cattggtgca taaattgacc    480
   gaaagaatga agcggggcga accggagaag aaaacggggg ccaacaaaac agatatcgcc    540
   ctgcattact tttatcgtgc tttgttaacg tctgatgagg ctttgctttc cttcgccgta    600
   aaagaattgt tttatcccgt acagtttgta cactatgagg aaggcctgca atacgattat    660
   tcctacctgc agcacggtcc gcaattacag atatcgagct acggtgccgt atttattacc    720
   ggggtactga aacttgccaa ttacgttagg gatacccctt atgctttaag taccgagaaa    780
   ctggctatat tttcaaagta ttaccgcgac agttatctga aagctatccg tggaagttat    840
   atggatttta acgtagaagg ccgcggagta agccggccag acattctaaa taaaaaggca    900
   gaaaaaaaga ggttgctggt ggcgaagatg atcgatctta agcatactga agaatgggct 960
   gatgcgatag ccaggacaga tagcacagtt gcggccggct ataagattga gccctatcac 1020
   catcagttct ggaatggtga ttatgtgcaa catttaagac ctgcctattc ttttaatgtt 1080
   cgtatggtga gtaagcggac ccgacgcagt gaatccggca ataaagaaaa cctgctgggc 1140
   aggtatttat ctgatggggc tactaacata caattgcgcg gaccagaata ctataacatt 1200
   atgccggtat gggaatggga caagattcct ggcataacca gccgtgatta tttaaccgac 1260
   agacctttga cgaagctttg gggagagcag gggagcaatg actttgcagg aggggtgtct 1320
   gatggtgtat acggggccag tgcctacgca ttggattacg atagcttaca ggcaaagaaa 1380
   gcctggttct tttttgacaa agagattgta tgtcttggtg ccggtatcaa cagcaatgcc 1440
   cctgaaaaca ttaccactac ccttaaccag agctggttaa atggcccggt tataagtact 1500
   gcaggtaaaa ccggccgggg taaaataaca acgtttaaag cacagggaca gttctggttg 1560
   ttgcacgatg cgattggtta ttactttcct gaaggggcca accttagtct gagtacccag 1620
   tcgcaaaaag gcaattggtt ccacatcaac aattcacatt caaaagatga agtttctggt 1680
   gatgtattta agctttggat caaccatggt gccaggccag aaaatgcgca gtatgcttat 1740
   atcgttttgc cgggaataaa caagccggaa gaaattaaaa aatataatgg aacggcaccg 1800
   aaagtccttg ccaataccaa ccagctgcag gcagtttatc atcagcagtt agatatggta 1860
   caggctatct tctatacagc tggaaaatta agcgtagcgg gcatagaaat tgaaacagat 1920
   aagccatgtg cagtgctgat caagcacatc aatggcaagc aggtaatttg ggctgccgat 1980
   ccattgcaaa aagaaaagac tgcagtgttg agcatcaggg atttaaaaac aggaaaaaca 2040
   aatcgggtaa aaattgattt tccgcaacag gaatttgcag gtgcaacggt tgaactgaaa 2100
   tag 2103
<210> 19
   <211> 1067
   <212> DNA
   <213> Artificial
<220>
   <223> Synthetic polynucleotide of chondroitinase AC nucleic acid deletion N(delta)50 C(delta)275 (a[sub]220 - t[sub]1278)
<400> 19
   atgccatgac caattggttg ccaaacaacc acctgctaca attggaaact attatacagg 60
   cttatattga aaaagatagt cactattatg gcgacgataa agtgtttgac cagatttcca 120
   aagcttttaa gtattggtat gacagcgacc cgaaaagccg caactggtgg cacaatgaaa 180
   ttgccactcc gcaggccctt ggtgaaatgc tgatcctgat gcgttacggt aaaaagccgc 240
   ttgatgaagc attggtgcat aaattgaccg aaagaatgaa gcggggcgaa ccggagaaga 300
   aaacgggggc caacaaaaca gatatcgccc tgcattactt ttatcgtgct ttgttaacgt 360
   ctgatgaggc tttgctttcc ttcgccgtaa aagaattgtt ttatcccgta cagtttgtac 420
   actatgagga aggcctgcaa tacgattatt cctacctgca gcacggtccg caattacaga 480
   tatcgagcta cggtgccgta tttattaccg gggtactgaa acttgccaat tacgttaggg    540
   atacccctta tgctttaagt accgagaaac tggctatatt ttcaaagtat taccgcgaca    600
   gttatctgaa agctatccgt ggaagttata tggattttaa cgtagaaggc cgcggagtaa 660
   gccggccaga cattctaaat aaaaaggcag aaaaaaagag gttgctggtg gcgaagatga 720
   tcgatcttaa gcatactgaa gaatgggctg atgcgatagc caggacagat agcacagttg 780
   cggccggcta taagattgag ccctatcacc atcagttctg gaatggtgat tatgtgcaac 840
   atttaagacc tgcctattct tttaatgttc gtatggtgag taagcggacc cgacgcagtg 900
   aatccggcaa taaagaaaac ctgctgggca ggtatttatc tgatggggct actaacatac 960
   aattgcgcgg accagaatac tataacatta tgccggtatg ggaatgggac aagattcctg 1020
   gcataaccag ccgtgattat ttaaccgaca gacctttgac gaagctt 1067
<210> 20
   <211> 1521
   <212> DNA
   <213> Pedobacter Heparinus
<220>
   <223> synthetic polynucleotide, LOCUS (CHU27584) Pedobacter heparinus chondroitinase B precursor (csIB) gene
<400> 20
   atgaagatgc tgaataaact agccggatac ttattgccga tcatggtgct gctgaatgtg      60
   gcaccatgct taggtcaggt tgttgcttca aatgaaactt tataccaggt tgtaaaggag     120
   gtaaaacccg gtggtctggt acagattgcc gatgggactt ataaagatgt tcagctgatt     180
   gtcagcaatt caggaaaatc tggtttgccc atcactatta aagccctgaa cccgggtaag     240
   gtttttttta ccggagatgc taaagtagag ctgaggggcg agcacctgat actggaaggc     300
   atctggttta aagacgggaa cagagctatt caggcatgga aatcacatgg acccggattg     360
   gtggctatat atggtagcta taaccgcatt accgcatgtg tatttgattg ttttgatgaa     420
   gccaattctg cttacattac tacttcgctt accgaagacg gaaaggtacc tcaacattgc     480
   cgcatagacc attgcagttt taccgataag atcacttttg accaggtaat taacctgaac     540
   aatacagcca gagctattaa agacggttcg gtgggaggac cggggatgta ccatcgtgtt     600
   gatcactgtt ttttttccaa tccgcaaaaa ccgggtaatg ccggaggggg aatcaggatt     660
   ggctattacc gtaatgatat aggccgttgt ctggtagact ctaacctgtt tatgcgtcag     720
   gattcggaag cagagatcat caccagcaaa tcgcaggaaa atgtttatta tggtaatact     780
   tacctgaatt gccagggcac catgaacttt cgtcacggtg atcatcaggt ggccattaac     840
   aatttttata taggcaatga ccagcgattt ggatacgggg gaatgtttgt ttggggaagc     900
   aggcatgtca tagcctgtaa ttattttgag ctgtccgaaa ccataaagtc gagggggaac     960
   gccgcattgt atttaaaccc cggtgctatg gcttcggagc atgctcttgc tttcgatatg    1020
   ttgatagcca acaacgcttt catcaatgta aatgggtatg ccatccattt taatccattg    1080
   gatgagcgca gaaaagaata ttgtgcagcc aataggctta agttcgaaac cccgcaccag    1140
   ctaatgttaa aaggcaatct tttctttaag gataaacctt atgtttaccc attttttaaa     1200
   gatgattatt ttatagcagg gaaaaatagc tggactggta atgtagcctt aggtgtggaa    1260
   aagggaatcc ctgttaacat ttcggccaat aggtctgcct ataagccggt aaaaattaaa    1320
   gatatccagc ccatagaagg aatcgctctt gatctcaatg cgctgatcag caaaggcatt    1380
   acaggaaagc cccttagctg ggatgaagta aggccctact ggttaaaaga aatgcccggg    1440
   acgtatgctt taacggccag gctttctgca gatagggctg caaagtttaa agccgtaatt    1500
   aaaagaaata aagagcactg a                                               1521
<210> 21
   <211> 735
   <212> DNA
   <213> Artificial
<220>
   <223> Synthetic polynucleotide of chondroitinase B nucleic acid deletion N(delta)120 C(delta)120 (a[sub]436 - g[sub]1170)
<400> 21
   attactactt cgcttaccga agacggaaag gtacctcaac attgccgcat agaccattgc 60
   agttttaccg ataagatcac ttttgaccag gtaattaacc tgaacaatac agccagagct 120
   attaaagacg gttcggtggg aggaccgggg atgtaccatc gtgttgatca ctgttttttt 180
   tccaatccgc aaaaaccggg taatgccgga gggggaatca ggattggcta ttaccgtaat 240
   gatataggcc gttgtctggt agactctaac ctgtttatgc gtcaggattc ggaagcagag 300
   atcatcacca gcaaatcgca ggaaaatgtt tattatggta atacttacct gaattgccag 360
   ggcaccatga actttcgtca cggtgatcat caggtggcca ttaacaattt ttatataggc 420
   aatgaccagc gatttggata cgggggaatg tttgtttggg gaagcaggca tgtcatagcc 480
   tgtaattatt ttgagctgtc cgaaaccata aagtcgaggg ggaacgccgc attgtattta 540
   aaccccggtg ctatggcttc ggagcatgct cttgctttcg atatgttgat agccaacaac 600
   gctttcatca atgtaaatgg gtatgccatc cattttaatc cattggatga gcgcagaaaa 660
   gaatattgtg cagccaatag gcttaagttc gaaaccccgc accagctaat gttaaaaggc 720
   aatcttttct ttaag 735
<210> 22
   <211> 3980
   <212> DNA
   <213> Artificial
<220>
   <223> Synthetic polynucleotide LOCUS (I29953)
<400> 22
   ggaattccat cactcaatca ttaaatttag gcacaacgat gggctatcag cgttatgaca 60
   aatttaatga aggacgcatt ggtttcactg ttagccagcg tttctaagga gaaaaataat 120
   gccgatattt cgttttactg cacttgcaat gacattgggg ctattatcag cgccttataa     180
   cgcgatggca gccaccagca atcctgcatt tgatcctaaa aatctgatgc agtcagaaat     240
   ttaccatttt gcacaaaata acccattagc agacttctca tcagataaaa actcaatact     300
   aacgttatct gataaacgta gcattatggg aaaccaatct cttttatgga aatggaaagg     360
   tggtagtagc tttactttac ataaaaaact gattgtcccc accgataaag aagcatctaa     420
   agcatgggga cgctcatcta cccccgtttt ctcattttgg ctttacaatg aaaaaccgat     480
   tgatggttat cttactatcg atttcggaga aaaactcatt tcaaccagtg aggctcaggc     540
   aggctttaaa gtaaaattag atttcactgg ctggcgtgct gtgggagtct ctttaaataa     600
   cgatcttgaa aatcgagaga tgaccttaaa tgcaaccaat acctcctctg atggtactca     660
   agacagcatt gggcgttctt taggtgctaa agtcgatagt attcgtttta aagcgccttc     720
   taatgtgagt cagggtgaaa tctatatcga ccgtattatg ttttctgtcg atgatgctcg     780
   ctaccaatgg tctgattatc aagtaaaaac tcgcttatca gaacctgaaa ttcaatttca     840
   caacgtaaag ccacaactac ctgtaacacc tgaaaattta gcggccattg atcttattcg     900
   ccaacgtcta attaatgaat ttgtcggagg tgaaaaagag acaaacctcg cattagaaga     960
   gaatatcagc aaattaaaaa gtgatttcga tgctcttaat attcacactt tagcaaatgg    1020
   tggaacgcaa ggcagacatc tgatcactga taaacaaatc attatttatc aaccagagaa    1080
   tcttaactcc caagataaac aactatttga taattatgtt attttaggta attacacgac    1140
   attaatgttt aatattagcc gtgcttatgt gctggaaaaa gatcccacac aaaaggcgca    1200
   actaaagcag atgtacttat taatgacaaa gcatttatta gatcaaggct ttgttaaagg    1260
   gagtgcttta gtgacaaccc atcactgggg atacagttct cgttggtggt atatttccac    1320
   gttattaatg tctgatgcac taaaagaagc gaacctacaa actcaagttt atgattcatt    1380
   actgtggtat tcacgtgagt ttaaaagtag ttttgatatg aaagtaagtg ctgatagctc    1440
   tgatctagat tatttcaata ccttatctcg ccaacattta gccttattat tactagagcc    1500
   tgatgatcaa aagcgtatca acttagttaa tactttcagc cattatatca ctggcgcatt    1560
   aacgcaagtg ccaccgggtg gtaaagatgg tttacgccct gatggtacag catggcgaca    1620
   tgaaggcaac tatccgggct actctttccc agcctttaaa aatgcctctc agcttattta    1680
   tttattacgc gatacaccat tttcagtggg tgaaagtggt tggaataacc tgaaaaaagc    1740
   gatggtttca gcgtggatct acagtaatcc agaagttgga ttaccgcttg caggaagaca    1800
   cccttttaac tcaccttcgt taaaatcagt cgctcaaggc tattactggc ttgccatgtc    1860
   tgcaaaatca tcgcctgata aaacacttgc atctatttat cttgcgatta gtgataaaac    1920
   acaaaatgaa tcaactgcta tttttggaga aactattaca ccagcgtctt tacctcaagg    1980
   tttctatgcc tttaatggcg gtgcttttgg tattcatcgt tggcaagata aaatggtgac    2040
   actgaaagct tataacacca atgtttggtc atctgaaatt tataacaaag ataaccgtta    2100
   tggccgttac caaagtcatg gtgtcgctca aatagtgagt aatggctcgc agctttcaca    2160
   gggctatcag caagaaggtt gggattggaa tagaatgcaa ggggcaacca ctattcacct 2220
   tcctcttaaa gacttagaca gtcctaaacc tcatacctta atgcaacgtg gagagcgtgg 2280
   atttagcgga acatcatccc ttgaaggtca atatggcatg atggcattcg atcttattta 2340
   tcccgccaat cttgagcgtt ttgatcctaa tttcactgcg aaaaagagtg tattagccgc 2400
   tgataatcac ttaattttta ttggtagcaa tataaatagt agtgataaaa ataaaaatgt 2460
   tgaaacgacc ttattccaac atgccattac tccaacatta aatacccttt ggattaatgg 2520
   acaaaagata gaaaacatgc cttatcaaac aacacttcaa caaggtgatt ggttaattga 2580
   tagcaatggc aatggttact taattactca agcagaaaaa gtaaatgtaa gtcgccaaca 2640
   tcaggtttca gcggaaaata aaaatcgcca accgacagaa ggaaacttta gctcggcatg 2700
   gatcgatcac agcactcgcc ccaaagatgc cagttatgag tatatggtct ttttagatgc 2760
   gacacctgaa aaaatgggag agatggcaca aaaattccgt gaaaataatg ggttatatca 2820
   ggttcttcgt aaggataaag acgttcatat tattctcgat aaactcagca atgtaacggg 2880
   atatgccttt tatcagccag catcaattga agacaaatgg atcaaaaagg ttaataaacc 2940
   tgcaattgtg atgactcatc gacaaaaaga cactcttatt gtcagtgcag ttacacctga 3000
   tttaaatatg actcgccaaa aagcagcaac tcctgtcacc atcaatgtca cgattaatgg 3060
   caaatggcaa tctgctgata aaaatagtga agtgaaatat caggtttctg gtgataacac 3120
   tgaactgacg tttacgagtt actttggtat tccacaagaa atcaaactct cgccactccc 3180
   ttgatttaat caaaagaacg ctcttgcgtt ccttttttat ttgcaggaaa tctgattatg 3240
   ctaataaaaa accctttagc ccacgcggtt acattaagcc tctgtttatc attacccgca 3300
   caagcattac ccactctgtc tcatgaagct ttcggcgata tttatctttt tgaaggtgaa 3360
   ttacccaata cccttaccac ttcaaataat aatcaattat cgctaagcaa acagcatgct 3420
   aaagatggtg aacaatcact caaatggcaa tatcaaccac aagcaacatt aacactaaat 3480
   aatattgtta attaccaaga tgataaaaat acagccacac cactcacttt tatgatgtgg 3540
   atttataatg aaaaacctca atcttcccca ttaacgttag catttaaaca aaataataaa 3600
   attgcactaa gttttaatgc tgaacttaat tttacggggt ggcgaggtat tgctgttcct 3660
   tttcgtgata tgcaaggctc tgcgacaggt caacttgatc aattagtgat caccgctcca 3720
   aaccaagccg gaacactctt ttttgatcaa atcatcatga gtgtaccgtt agacaatcgt 3780
   tgggcagtac ctgactatca aacaccttac gtaaataacg cagtaaacac gatggttagt 3840
   aaaaactgga gtgcattatt gatgtacgat cagatgtttc aagcccatta ccctacttta 3900
   aacttcgata ctgaatttcg cgatgaccaa acagaaatgg cttcgattta tcagcgcttt 3960
   gaatattatc aaggaattcc 3980
<210> 23
   <211> 3835
   <212> DNA
   <213> Artificial
<220>
   <223> Synthetic polynucleotide, TAT fusion chondroitinase ABCI nucleic acid
<400> 23
   ggtcgtaaaa agcgtcgtca acgtcgtcgt ggtggtggtg gtggtgccac cagcaatcct     60
   gcatttgatc ctaaaaatct gatgcagtca gaaatttacc attttgcaca aaataaccca    120
   ttagcagact tctcatcaga taaaaactca atactaacgt tatctgataa acgtagcatt    180
   atgggaaacc aatctctttt atggaaatgg aaaggtggta gtagctttac tttacataaa    240
   aaactgattg tccccaccga taaagaagca tctaaagcat ggggacgctc atctaccccc    300
   gttttctcat tttggcttta caatgaaaaa ccgattgatg gttatcttac tatcgatttc    360
   ggagaaaaac tcatttcaac cagtgaggct caggcaggct ttaaagtaaa attagatttc    420
   actggctggc gtgctgtggg agtctcttta aataacgatc ttgaaaatcg agagatgacc    480
   ttaaatgcaa ccaatacctc ctctgatggt actcaagaca gcattgggcg ttctttaggt    540
   gctaaagtcg atagtattcg ttttaaagcg ccttctaatg tgagtcaggg tgaaatctat    600
   atcgaccgta ttatgttttc tgtcgatgat gctcgctacc aatggtctga ttatcaagta    660
   aaaactcgct tatcagaacc tgaaattcaa tttcacaacg taaagccaca actacctgta    720
   acacctgaaa atttagcggc cattgatctt attcgccaac gtctaattaa tgaatttgtc    780
   ggaggtgaaa aagagacaaa cctcgcatta gaagagaata tcagcaaatt aaaaagtgat    840
   ttcgatgctc ttaatattca cactttagca aatggtggaa cgcaaggcag acatctgatc    900
   actgataaac aaatcattat ttatcaacca gagaatctta actcccaaga taaacaacta    960
   tttgataatt atgttatttt aggtaattac acgacattaa tgtttaatat tagccgtgct   1020
   tatgtgctgg aaaaagatcc cacacaaaag gcgcaactaa agcagatgta cttattaatg   1080
   acaaagcatt tattagatca aggctttgtt aaagggagtg ctttagtgac aacccatcac   1140
   tggggataca gttctcgttg gtggtatatt tccacgttat taatgtctga tgcactaaaa   1200
   gaagcgaacc tacaaactca agtttatgat tcattactgt ggtattcacg tgagtttaaa   1260
   agtagttttg atatgaaagt aagtgctgat agctctgatc tagattattt caatacctta   1320
   tctcgccaac atttagcctt attattacta gagcctgatg atcaaaagcg tatcaactta   1380
   gttaatactt tcagccatta tatcactggc gcattaacgc aagtgccacc gggtggtaaa   1440
   gatggtttac gccctgatgg tacagcatgg cgacatgaag gcaactatcc gggctactct   1500
   ttcccagcct ttaaaaatgc ctctcagctt atttatttat tacgcgatac accattttca   1560
   gtgggtgaaa gtggttggaa taacctgaaa aaagcgatgg tttcagcgtg gatctacagt   1620
   aatccagaag ttggattacc gcttgcagga agacaccctt ttaactcacc ttcgttaaaa   1680
   tcagtcgctc aaggctatta ctggcttgcc atgtctgcaa aatcatcgcc tgataaaaca   1740
   cttgcatcta tttatcttgc gattagtgat aaaacacaaa atgaatcaac tgctattttt   1800
   ggagaaacta ttacaccagc gtctttacct caaggtttct atgcctttaa tggcggtgct   1860
   tttggtattc atcgttggca agataaaatg gtgacactga aagcttataa caccaatgtt   1920
   tggtcatctg aaatttataa caaagataac cgttatggcc gttaccaaag tcatggtgtc 1980
   gctcaaatag tgagtaatgg ctcgcagctt tcacagggct atcagcaaga aggttgggat 2040
   tggaatagaa tgcaaggggc aaccactatt caccttcctc ttaaagactt agacagtcct 2100
   aaacctcata ccttaatgca acgtggagag cgtggattta gcggaacatc atcccttgaa 2160
   ggtcaatatg gcatgatggc attcgatctt atttatcccg ccaatcttga gcgttttgat 2220
   cctaatttca ctgcgaaaaa gagtgtatta gccgctgata atcacttaat ttttattggt 2280
   agcaatataa atagtagtga taaaaataaa aatgttgaaa cgaccttatt ccaacatgcc 2340
   attactccaa cattaaatac cctttggatt aatggacaaa agatagaaaa catgccttat 2400
   caaacaacac ttcaacaagg tgattggtta attgatagca atggcaatgg ttacttaatt 2460
   actcaagcag aaaaagtaaa tgtaagtcgc caacatcagg tttcagcgga aaataaaaat 2520
   cgccaaccga cagaaggaaa ctttagctcg gcatggatcg atcacagcac tcgccccaaa 2580
   gatgccagtt atgagtatat ggtcttttta gatgcgacac ctgaaaaaat gggagagatg 2640
   gcacaaaaat tccgtgaaaa taatgggtta tatcaggttc ttcgtaagga taaagacgtt 2700
   catattattc tcgataaact cagcaatgta acgggatatg ccttttatca gccagcatca 2760
   attgaagaca aatggatcaa aaaggttaat aaacctgcaa ttgtgatgac tcatcgacaa 2820
   aaagacactc ttattgtcag tgcagttaca cctgatttaa atatgactcg ccaaaaagca 2880
   gcaactcctg tcaccatcaa tgtcacgatt aatggcaaat ggcaatctgc tgataaaaat 2940
   agtgaagtga aatatcaggt ttctggtgat aacactgaac tgacgtttac gagttacttt 3000
   ggtattccac aagaaatcaa actctcgcca ctcccttgat ttaatcaaaa gaacgctctt 3060
   gcgttccttt tttatttgca ggaaatctga ttatgctaat aaaaaaccct ttagcccacg 3120
   cggttacatt aagcctctgt ttatcattac ccgcacaagc attacccact ctgtctcatg 3180
   aagctttcgg cgatatttat ctttttgaag gtgaattacc caataccctt accacttcaa 3240
   ataataatca attatcgcta agcaaacagc atgctaaaga tggtgaacaa tcactcaaat 3300
   ggcaatatca accacaagca acattaacac taaataatat tgttaattac caagatgata 3360
   aaaatacagc cacaccactc acttttatga tgtggattta taatgaaaaa cctcaatctt 3420
   ccccattaac gttagcattt aaacaaaata ataaaattgc actaagtttt aatgctgaac 3480
   ttaattttac ggggtggcga ggtattgctg ttccttttcg tgatatgcaa ggctctgcga 3540
   caggtcaact tgatcaatta gtgatcaccg ctccaaacca agccggaaca ctcttttttg 3600
   atcaaatcat catgagtgta ccgttagaca atcgttgggc agtacctgac tatcaaacac 3660
   cttacgtaaa taacgcagta aacacgatgg ttagtaaaaa ctggagtgca ttattgatgt 3720
   acgatcagat gtttcaagcc cattacccta ctttaaactt cgatactgaa tttcgcgatg 3780
   accaaacaga aatggcttcg atttatcagc gctttgaata ttatcaagga attcc 3835
<210> 24
   <211> 15
   <212> PRT
   <213> Artificial
<220>
   <223> Synthetic polypeptide, HIV TAT Sequence and Gly penta linker
<400> 24
   Gly Arg Lys Lys Arg Arg Gln Arg Arg Arg Gly Gly Gly Gly Gly
   1               5                   10                  15
<210> 25
   <211> 45
   <212> DNA
   <213> Artificial
<220>
   <223> Synthetic polynucleotide, HIV-1 Tat Sequence and Gly penta linker nucleic acid
<400> 25
   ggtcgtaaaa agcgtcgtca acgtcgtcgt ggtggtggtg gtggt 45
<210> 26
   <211> 2973
   <212> DNA
   <213> Artificial
<220>
   <223> Synthetic polynucleotide, Chondroitinase ABC II Nucleic acid
<400> 26
   ttacccactc tgtctcatga agctttcggc gatatttatc tttttgaagg cgaattaccc 60
   aatatcctta ccacttcaaa taataatcaa ttatcgctaa gcaaacagca tgctaaagat 120
   ggtgaacaat cactcaaatg gcaatatcaa ccacaagcaa cattaacact aaataatatt 180
   gttaattacc aagatgataa aaatacagcc acaccactca cttttatgat gtggatttat 240
   aatgaaaaac ctcaatcttc cccattaacg ttagcattta aacaaaataa taaaattgca 300
   ctaagtttta atgctgaact taattttacg gggtggcgag gtattgctgt tccttttcgt 360
   gatatgcaag gctctgcgac aggtcaactt gatcaattag tgatcaccgc tccaaaccaa 420
   gccggaacac tcttttttga tcaaatcatc atgagtgtac cgttagacaa tcgttgggca 480
   gtacctgact atcaaacacc ttacgtaaat aacgcagtaa acacgatggt tagtaaaaac 540
   tggagtgcat tattgatgta cgatcagatg tttcaagccc attaccctac tttaaacttc 600
   gatactgaat ttcgcgatga ccaaacagaa atggcttcga tttatcagcg ctttgaatat 660
   tatcaaggaa ttcgtagtga taaaaaaatt actccagata tgctagataa acatttagcg 720
   ttatgggaaa aattggggtt aacacaacac gctgatggct caatcacagg aaaagccctt 780
   gatcacccta accggcaaca ttttatgaaa gtcgaaggtg tatttagtga ggggactcaa 840
   aaagcattac ttgatgccaa tatgctaaga gatgtgggca aaacgcttct tcaaactgct 900
   atttacttgc gtagcgattc attatcagca actggtagaa aaaaattaga agagcgctat 960
   ttattaggta ctcgttatgt ccttgaacaa ggttttacac gaggaagtgg ttatcaaatt 1020
   attactcatg ttggttacca aaccagagaa ctttttgatg catggtttat tggccgtcat 1080
   gttcttgcaa aaaataacct tttagccccc actcaacaag ctatgatgtg gtacaacgcc    1140
   acaggacgta tttttgaaaa agataatgaa attgttgatg caaatgtcga tattctcaat    1200
   actcaattgc aatggatgat aaaaagctta ttgatgctac cggattatca acaacgtcaa    1260
   caagccttag cgcaactgca aagttggcta aataaaacca ttctaagctc aaaaggtgtt    1320
   gctggcggtt tcaaatctga tggttctatt tttcaccatt cacaacatta ccccgcttat    1380
   gctaaagatg catttggtgg tttagcaccc agtgtttatg cattaagtga ttcacctttt    1440
   cgcttatcta cttcagcaca tgagcattta aaagatgttt tgttaaaaat gcggatctac    1500
   accaaagaga cacaaattcc tgtggtatta agtggtcgtc atccaactgg gttgcataaa    1560
   atagggatcg cgccatttaa atggatggca ttagcaggaa ccccagatgg caaacaaaag    1620
   ttagatacca cattatccgc cgcttatgca aacttagaca acaaaacgca ttttgaaggc    1680
   attaacgctg aaagtgagcc agtcggcgca tgggcaatga attatgcatc aatggcaata    1740
   caacgaagag catcgaccca atcaccacaa caaagctggc tcgccatagc gcgcggtttt    1800
   agccgttatc ttgttggtaa tgaaagctat gaaaataaca accgttatgg tcgttattta    1860
   caatatggac aattggaaat tattccagct gatttaactc aatcagggtt tagccatgct    1920
   ggatgggatt ggaatagata tccaggtaca acaactattc atcttcccta taacgaactt    1980
   gaagcaaaac ttaatcaatt acctgctgca ggtattgaag aaatgttgct ttcaacagaa    2040
   agttactctg gtgcaaatac ccttaataat aacagtatgt ttgccatgaa attacacggt    2100
   cacagtaaat atcaacaaca aagcttaagg gcaaataaat cctatttctt atttgataat    2160
   agagttattg ctttaggctc aggtattgaa aatgatgata aacaacatac gaccgaaaca    2220
   acactattcc agtttgccgt ccctaaatta cagtcagtga tcattaatgg caaaaaggta    2280
   aatcaattag atactcaatt aactttaaat aatgcagata cattaattga tcctgccggc    2340
   aatttatata agctcactaa aggacaaact gtaaaattta gttatcaaaa acaacattca    2400
   cttgatgata gaaattcaaa accaacagaa caattatttg caacagctgt tatttctcat    2460
   ggtaaggcac cgagtaatga aaattatgaa tatgcaatag ctatcgaagc acaaaataat    2520
   aaagctccca aatacacagt attacaacat aatgatcagc tccatgcggt aaaagataaa    2580
   ataacccaag aagagggata tggttttttt gaagccacta agttaaaatc agcggatgca    2640
   acattattat ccagtgatgc gccggttatg gtcatggcta aaatacaaaa tcagcaatta    2700
   acattaagta ttgttaatcc tgatttaaat ttatatcaag gtagagaaaa agatcaattt    2760
   gatgataaag gtaatcaaat cgaagttagt gtttattctc gtcattggct tacagcagaa    2820
   tcgcaatcaa caaatagtac tattaccgta aaaggaatat ggaaattaac gacacctcaa    2880
   cccggtgtta ttattaagca ccacaataac aacactctta ttacgacaac aaccatacag    2940
   gcaacaccta ctgttattaa tttagttaag taa                                 2973
<210> 27
   <211> 990
   <212> PRT
   <213> Artificial
<220>
   <223> Synthetic polypeptide, Chondroitinase ABC II protein
<400> 27
   Leu Pro Thr Leu Ser His Glu Ala Phe Gly Asp Ile Tyr Leu Phe Glu
   1               5                   10                  15
   Gly Glu Leu Pro Asn Ile Leu Thr Thr Ser Asn Asn Asn Gln Leu Ser
               20                  25                  30
   Leu Ser Lys Gln His Ala Lys Asp Gly Glu Gln Ser Leu Lys Trp Gln
           35                  40                  45
   Tyr Gln Pro Gln Ala Thr Leu Thr Leu Asn Asn Ile Val Asn Tyr Gln
       50                  55                  60
   Asp Asp Lys Asn Thr Ala Thr Pro Leu Thr Phe Met Met Trp Ile Tyr
   65                  70                  75                  80
   Asn Glu Lys Pro Gln Ser Ser Pro Leu Thr Leu Ala Phe Lys Gln Asn
                   85                  90                  95
   Asn Lys Ile Ala Leu Ser Phe Asn Ala Glu Leu Asn Phe Thr Gly Trp
               100                 105                 110
   Arg Gly Ile Ala Val Pro Phe Arg Asp Met Gln Gly Ser Ala Thr Gly
           115                 120                 125
   Gln Leu Asp Gln Leu Val Ile Thr Ala Pro Asn Gln Ala Gly Thr Leu
       130                 135                 140
   Phe Phe Asp Gln Ile Ile Met Ser Val Pro Leu Asp Asn Arg Trp Ala
   145                 150                 155                 160
   Val Pro Asp Tyr Gln Thr Pro Tyr Val Asn Asn Ala Val Asn Thr Met
                   165                 170                 175
   Val Ser Lys Asn Trp Ser Ala Leu Leu Met Tyr Asp Gln Met Phe Gln
               180                 185                 190
   Ala His Tyr Pro Thr Leu Asn Phe Asp Thr Glu Phe Arg Asp Asp Gln
           195                 200                 205
   Thr Glu Met Ala Ser Ile Tyr Gln Arg Phe Glu Tyr Tyr Gln Gly Ile
       210                 215                 220
   Arg Ser Asp Lys Lys Ile Thr Pro Asp Met Leu Asp Lys His Leu Ala
   225                 230                 235                 240
   Leu Trp Glu Lys Leu Gly Leu Thr Gln His Ala Asp Gly Ser Ile Thr
                   245                 250                 255
   Gly Lys Ala Leu Asp His Pro Asn Arg Gln His Phe Met Lys Val Glu
               260                 265                 270
   Gly Val Phe Ser Glu Gly Thr Gln Lys Ala Leu Leu Asp Ala Asn Met
           275                 280                 285
   Leu Arg Asp Val Gly Lys Thr Leu Leu Gln Thr Ala Ile Tyr Leu Arg
       290                 295                 300
   Ser Asp Ser Leu Ser Ala Thr Gly Arg Lys Lys Leu Glu Glu Arg Tyr
   305                 310                 315                 320
   Leu Leu Gly Thr Arg Tyr Val Leu Glu Gln Gly Phe Thr Arg Gly Ser
                   325                 330                 335
   Gly Tyr Gln Ile Ile Thr His Val Gly Tyr Gln Thr Arg Glu Leu Phe
               340                 345                 350
   Asp Ala Trp Phe Ile Gly Arg His Val Leu Ala Lys Asn Asn Leu Leu
           355                 360                 365
   Ala Pro Thr Gln Gln Ala Met Met Trp Tyr Asn Ala Thr Gly Arg Ile
       370                 375                 380
   Phe Glu Lys Asp Asn Glu Ile Val Asp Ala Asn Val Asp Ile Leu Asn
   385                 390                 395                 400
   Thr Gln Leu Gln Trp Met Ile Lys Ser Leu Leu Met Leu Pro Asp Tyr
                   405                 410                 415
   Gln Gln Arg Gln Gln Ala Leu Ala Gln Leu Gln Ser Trp Leu Asn Lys
               420                 425                 430
   Thr Ile Leu Ser Ser Lys Gly Val Ala Gly Gly Phe Lys Ser Asp Gly
           435                 440                 445
   Ser Ile Phe His His Ser Gln His Tyr Pro Ala Tyr Ala Lys Asp Ala
       450                 455                 460
   Phe Gly Gly Leu Ala Pro Ser Val Tyr Ala Leu Ser Asp Ser Pro Phe
   465                 470                 475                 480
   Arg Leu Ser Thr Ser Ala His Glu His Leu Lys Asp Val Leu Leu Lys
                   485                 490                 495
   Met Arg Ile Tyr Thr Lys Glu Thr Gln Ile Pro Val Val Leu Ser Gly
               500                 505                 510
   Arg His Pro Thr Gly Leu His Lys Ile Gly Ile Ala Pro Phe Lys Trp
           515                 520                 525
   Met Ala Leu Ala Gly Thr Pro Asp Gly Lys Gln Lys Leu Asp Thr Thr
       530                 535                 540
   Leu Ser Ala Ala Tyr Ala Asn Leu Asp Asn Lys Thr His Phe Glu Gly
   545                 550                 555                 560
   Ile Asn Ala Glu Ser Glu Pro Val Gly Ala Trp Ala Met Asn Tyr Ala
                   565                 570                 575
   Ser Met Ala Ile Gln Arg Arg Ala Ser Thr Gln Ser Pro Gln Gln Ser
               580                 585                 590
   Trp Leu Ala Ile Ala Arg Gly Phe Ser Arg Tyr Leu Val Gly Asn Glu
           595                 600                 605
   Ser Tyr Glu Asn Asn Asn Arg Tyr Gly Arg Tyr Leu Gln Tyr Gly Gln
       610                 615                 620
   Leu Glu Ile Ile Pro Ala Asp Leu Thr Gln Ser Gly Phe Ser His Ala
   625                 630                 635                 640
   Gly Trp Asp Trp Asn Arg Tyr Pro Gly Thr Thr Thr Ile His Leu Pro
                   645                 650                 655
   Tyr Asn Glu Leu Glu Ala Lys Leu Asn Gln Leu Pro Ala Ala Gly Ile
               660                 665                 670
   Glu Glu Met Leu Leu Ser Thr Glu Ser Tyr Ser Gly Ala Asn Thr Leu
           675                 680                 685
   Asn Asn Asn Ser Met Phe Ala Met Lys Leu His Gly His Ser Lys Tyr
       690                 695                 700
   Gln Gln Gln Ser Leu Arg Ala Asn Lys Ser Tyr Phe Leu Phe Asp Asn
   705                 710                 715                 720
   Arg Val Ile Ala Leu Gly Ser Gly Ile Glu Asn Asp Asp Lys Gln His
                   725                 730                 735
   Thr Thr Glu Thr Thr Leu Phe Gln Phe Ala Val Pro Lys Leu Gln Ser
               740                 745                 750
   Val Ile Ile Asn Gly Lys Lys Val Asn Gln Leu Asp Thr Gln Leu Thr
           755                 760                 765
   Leu Asn Asn Ala Asp Thr Leu Ile Asp Pro Ala Gly Asn Leu Tyr Lys
       770                 775                 780
   Leu Thr Lys Gly Gln Thr Val Lys Phe Ser Tyr Gln Lys Gln His Ser
   785                 790                 795                 800
   Leu Asp Asp Arg Asn Ser Lys Pro Thr Glu Gln Leu Phe Ala Thr Ala
                   805                 810                 815
   Val Ile Ser His Gly Lys Ala Pro Ser Asn Glu Asn Tyr Glu Tyr Ala
               820                 825                 830
   Ile Ala Ile Glu Ala Gln Asn Asn Lys Ala Pro Lys Tyr Thr Val Leu
           835                 840                 845
   Gln His Asn Asp Gln Leu His Ala Val Lys Asp Lys Ile Thr Gln Glu
       850                 855                 860
   Glu Gly Tyr Gly Phe Phe Glu Ala Thr Lys Leu Lys Ser Ala Asp Ala
   865                 870                 875                 880
   Thr Leu Leu Ser Ser Asp Ala Pro Val Met Val Met Ala Lys Ile Gln
                   885                 890                 895
   Asn Gln Gln Leu Thr Leu Ser Ile Val Asn Pro Asp Leu Asn Leu Tyr
               900                 905                 910
   Gln Gly Arg Glu Lys Asp Gln Phe Asp Asp Lys Gly Asn Gln Ile Glu
           915                 920                 925
   Val Ser Val Tyr Ser Arg His Trp Leu Thr Ala Glu Ser Gln Ser Thr
       930                 935                 940
   Asn Ser Thr Ile Thr Val Lys Gly Ile Trp Lys Leu Thr Thr Pro Gln
   945                 950                 955                 960
   Pro Gly Val Ile Ile Lys His His Asn Asn Asn Thr Leu Ile Thr Thr
                   965                 970                 975
   Thr Thr Ile Gln Ala Thr Pro Thr Val Ile Asn Leu Val Lys
               980                 985                 990
<210> 28
   <211> 2994
   <212> DNA
   <213> Artificial
<220>
   <223> Synthetic polynucleotide for Chondroitinase ABCI
<220>
   <221> misc_feature
   <222> (1086)..(1086)
   <223> Unknown nucleotide
<400> 28
   gccaccagca atcctgcatt tgatcctaaa aatctgatgc agtcagaaat ttaccatttt 60
   gcacaaaata acccattagc agacttctca tcagataaaa actcaatact aacgttatct 120
   gataaacgta gcattatggg aaaccaatct cttttatgga aatggaaagg tggtagtagc 180
   tttactttac ataaaaaact gattgtcccc accgataaag aagcatctaa agcatgggga    240
   cgctcatcca cccccgtttt ctcattttgg ctttacaatg aaaaaccgat tgatggttat    300
   cttactatcg atttcggaga aaaactcatt tcaaccagtg aggctcaggc aggctttaaa    360
   gtaaaattag atttcactgg ctggcgtact gtgggagtct ctttaaataa cgatcttgaa    420
   aatcgagaga tgaccttaaa tgcaaccaat acctcctctg atggtactca agacagcatt    480
   gggcgttctt taggtgctaa agtcgatagt attcgtttta aagcgccttc taatgtgagt    540
   cagggtgaaa tctatatcga ccgtattatg ttttctgtcg atgatgctcg ctaccaatgg    600
   tctgattatc aagtaaaaac tcgcttatca gaacctgaaa ttcaatttca caacgtaaag    660
   ccacaactac ctgtaacacc tgaaaattta gcggccattg atcttattcg ccaacgtcta    720
   attaatgaat ttgtcggagg tgaaaaagag acaaacctcg cattagaaga gaatatcagc    780
   aaattaaaaa gtgatttcga tgctcttaat actcacactt tagcaaatgg tggaacgcaa    840
   ggcagacatc tgatcactga taaacaaatc attatttatc aaccagagaa tcttaactct    900
   caagataaac aactatttga taattatgtt attttaggta attacacgac attaatgttt    960
   aatattagcc gtgcttatgt gctggaaaaa gatcccacac aaaaggcgca actaaagcag   1020
   atgtacttat taatgacaaa gcatttatta gatcaaggct ttgttaaagg gagtgcttta   1080
   gtgacnaccc atcactgggg atacagttct cgttggtggt atatttccac gttattaatg   1140
   tctgatgcac taaaagaagc gaacctacaa actcaagttt atgattcatt actgtggtat   1200
   tcacgtgagt ttaaaagtag ttttgatatg aaagtaagtg ctgatagctc tgatctagat   1260
   tatttcaata ccttatctcg ccaacattta gccttattac tactagagcc tgatgatcaa   1320
   aagcgtatca acttagttaa tactttcagc cattatatca ctggcgcatt aacgcaagtg   1380
   ccaccgggtg gtaaagatgg tttacgccct gatggtacag catggcgaca tgaaggcaac   1440
   tatccgggct actctttccc agcctttaaa aatgcctctc agcttattta tttattacgc   1500
   gatacaccat tttcagtggg tgaaagtggt tggaatagcc tgaaaaaagc gatggtttca   1560
   gcgtggatct acagtaatcc agaagttgga ttaccgcttg caggaagaca ccctcttaac   1620
   tcaccttcgt taaaatcagt cgctcaaggc tattactggc ttgccatgtc tgcaaaatca   1680
   tcgcctgata aaacacttgc atctatttat cttgcgatta gtgataaaac acaaaatgaa   1740
   tcaactgcta tttttggaga aactattaca ccagcgtctt tacctcaagg tttctatgcc   1800
   tttaatggcg gtgcttttgg tattcatcgt tggcaagata aaatggtgac actgaaagct   1860
   tataacacca atgtttggtc atctgaaatt tataacaaag ataaccgtta tggccgttac   1920
   caaagtcatg gtgtcgctca aatagtgagt aatggctcgc agctttcaca gggctatcag   1980
   caagaaggtt gggattggaa tagaatgcca ggggcaacca ctatccacct tcctcttaaa   2040
   gacttagaca gtcctaaacc tcatacctta  atgcaacgtg gagagcgtgg atttagcgga   2100
   acatcatccc ttgaaggtca atatggcatg atggcattcg atcttattta tcccgccaat   2160
   cttgagcgtt ttgatcctaa tttcactgcg aaaaagagtg tattagccgc tgataatcac   2220
   ttaattttta ttggtagcaa tataaatagt agtgataaaa ataaaaatgt tgaaacgacc    2280
   ttattccaac atgccattac tccaacatta aatacccttt ggattaatgg acaaaagata   2340
   gaaaacatgc cttatcaaac aacacttcaa caaggtgatt ggttaattga tagcaatggc   2400
   aatggttact taattactca agcagaaaaa gtaaatgtaa gtcgccaaca tcaggtttca   2460
   gcggaaaata aaaatcgcca accgacagaa ggaaacttta gctcggcatg gatcgatcac   2520
   agcactcgcc ccaaagatgc cagttatgag tatatggtct ttttagatgc gacacctgaa   2580
   aaaatgggag agatggcaca aaaattccgt gaaaataatg ggttatatca ggttcttcgt   2640
   aaggataaag acgttcatat tattctcgat aaactcagca atgtaacggg atatgccttt   2700
   tatcagccag catcaattga agacaaatgg atcaaaaagg ttaataaacc tgcaattgtg   2760
   atgactcatc gacaaaaaga cactcttatt gtcagtgcag ttacacctga tttaaatatg   2820
   actcgccaaa aagcagcaac tcctgtcacc atcaatgtca cgattaatgg caaatggcaa   2880
   tctgctgata aaaatagtga agtgaaatat caggtttctg gtgataacac tgaactgacg   2940
   tttacgagtt actttggtat tccacaagaa atcaaactct cgccactccc ttga         2994
<210> 29
   <211> 997
   <212> PRT
   <213> Artificial
<220>
   <223> Synthetic polypeptide, Chondroitinase ABC I protein
<400> 29
   Ala Thr Ser Asn Pro Ala Phe Asp Pro Lys Asn Leu Met Gln Ser Glu
   1               5                   10                  15
   Ile Tyr His Phe Ala Gln Asn Asn Pro Leu Ala Asp Phe Ser Ser Asp
               20                  25                  30
   Lys Asn Ser Ile Leu Thr Leu Ser Asp Lys Arg Ser Ile Met Gly Asn
           35                  40                  45
   Gln Ser Leu Leu Trp Lys Trp Lys Gly Gly Ser Ser Phe Thr Leu His
       50                  55                  60
   Lys Lys Leu Ile Val Pro Thr Asp Lys Glu Ala Ser Lys Ala Trp Gly
   65                  70                  75                  80
   Arg Ser Ser Thr Pro Val Phe Ser Phe Trp Leu Tyr Asn Glu Lys Pro
                   85                  90                  95
   Ile Asp Gly Tyr Leu Thr Ile Asp Phe Gly Glu Lys Leu Ile Ser Thr
               100                 105                 110
   Ser Glu Ala Gln Ala Gly Phe Lys Val Lys Leu Asp Phe Thr Gly Trp
           115                 120                 125
   Arg Thr Val Gly Val Ser Leu Asn Asn Asp Leu Glu Asn Arg Glu Met
   130 135 140
   Thr Leu Asn Ala Thr Asn Thr Ser Ser Asp Gly Thr Gln Asp Ser Ile
   145 150 155 160
   Gly Arg Ser Leu Gly Ala Lys Val Asp Ser Ile Arg Phe Lys Ala Pro
   165 170 175
   Ser Asn Val Ser Gln Gly Glu Ile Tyr Ile Asp Arg Ile Met Phe Ser
   180 185 190
   Val Asp Asp Ala Arg Tyr Gln Trp Ser Asp Tyr Gln Val Lys Thr Arg
   195 200 205
   Leu Ser Glu Pro Glu Ile Gln Phe His Asn Val Lys Pro Gln Leu Pro
   210 215 220
   Val Thr Pro Glu Asn Leu Ala Ala Ile Asp Leu Ile Arg Gln Arg Leu
   225 230 235 240
   Ile Asn Glu Phe Val Gly Gly Glu Lys Glu Thr Asn Leu Ala Leu Glu
   245 250 255
   Glu Asn Ile Ser Lys Leu Lys Ser Asp Phe Asp Ala Leu Asn Thr His
   260 265 270
   Thr Leu Ala Asn Gly Gly Thr Gln Gly Arg His Leu Ile Thr Asp Lys
   275 280 285
   Gln Ile Ile Ile Tyr Gln Pro Glu Asn Leu Asn Ser Gln Asp Lys Gln
   290 295 300
   Leu Phe Asp Asn Tyr Val Ile Leu Gly Asn Tyr Thr Thr Leu Met Phe
   305 310 315 320
   Asn Ile Ser Arg Ala Tyr Val Leu Glu Lys Asp Pro Thr Gln Lys Ala
   325 330 335
   Gln Leu Lys Gln Met Tyr Leu Leu Met Thr Lys His Leu Leu Asp Gln
   340 345 350
   Gly Phe Val Lys Gly Ser Ala Leu Val Thr Thr His His Trp Gly Tyr
   355 360 365
   Ser Ser Arg Trp Trp Tyr Ile Ser Thr Leu Leu Met Ser Asp Ala Leu
   370 375 380
   Lys Glu Ala Asn Leu Gln Thr Gln Val Tyr Asp Ser Leu Leu Trp Tyr
   385 390 395 400
   Ser Arg Glu Phe Lys Ser Ser Phe Asp Met Lys Val Ser Ala Asp Ser
   405 410 415
   Ser Asp Leu Asp Tyr Phe Asn Thr Leu Ser Arg Gln His Leu Ala Leu
               420                 425                 430
   Leu Leu Leu Glu Pro Asp Asp Gln Lys Arg Ile Asn Leu Val Asn Thr
           435                 440                 445
   Phe Ser His Tyr Ile Thr Gly Ala Leu Thr Gln Val Pro Pro Gly Gly
       450                 455                 460
   Lys Asp Gly Leu Arg Pro Asp Gly Thr Ala Trp Arg His Glu Gly Asn
   465                 470                 475                 480
   Tyr Pro Gly Tyr Ser Phe Pro Ala Phe Lys Asn Ala Ser Gln Leu Ile
                   485                 490                 495
   Tyr Leu Leu Arg Asp Thr Pro Phe Ser Val Gly Glu Ser Gly Trp Asn
               500                 505                 510
   Asn Leu Lys Lys Ala Met Val Ser Ala Trp Ile Tyr Ser Asn Pro Glu
           515                 520                 525
   Val Gly Leu Pro Leu Ala Gly Arg His Pro Phe Asn Ser Pro Ser Leu
       530                 535                 540
   Lys Ser Val Ala Gln Gly Tyr Tyr Trp Leu Ala Met Ser Ala Lys Ser
   545                 550                 555                 560
   Ser Pro Asp Lys Thr Leu Ala Ser Ile Tyr Leu Ala Ile Ser Asp Lys
                   565                 570                 575
   Thr Gln Asn Glu Ser Thr Ala Ile Phe Gly Glu Thr Ile Thr Pro Ala
               580                 585                 590
   Ser Leu Pro Gln Gly Phe Tyr Ala Phe Asn Gly Gly Ala Phe Gly Ile
           595                 600                 605
   His Arg Trp Gln Asp Lys Met Val Thr Leu Lys Ala Tyr Asn Thr Asn
       610                 615                 620
   Val Trp Ser Ser Glu Ile Tyr Asn Lys Asp Asn Arg Tyr Gly Arg Tyr
   625                 630                 635                 640
   Gln Ser His Gly Val Ala Gln Ile Val Ser Asn Gly Ser Gln Leu Ser
                   645                 650                 655
   Gln Gly Tyr Gln Gln Glu Gly Trp Asp Trp Asn Arg Met Glu Gly Ala
               660                 665                 670
   Thr Thr Ile His Leu Pro Leu Lys Asp Leu Asp Ser Pro Lys Pro His
           675                 680                 685
   Thr Leu Met Gln Arg Gly Glu Arg Gly Phe Ser Gly Thr Ser Ser Leu
       690                 695                 700
   Glu Gly Gln Tyr Gly Met Met Ala Phe Asn Leu Ile Tyr Pro Ala Asn
   705                 710                 715                 720
   Leu Glu Arg Phe Asp Pro Asn Phe Thr Ala Lys Lys Ser Val Leu Ala
                   725                 730                 735
   Ala Asp Asn His Leu Ile Phe Ile Gly Ser Asn Ile Asn Ser Ser Asp
               740                 745                 750
   Lys Asn Lys Asn Val Glu Thr Thr Leu Phe Gln His Ala Ile Thr Pro
           755                 760                 765
   Thr Leu Asn Thr Leu Trp Ile Asn Gly Gln Lys Ile Glu Asn Met Pro
       770                 775                 780
   Tyr Gln Thr Thr Leu Gln Gln Gly Asp Trp Leu Ile Asp Ser Asn Gly
   785                 790                 795                 800
   Asn Gly Tyr Leu Ile Thr Gln Ala Glu Lys Val Asn Val Ser Arg Gln
                   805                 810                 815
   His Gln Val Ser Ala Glu Asn Lys Asn Arg Gln Pro Thr Glu Gly Asn
               820                 825                 830
   Phe Ser Ser Ala Trp Ile Asp His Ser Thr Arg Pro Lys Asp Ala Ser
           835                 840                 845
   Tyr Glu Tyr Met Val Phe Leu Asp Ala Thr Pro Glu Lys Met Gly Glu
       850                 855                 860
   Met Ala Gln Lys Phe Arg Glu Asn Asn Gly Leu Tyr Gln Val Leu Arg
   865                 870                 875                 880
   Lys Asp Lys Asp Val His Ile Ile Leu Asp Lys Leu Ser Asn Val Thr
                   885                 890                 895
   Gly Tyr Ala Phe Tyr Gln Pro Ala Ser Ile Glu Asp Lys Trp Ile Lys
               900                 905                 910
   Lys Val Asn Lys Pro Ala Ile Val Met Thr His Arg Gln Lys Asp Thr
           915                 920                 925
   Leu Ile Val Ser Ala Val Thr Pro Asp Leu Asn Met Thr Arg Gln Lys
       930                 935                 940
   Ala Ala Thr Pro Val Thr Ile Asn Val Thr Ile Asn Gly Lys Trp Gln
   945                 950                 955                 960
   Ser Ala Asp Lys Asn Ser Glu Val Lys Tyr Gln Val Ser Gly Asp Asn
                   965                 970                 975
   Thr Glu Leu Thr Phe Thr Ser Tyr Phe Gly Ile Pro Gln Glu Ile Lys
               980                 985                 990
   Leu Ser Pro Leu Pro
           995
<210> 37
   <211> 1023
   <212> PRT
   <213> Pedobacter heparinus
<220>
   <223> chondroitinase ABCI protein Locus P59807
<220>
   <221> misc_feature
   <222> (1022)..(1022)
   <223> Unspecified amino acid
<220>
   <221> misc_feature
   <222> (1023)..(1023)
   <223> Unspecified amino acid
<400> 37
   Met Pro Ile Phe Arg Phe Thr Ala Leu Ala Met Thr Leu Gly Leu Leu
   1               5                   10                  15
   Ser Ala Pro Tyr Asn Ala Met Ala Ala Thr Ser Asn Pro Ala Phe Asp
               20                  25                  30
   Pro Lys Asn Leu Met Gln Ser Glu Ile Tyr His Phe Ala Gln Asn Asn
           35                  40                  45
   Pro Leu Ala Asp Phe Ser Ser Asp Lys Asn Ser Ile Leu Thr Leu Ser
       50                  55                  60
   Asp Lys Arg Ser Ile Met Gly Asn Gln Ser Leu Leu Trp Lys Trp Lys
   65                  70                  75                  80
   Gly Gly Ser Ser Phe Thr Leu His Lys Lys Leu Ile Val Pro Thr Asp
                   85                  90                  95
   Lys Glu Ala Ser Lys Ala Trp Gly Arg Ser Ser Thr Pro Val Phe Ser
               100                 105                 110
   Phe Trp Leu Tyr Asn Glu Lys Pro Ile Asp Gly Tyr Leu Thr Ile Asp
           115                 120                 125
   Phe Gly Glu Lys Leu Ile Ser Thr Ser Glu Ala Gln Ala Gly Phe Lys
       130                 135                 140
   Val Lys Leu Asp Phe Thr Gly Trp Arg Ala Val Gly Val Ser Leu Asn
   145 150 155 160
   Asn Asp Leu Glu Asn Arg Glu Met Thr Leu Asn Ala Thr Asn Thr Ser
   165 170 175
   Ser Asp Gly Thr Gln Asp Ser Ile Gly Arg Ser Leu Gly Ala Lys Val
   180 185 190
   Asp Ser Ile Arg Phe Lys Ala Pro Ser Asn Val Ser Gln Gly Glu Ile
   195 200 205
   Tyr Ile Asp Arg Ile Met Phe Ser Val Asp Asp Ala Arg Tyr Gln Trp
   210 215 220
   Ser Asp Tyr Gln Val Lys Thr Arg Leu Ser Glu Pro Glu Ile Gln Phe
   225 230 235 240
   His Asn Val Lys Pro Gln Leu Pro Val Thr Pro Glu Asn Leu Ala Ala
   245 250 255
   Ile Asp Leu Ile Arg Gln Arg Leu Ile Asn Glu Phe Val Gly Gly Glu
   260 265 270
   Lys Glu Thr Asn Leu Ala Leu Glu Glu Asn Ile Ser Lys Leu Lys Ser
   275 280 285
   Asp Phe Asp Ala Leu Asn Ile His Thr Leu Ala Asn Gly Gly Thr Gln
   290 295 300
   Gly Arg His Leu Ile Thr Asp Lys Gln Ile Ile Ile Tyr Gln Pro Glu
   305 310 315 320
   Asn Leu Asn Ser Gln Asp Lys Gln Leu Phe Asp Asn Tyr Val Ile Leu
   325 330 335
   Gly Asn Tyr Thr Thr Leu Met Phe Asn Ile Ser Arg Ala Tyr Val Leu
   340 345 350
   Glu Lys Asp Pro Thr Gln Lys Ala Gln Leu Lys Gln Met Tyr Leu Leu
   355 360 365
   Met Thr Lys His Leu Leu Asp Gln Gly Phe Val Lys Gly Ser Ala Leu
   370 375 380
   Val Thr Thr His His Trp Gly Tyr Ser Ser Arg Trp Trp Tyr Ile Ser
   385 390 395 400
   Thr Leu Leu Met Ser Asp Ala Leu Lys Glu Ala Asn Leu Gln Thr Gln
   405 410 415
   Val Tyr Asp Ser Leu Leu Trp Tyr Ser Arg Glu Phe Lys Ser Ser Phe
   420 425 430
   Asp Met Lys Val Ser Ala Asp Ser Ser Asp Leu Asp Tyr Phe Asn Thr
           435                 440                 445
   Leu Ser Arg Gln His Leu Ala Leu Leu Leu Leu Glu Pro Asp Asp Gln
       450                 455                 460
   Lys Arg Ile Asn Leu Val Asn Thr Phe Ser His Tyr Ile Thr Gly Ala
   465                 470                 475                 480
   Leu Thr Gln Val Pro Pro Gly Gly Lys Asp Gly Leu Arg Pro Asp Gly
                   485                 490                 495
   Thr Ala Trp Arg His Glu Gly Asn Tyr Pro Gly Tyr Ser Phe Pro Ala
               500                 505                 510
   Phe Lys Asn Ala Ser Gln Leu Ile Tyr Leu Leu Arg Asp Thr Pro Phe
           515                 520                 525
   Ser Val Gly Glu Ser Gly Trp Asn Asn Leu Lys Lys Ala Met Val Ser
       530                 535                 540
   Ala Trp Ile Tyr Ser Asn Pro Glu Val Gly Leu Pro Leu Ala Gly Arg
   545                 550                 555                 560
   His Pro Phe Asn Ser Pro Ser Leu Lys Ser Val Ala Gln Gly Tyr Tyr
                   565                 570                 575
   Trp Leu Ala Met Ser Ala Lys Ser Ser Pro Asp Lys Thr Leu Ala Ser
               580                 585                 590
   Ile Tyr Leu Ala Ile Ser Asp Lys Thr Gln Asn Glu Ser Thr Ala Ile
           595                 600                 605
   Phe Gly Glu Thr Ile Thr Pro Ala Ser Leu Pro Gln Gly Phe Tyr Ala
       610                 615                 620
   Phe Asn Gly Gly Ala Phe Gly Ile His Arg Trp Gln Asp Lys Met Val
   625                 630                 635                 640
   Thr Leu Lys Ala Tyr Asn Thr Asn Val Trp Ser Ser Glu Ile Tyr Asn
                   645                 650                 655
   Lys Asp Asn Arg Tyr Gly Arg Tyr Gln Ser His Gly Val Ala Gln Ile
               660                 665                 670
   Val Ser Asn Gly Ser Gln Leu Ser Gln Gly Tyr Gln Gln Glu Gly Trp
           675                 680                 685
   Asp Trp Asn Arg Met Glu Gly Ala Thr Thr Ile His Leu Pro Leu Lys
       690                 695                 700
   Asp Leu Asp Ser Pro Lys Pro His Thr Leu Met Gln Arg Gly Glu Arg
   705                 710                 715                 720
   Gly Phe Ser Gly Thr Ser Ser Leu Glu Gly Gln Tyr Gly Met Met Ala
                   725                 730                 735
   Phe Asn Leu Ile Tyr Pro Ala Asn Leu Glu Arg Phe Asp Pro Asn Phe
               740                 745                 750
   Thr Ala Lys Lys Ser Val Leu Ala Ala Asp Asn His Leu Ile Phe Ile
           755                 760                 765
   Gly Ser Asn Ile Asn Ser Ser Asp Lys Asn Lys Asn Val Glu Thr Thr
       770                 775                 780
   Leu Phe Gln His Ala Ile Thr Pro Thr Leu Asn Thr Leu Trp Ile Asn
   785                 790                 795                 800
   Gly Gln Lys Ile Glu Asn Met Pro Tyr Gln Thr Thr Leu Gln Gln Gly
                   805                 810                 815
   Asp Trp Leu Ile Asp Ser Asn Gly Asn Gly Tyr Leu Ile Thr Gln Ala
               820                 825                 830
   Glu Lys Val Asn Val Ser Arg Gln His Gln Val Ser Ala Glu Asn Lys
           835                 840                 845
   Asn Arg Gln Pro Thr Glu Gly Asn Phe Ser Ser Ala Trp Ile Asp His
       850                 855                 860
   Ser Thr Arg Pro Lys Asp Ala Ser Tyr Glu Tyr Met Val Phe Leu Asp
   865                 870                 875                 880
   Ala Thr Pro Glu Lys Met Gly Glu Met Ala Gln Lys Phe Arg Glu Asn
                   885                 890                 895
   Asn Gly Leu Tyr Gln Val Leu Arg Lys Asp Lys Asp Val His Ile Ile
               900                 905                 910
   Leu Asp Lys Leu Ser Asn Val Thr Gly Tyr Ala Phe Tyr Gln Pro Ala
           915                 920                 925
   Ser Ile Glu Asp Lys Trp Ile Lys Lys Val Asn Lys Pro Ala Ile Val
       930                 935                 940
   Met Thr His Arg Gln Lys Asp Thr Leu Ile Val Ser Ala Val Thr Pro
   945                 950                 955                 960
   Asp Leu Asn Met Thr Arg Gln Lys Ala Ala Thr Pro Val Thr Ile Asn
                   965                 970                 975
   Val Thr Ile Asn Gly Lys Trp Gln Ser Ala Asp Lys Asn Ser Glu Val
   980 985 990
   Lys Tyr Gln Val Ser Gly Asp Asn Thr Glu Leu Thr Phe Thr Ser Tyr
   995 1000 1005
   Phe Gly Ile Pro Gln Glu Ile Lys Leu Ser Pro Leu Pro Xaa Xaa
   1010 1015 1020
<210> 38
   <211> 979
   <212> PRT
   <213> Artificial
<220>
   <223> Synthetic polypeptide, N(delta) 20 chondroitinase ABCI having gwra and dalni sequences
<220>
   <221> misc_feature
   <222> (978)..(978)
   <223> Unspecified amino acid
<220>
   <221> misc_feature
   <222> (979)..(979)
   <223> Unspecified amino acid
<400> 38
   Ala Gln Asn Asn Pro Leu Ala Asp Phe Ser Ser Asp Lys Asn Ser Ile
   1               5                   10                  15
   Leu Thr Leu Ser Asp Lys Arg Ser Ile Met Gly Asn Gln Ser Leu Leu
               20                  25                  30
   Trp Lys Trp Lys Gly Gly Ser Ser Phe Thr Leu His Lys Lys Leu Ile
           35                  40                  45
   Val Pro Thr Asp Lys Glu Ala Ser Lys Ala Trp Gly Arg Ser Ser Thr
       50                  55                  60
   Pro Val Phe Ser Phe Trp Leu Tyr Asn Glu Lys Pro Ile Asp Gly Tyr
   65                  70                  75                  80
   Leu Thr Ile Asp Phe Gly Glu Lys Leu Ile Ser Thr Ser Glu Ala Gln
                   85                  90                  95
   Ala Gly Phe Lys Val Lys Leu Asp Phe Thr Gly Trp Arg Ala Val Gly
               100                 105                 110
   Val Ser Leu Asn Asn Asp Leu Glu Asn Arg Glu Met Thr Leu Asn Ala
           115                 120                 125
   Thr Asn Thr Ser Ser Asp Gly Thr Gln Asp Ser Ile Gly Arg Ser Leu
       130                 135                 140
   Gly Ala Lys Val Asp Ser Ile Arg Phe Lys Ala Pro Ser Asn Val Ser
   145                 150                 155                 160
   Gln Gly Glu Ile Tyr Ile Asp Arg Ile Met Phe Ser Val Asp Asp Ala
                   165                 170                 175
   Arg Tyr Gln Trp Ser Asp Tyr Gln Val Lys Thr Arg Leu Ser Glu Pro
               180                 185                 190
   Glu Ile Gln Phe His Asn Val Lys Pro Gln Leu Pro Val Thr Pro Glu
           195                 200                 205
   Asn Leu Ala Ala Ile Asp Leu Ile Arg Gln Arg Leu Ile Asn Glu Phe
       210                 215                 220
   Val Gly Gly Glu Lys Glu Thr Asn Leu Ala Leu Glu Glu Asn Ile Ser
   225                 230                 235                 240
   Lys Leu Lys Ser Asp Phe Asp Ala Leu Asn Ile His Thr Leu Ala Asn
                   245                 250                 255
   Gly Gly Thr Gln Gly Arg His Leu Ile Thr Asp Lys Gln Ile Ile Ile
               260                 265                 270
   Tyr Gln Pro Glu Asn Leu Asn Ser Gln Asp Lys Gln Leu Phe Asp Asn
           275                 280                 285
   Tyr Val Ile Leu Gly Asn Tyr Thr Thr Leu Met Phe Asn Ile Ser Arg
       290                 295                 300
   Ala Tyr Val Leu Glu Lys Asp Pro Thr Gln Lys Ala Gln Leu Lys Gln
   305                 310                 315                 320
   Met Tyr Leu Leu Met Thr Lys His Leu Leu Asp Gln Gly Phe Val Lys
                   325                 330                 335
   Gly Ser Ala Leu Val Thr Thr His His Trp Gly Tyr Ser Ser Arg Trp
               340                 345                 350
   Trp Tyr Ile Ser Thr Leu Leu Met Ser Asp Ala Leu Lys Glu Ala Asn
           355                 360                 365
   Leu Gln Thr Gln Val Tyr Asp Ser Leu Leu Trp Tyr Ser Arg Glu Phe
       370                 375                 380
   Lys Ser Ser Phe Asp Met Lys Val Ser Ala Asp Ser Ser Asp Leu Asp
   385                 390                 395                 400
   Tyr Phe Asn Thr Leu Ser Arg Gln His Leu Ala Leu Leu Leu Leu Glu
                   405                 410                 415
   Pro Asp Asp Gln Lys Arg Ile Asn Leu Val Asn Thr Phe Ser His Tyr
               420                 425                 430
   Ile Thr Gly Ala Leu Thr Gln Val Pro Pro Gly Gly Lys Asp Gly Leu
           435                 440                 445
   Arg Pro Asp Gly Thr Ala Trp Arg His Glu Gly Asn Tyr Pro Gly Tyr
       450                 455                 460
   Ser Phe Pro Ala Phe Lys Asn Ala Ser Gln Leu Ile Tyr Leu Leu Arg
   465                 470                 475                 480
   Asp Thr Pro Phe Ser Val Gly Glu Ser Gly Trp Asn Asn Leu Lys Lys
                   485                 490                 495
   Ala Met Val Ser Ala Trp Ile Tyr Ser Asn Pro Glu Val Gly Leu Pro
               500                 505                 510
   Leu Ala Gly Arg His Pro Phe Asn Ser Pro Ser Leu Lys Ser Val Ala
           515                 520                 525
   Gln Gly Tyr Tyr Trp Leu Ala Met Ser Ala Lys Ser Ser Pro Asp Lys
       530                 535                 540
   Thr Leu Ala Ser Ile Tyr Leu Ala Ile Ser Asp Lys Thr Gln Asn Glu
   545                 550                 555                 560
   Ser Thr Ala Ile Phe Gly Glu Thr Ile Thr Pro Ala Ser Leu Pro Gln
                   565                 570                 575
   Gly Phe Tyr Ala Phe Asn Gly Gly Ala Phe Gly Ile His Arg Trp Gln
               580                 585                 590
   Asp Lys Met Val Thr Leu Lys Ala Tyr Asn Thr Asn Val Trp Ser Ser
           595                 600                 605
   Glu Ile Tyr Asn Lys Asp Asn Arg Tyr Gly Arg Tyr Gln Ser His Gly
       610                 615                 620
   Val Ala Gln Ile Val Ser Asn Gly Ser Gln Leu Ser Gln Gly Tyr Gln
   625                 630                 635                 640
   Gln Glu Gly Trp Asp Trp Asn Arg Met Glu Gly Ala Thr Thr Ile His
                   645                 650                 655
   Leu Pro Leu Lys Asp Leu Asp Ser Pro Lys Pro His Thr Leu Met Gln
               660                 665                 670
   Arg Gly Glu Arg Gly Phe Ser Gly Thr Ser Ser Leu Glu Gly Gln Tyr
           675                 680                 685
   Gly Met Met Ala Phe Asn Leu Ile Tyr Pro Ala Asn Leu Glu Arg Phe
   690 695 700
   Asp Pro Asn Phe Thr Ala Lys Lys Ser Val Leu Ala Ala Asp Asn His
   705 710 715 720
   Leu Ile Phe Ile Gly Ser Asn Ile Asn Ser Ser Asp Lys Asn Lys Asn
   725 730 735
   Val Glu Thr Thr Leu Phe Gln His Ala Ile Thr Pro Thr Leu Asn Thr
   740 745 750
   Leu Trp Ile Asn Gly Gln Lys Ile Glu Asn Met Pro Tyr Gln Thr Thr
   755 760 765
   Leu Gln Gln Gly Asp Trp Leu Ile Asp Ser Asn Gly Asn Gly Tyr Leu
   770 775 780
   Ile Thr Gln Ala Glu Lys Val Asn Val Ser Arg Gln His Gln Val Ser
   785 790 795 800
   Ala Glu Asn Lys Asn Arg Gln Pro Thr Glu Gly Asn Phe Ser Ser Ala
   805 810 815
   Trp Ile Asp His Ser Thr Arg Pro Lys Asp Ala Ser Tyr Glu Tyr Met
   820 825 830
   Val Phe Leu Asp Ala Thr Pro Glu Lys Met Gly Glu Met Ala Gln Lys
   835 840 845
   Phe Arg Glu Asn Asn Gly Leu Tyr Gln Val Leu Arg Lys Asp Lys Asp
   850 855 860
   Val His Ile Ile Leu Asp Lys Leu Ser Asn Val Thr Gly Tyr Ala Phe
   865 870 875 880
   Tyr Gln Pro Ala Ser Ile Glu Asp Lys Trp Ile Lys Lys Val Asn Lys
   885 890 895
   Pro Ala Ile Val Met Thr His Arg Gln Lys Asp Thr Leu Ile Val Ser
   900 905 910
   Ala Val Thr Pro Asp Leu Asn Met Thr Arg Gln Lys Ala Ala Thr Pro
   915 920 925
   Val Thr Ile Asn Val Thr Ile Asn Gly Lys Trp Gln Ser Ala Asp Lys
   930 935 940
   Asn Ser Glu Val Lys Tyr Gln Val Ser Gly Asp Asn Thr Glu Leu Thr
   945 950 955 960
   Phe Thr Ser Tyr Phe Gly Ile Pro Gln Glu Ile Lys Leu Ser Pro Leu
   965 970 975
   Pro Xaa Xaa
<210> 39
   <211> 939
   <212> PRT
   <213> Artificial
<220>
   <223> Synthetic polypeptide, N(delta) 60 c(delta)80 ABCI (F[sub]85-N[sub]1023)
<220>
   <221> misc_feature
   <222> (938)..(938)
   <223> Unspecified amino acid
<220>
   <221> misc_feature
   <222> (939)..(939)
   <223> Unspecified amino acid
<400> 39
   Phe Thr Leu His Lys Lys Leu Ile Val Pro Thr Asp Lys Glu Ala Ser
   1               5                   10                  15
   Lys Ala Trp Gly Arg Ser Ser Thr Pro Val Phe Ser Phe Trp Leu Tyr
               20                  25                  30
   Asn Glu Lys Pro Ile Asp Gly Tyr Leu Thr Ile Asp Phe Gly Glu Lys
           35                  40                  45
   Leu Ile Ser Thr Ser Glu Ala Gln Ala Gly Phe Lys Val Lys Leu Asp
       50                  55                  60
   Phe Thr Gly Trp Arg Ala Val Gly Val Ser Leu Asn Asn Asp Leu Glu
   65                  70                  75                  80
   Asn Arg Glu Met Thr Leu Asn Ala Thr Asn Thr Ser Ser Asp Gly Thr
                   85                  90                  95
   Gln Asp Ser Ile Gly Arg Ser Leu Gly Ala Lys Val Asp Ser Ile Arg
               100                 105                 110
   Phe Lys Ala Pro Ser Asn Val Ser Gln Gly Glu Ile Tyr Ile Asp Arg
           115                 120                 125
   Ile Met Phe Ser Val Asp Asp Ala Arg Tyr Gln Trp Ser Asp Tyr Gln
       130                 135                 140
   Val Lys Thr Arg Leu Ser Glu Pro Glu Ile Gln Phe His Asn Val Lys
   145                 150                 155                 160
   Pro Gln Leu Pro Val Thr Pro Glu Asn Leu Ala Ala Ile Asp Leu Ile
                   165                 170                 175
   Arg Gln Arg Leu Ile Asn Glu Phe Val Gly Gly Glu Lys Glu Thr Asn
               180                 185                 190
   Leu Ala Leu Glu Glu Asn Ile Ser Lys Leu Lys Ser Asp Phe Asp Ala
           195                 200                 205
   Leu Asn Ile His Thr Leu Ala Asn Gly Gly Thr Gln Gly Arg His Leu
       210                 215                 220
   Ile Thr Asp Lys Gln Ile Ile Ile Tyr Gln Pro Glu Asn Leu Asn Ser
   225                 230                 235                 240
   Gln Asp Lys Gln Leu Phe Asp Asn Tyr Val Ile Leu Gly Asn Tyr Thr
                   245                 250                 255
   Thr Leu Met Phe Asn Ile Ser Arg Ala Tyr Val Leu Glu Lys Asp Pro
               260                 265                 270
   Thr Gln Lys Ala Gln Leu Lys Gln Met Tyr Leu Leu Met Thr Lys His
           275                 280                 285
   Leu Leu Asp Gln Gly Phe Val Lys Gly Ser Ala Leu Val Thr Thr His
       290                 295                 300
   His Trp Gly Tyr Ser Ser Arg Trp Trp Tyr Ile Ser Thr Leu Leu Met
   305                 310                 315                 320
   Ser Asp Ala Leu Lys Glu Ala Asn Leu Gln Thr Gln Val Tyr Asp Ser
                   325                 330                 335
   Leu Leu Trp Tyr Ser Arg Glu Phe Lys Ser Ser Phe Asp Met Lys Val
               340                 345                 350
   Ser Ala Asp Ser Ser Asp Leu Asp Tyr Phe Asn Thr Leu Ser Arg Gln
           355                 360                 365
   His Leu Ala Leu Leu Leu Leu Glu Pro Asp Asp Gln Lys Arg Ile Asn
       370                 375                 380
   Leu Val Asn Thr Phe Ser His Tyr Ile Thr Gly Ala Leu Thr Gln Val
   385                 390                 395                 400
   Pro Pro Gly Gly Lys Asp Gly Leu Arg Pro Asp Gly Thr Ala Trp Arg
                   405                 410                 415
   His Glu Gly Asn Tyr Pro Gly Tyr Ser Phe Pro Ala Phe Lys Asn Ala
               420                 425                 430
   Ser Gln Leu Ile Tyr Leu Leu Arg Asp Thr Pro Phe Ser Val Gly Glu
           435                 440                 445
   Ser Gly Trp Asn Asn Leu Lys Lys Ala Met Val Ser Ala Trp Ile Tyr
       450                 455                 460
   Ser Asn Pro Glu Val Gly Leu Pro Leu Ala Gly Arg His Pro Phe Asn
   465                 470                 475                 480
   Ser Pro Ser Leu Lys Ser Val Ala Gln Gly Tyr Tyr Trp Leu Ala Met
                   485                 490                 495
   Ser Ala Lys Ser Ser Pro Asp Lys Thr Leu Ala Ser Ile Tyr Leu Ala
               500                 505                 510
   Ile Ser Asp Lys Thr Gln Asn Glu Ser Thr Ala Ile Phe Gly Glu Thr
           515                 520                 525
   Ile Thr Pro Ala Ser Leu Pro Gln Gly Phe Tyr Ala Phe Asn Gly Gly
       530                 535                 540
   Ala Phe Gly Ile His Arg Trp Gln Asp Lys Met Val Thr Leu Lys Ala
   545                 550                 555                 560
   Tyr Asn Thr Asn Val Trp Ser Ser Glu Ile Tyr Asn Lys Asp Asn Arg
                   565                 570                 575
   Tyr Gly Arg Tyr Gln Ser His Gly Val Ala Gln Ile Val Ser Asn Gly
               580                 585                 590
   Ser Gln Leu Ser Gln Gly Tyr Gln Gln Glu Gly Trp Asp Trp Asn Arg
           595                 600                 605
   Met Glu Gly Ala Thr Thr Ile His Leu Pro Leu Lys Asp Leu Asp Ser
       610                 615                 620
   Pro Lys Pro His Thr Leu Met Gln Arg Gly Glu Arg Gly Phe Ser Gly
   625                 630                 635                 640
   Thr Ser Ser Leu Glu Gly Gln Tyr Gly Met Met Ala Phe Asn Leu Ile
                   645                 650                 655
   Tyr Pro Ala Asn Leu Glu Arg Phe Asp Pro Asn Phe Thr Ala Lys Lys
               660                 665                 670
   Ser Val Leu Ala Ala Asp Asn His Leu Ile Phe Ile Gly Ser Asn Ile
           675                 680                 685
   Asn Ser Ser Asp Lys Asn Lys Asn Val Glu Thr Thr Leu Phe Gln His
       690                 695                 700
   Ala Ile Thr Pro Thr Leu Asn Thr Leu Trp Ile Asn Gly Gln Lys Ile
   705                 710                 715                 720
   Glu Asn Met Pro Tyr Gln Thr Thr Leu Gln Gln Gly Asp Trp Leu Ile
   725 730 735
   Asp Ser Asn Gly Asn Gly Tyr Leu Ile Thr Gln Ala Glu Lys Val Asn
   740 745 750
   Val Ser Arg Gln His Gln Val Ser Ala Glu Asn Lys Asn Arg Gln Pro
   755 760 765
   Thr Glu Gly Asn Phe Ser Ser Ala Trp Ile Asp His Ser Thr Arg Pro
   770 775 780
   Lys Asp Ala Ser Tyr Glu Tyr Met Val Phe Leu Asp Ala Thr Pro Glu
   785 790 795 800
   Lys Met Gly Glu Met Ala Gln Lys Phe Arg Glu Asn Asn Gly Leu Tyr
   805 810 815
   Gln Val Leu Arg Lys Asp Lys Asp Val His Ile Ile Leu Asp Lys Leu
   820 825 830
   Ser Asn Val Thr Gly Tyr Ala Phe Tyr Gln Pro Ala Ser Ile Glu Asp
   835 840 845
   Lys Trp Ile Lys Lys Val Asn Lys Pro Ala Ile Val Met Thr His Arg
   850 855 860
   Gln Lys Asp Thr Leu Ile Val Ser Ala Val Thr Pro Asp Leu Asn Met
   865 870 875 880
   Thr Arg Gln Lys Ala Ala Thr Pro Val Thr Ile Asn Val Thr Ile Asn
   885 890 895
   Gly Lys Trp Gln Ser Ala Asp Lys Asn Ser Glu Val Lys Tyr Gln Val
   900 905 910
   Ser Gly Asp Asn Thr Glu Leu Thr Phe Thr Ser Tyr Phe Gly Ile Pro
   915 920 925
   Gln Glu Ile Lys Leu Ser Pro Leu Pro Xaa Xaa
   930 935
<210> 40
   <211> 858
   <212> PRT
   <213> Artificial
<220>
   <223> Synthetic polypeptide, N(delta)60 C(delta)80 chondroitinase ABCI having gwrt and dalnt sequences
<400> 40
   Phe Thr Leu His Lys Lys Leu Ile Val Pro Thr Asp Lys Glu Ala Ser
   1 5 10 15
   Lys Ala Trp Gly Arg Ser Ser Thr Pro Val Phe Ser Phe Trp Leu Tyr
   20 25 30
   Asn Glu Lys Pro Ile Asp Gly Tyr Leu Thr Ile Asp Phe Gly Glu Lys
   35 40 45
   Leu Ile Ser Thr Ser Glu Ala Gln Ala Gly Phe Lys Val Lys Leu Asp
   50 55 60
   Phe Thr Gly Trp Arg Ala Val Gly Val Ser Leu Asn Asn Asp Leu Glu
   65 70 75 80
   Asn Arg Glu Met Thr Leu Asn Ala Thr Asn Thr Ser Ser Asp Gly Thr
   85 90 95
   Gln Asp Ser Ile Gly Arg Ser Leu Gly Ala Lys Val Asp Ser Ile Arg
   100 105 110
   Phe Lys Ala Pro Ser Asn Val Ser Gln Gly Glu Ile Tyr Ile Asp Arg
   115 120 125
   Ile Met Phe Ser Val Asp Asp Ala Arg Tyr Gln Trp Ser Asp Tyr Gln
   130 135 140
   Val Lys Thr Arg Leu Ser Glu Pro Glu Ile Gln Phe His Asn Val Lys
   145 150 155 160
   Pro Gln Leu Pro Val Thr Pro Glu Asn Leu Ala Ala Ile Asp Leu Ile
   165 170 175
   Arg Gln Arg Leu Ile Asn Glu Phe Val Gly Gly Glu Lys Glu Thr Asn
   180 185 190
   Leu Ala Leu Glu Glu Asn Ile Ser Lys Leu Lys Ser Asp Phe Asp Ala
   195 200 205
   Leu Asn Ile His Thr Leu Ala Asn Gly Gly Thr Gln Gly Arg His Leu
   210 215 220
   Ile Thr Asp Lys Gln Ile Ile Ile Tyr Gln Pro Glu Asn Leu Asn Ser
   225 230 235 240
   Gln Asp Lys Gln Leu Phe Asp Asn Tyr Val Ile Leu Gly Asn Tyr Thr
   245 250 255
   Thr Leu Met Phe Asn Ile Ser Arg Ala Tyr Val Leu Glu Lys Asp Pro
   260 265 270
   Thr Gln Lys Ala Gln Leu Lys Gln Met Tyr Leu Leu Met Thr Lys His
   275 280 285
   Leu Leu Asp Gln Gly Phe Val Lys Gly Ser Ala Leu Val Thr Thr His
       290                 295                 300
   His Trp Gly Tyr Ser Ser Arg Trp Trp Tyr Ile Ser Thr Leu Leu Met
   305                 310                 315                 320
   Ser Asp Ala Leu Lys Glu Ala Asn Leu Gln Thr Gln Val Tyr Asp Ser
                   325                 330                 335
   Leu Leu Trp Tyr Ser Arg Glu Phe Lys Ser Ser Phe Asp Met Lys Val
               340                 345                 350
   Ser Ala Asp Ser Ser Asp Leu Asp Tyr Phe Asn Thr Leu Ser Arg Gln
           355                 360                 365
   His Leu Ala Leu Leu Leu Leu Glu Pro Asp Asp Gln Lys Arg Ile Asn
       370                 375                 380
   Leu Val Asn Thr Phe Ser His Tyr Ile Thr Gly Ala Leu Thr Gln Val
   385                 390                 395                 400
   Pro Pro Gly Gly Lys Asp Gly Leu Arg Pro Asp Gly Thr Ala Trp Arg
                   405                 410                 415
   His Glu Gly Asn Tyr Pro Gly Tyr Ser Phe Pro Ala Phe Lys Asn Ala
               420                 425                 430
   Ser Gln Leu Ile Tyr Leu Leu Arg Asp Thr Pro Phe Ser Val Gly Glu
           435                 440                 445
   Ser Gly Trp Asn Asn Leu Lys Lys Ala Met Val Ser Ala Trp Ile Tyr
       450                 455                 460
   Ser Asn Pro Glu Val Gly Leu Pro Leu Ala Gly Arg His Pro Phe Asn
   465                 470                 475                 480
   Ser Pro Ser Leu Lys Ser Val Ala Gln Gly Tyr Tyr Trp Leu Ala Met
                   485                 490                 495
   Ser Ala Lys Ser Ser Pro Asp Lys Thr Leu Ala Ser Ile Tyr Leu Ala
               500                 505                 510
   Ile Ser Asp Lys Thr Gln Asn Glu Ser Thr Ala Ile Phe Gly Glu Thr
           515                 520                 525
   Ile Thr Pro Ala Ser Leu Pro Gln Gly Phe Tyr Ala Phe Asn Gly Gly
       530                 535                 540
   Ala Phe Gly Ile His Arg Trp Gln Asp Lys Met Val Thr Leu Lys Ala
   545                 550                 555                 560
   Tyr Asn Thr Asn Val Trp Ser Ser Glu Ile Tyr Asn Lys Asp Asn Arg
   565 570 575
   Tyr Gly Arg Tyr Gln Ser His Gly Val Ala Gln Ile Val Ser Asn Gly
   580 585 590
   Ser Gln Leu Ser Gln Gly Tyr Gln Gln Glu Gly Trp Asp Trp Asn Arg
   595 600 605
   Met Glu Gly Ala Thr Thr Ile His Leu Pro Leu Lys Asp Leu Asp Ser
   610 615 620
   Pro Lys Pro His Thr Leu Met Gln Arg Gly Glu Arg Gly Phe Ser Gly
   625 630 635 640
   Thr Ser Ser Leu Glu Gly Gln Tyr Gly Met Met Ala Phe Asn Leu Ile
   645 650 655
   Tyr Pro Ala Asn Leu Glu Arg Phe Asp Pro Asn Phe Thr Ala Lys Lys
   660 665 670
   Ser Val Leu Ala Ala Asp Asn His Leu Ile Phe Ile Gly Ser Asn Ile
   675 680 685
   Asn Ser Ser Asp Lys Asn Lys Asn Val Glu Thr Thr Leu Phe Gln His
   690 695 700
   Ala Ile Thr Pro Thr Leu Asn Thr Leu Trp Ile Asn Gly Gln Lys Ile
   705 710 715 720
   Glu Asn Met Pro Tyr Gln Thr Thr Leu Gln Gln Gly Asp Trp Leu Ile
   725 730 735
   Asp Ser Asn Gly Asn Gly Tyr Leu Ile Thr Gln Ala Glu Lys Val Asn
   740 745 750
   Val Ser Arg Gln His Gln Val Ser Ala Glu Asn Lys Asn Arg Gln Pro
   755 760 765
   Thr Glu Gly Asn Phe Ser Ser Ala Trp Ile Asp His Ser Thr Arg Pro
   770 775 780
   Lys Asp Ala Ser Tyr Glu Tyr Met Val Phe Leu Asp Ala Thr Pro Glu
   785 790 795 800
   Lys Met Gly Glu Met Ala Gln Lys Phe Arg Glu Asn Asn Gly Leu Tyr
   805 810 815
   Gln Val Leu Arg Lys Asp Lys Asp Val His Ile Ile Leu Asp Lys Leu
   820 825 830
   Ser Asn Val Thr Gly Tyr Ala Phe Tyr Gln Pro Ala Ser Ile Glu Asp
   835 840 845
   Lys Trp Ile Lys Lys Val Asn Lys Pro Ala
       850                 855
<210> 41
   <211> 2976
   <212> DNA
   <213> Artificial
<220>
   <223> Synthetic polynucleotide, TAT-Chondroitinase ABC I N(delta)20 Nucleic Acid
<220>
   <221> misc_feature
   <222> (1068)..(1068)
   <223> Unknown nucleotide
<400> 41
   ggtcgtaaaa agcgtcgtca acgtcgtcgt cctcctcaat gcgcacaaaa taacccatta      60
   gcagacttct catcagataa aaactcaata ctaacgttat ctgataaacg tagcattatg     120
   ggaaaccaat ctcttttatg gaaatggaaa ggtggtagta gctttacttt acataaaaaa     180
   ctgattgtcc ccaccgataa agaagcatct aaagcatggg gacgctcatc cacccccgtt     240
   ttctcatttt ggctttacaa tgaaaaaccg attgatggtt atcttactat cgatttcgga     300
   gaaaaactca tttcaaccag tgaggctcag gcaggcttta aagtaaaatt agatttcact     360
   ggctggcgta ctgtgggagt ctctttaaat aacgatcttg aaaatcgaga gatgacctta     420
   aatgcaacca atacctcctc tgatggtact caagacagca ttgggcgttc tttaggtgct     480
   aaagtcgata gtattcgttt taaagcgcct tctaatgtga gtcagggtga aatctatatc     540
   gaccgtatta tgttttctgt cgatgatgct cgctaccaat ggtctgatta tcaagtaaaa     600
   actcgcttat cagaacctga aattcaattt cacaacgtaa agccacaact acctgtaaca     660
   cctgaaaatt tagcggccat tgatcttatt cgccaacgtc taattaatga atttgtcgga     720
   ggtgaaaaag agacaaacct cgcattagaa gagaatatca gcaaattaaa aagtgatttc     780
   gatgctctta atactcacac tttagcaaat ggtggaacgc aaggcagaca tctgatcact     840
   gataaacaaa tcattattta tcaaccagag aatcttaact ctcaagataa acaactattt     900
   gataattatg ttattttagg taattacacg acattaatgt ttaatattag ccgtgcttat     960
   gtgctggaaa aagatcccac acaaaaggcg caactaaagc agatgtactt attaatgaca    1020
   aagcatttat tagatcaagg ctttgttaaa gggagtgctt tagtgacnac ccatcactgg    1080
   ggatacagtt ctcgttggtg gtatatttcc acgttattaa tgtctgatgc actaaaagaa    1140
   gcgaacctac aaactcaagt ttatgattca ttactgtggt attcacgtga gtttaaaagt    1200
   agttttgata tgaaagtaag tgctgatagc tctgatctag attatttcaa taccttatct    1260
   cgccaacatt tagccttatt actactagag cctgatgatc aaaagcgtat caacttagtt    1320
   aatactttca gccattatat cactggcgca ttaacgcaag tgccaccggg tggtaaagat    1380
   ggtttacgcc ctgatggtac agcatggcga catgaaggca actatccggg ctactctttc    1440
   ccagccttta aaaatgcctc tcagcttatt tatttattac gcgatacacc attttcagtg   1500
   ggtgaaagtg gttggaatag cctgaaaaaa gcgatggttt cagcgtggat ctacagtaat   1560
   ccagaagttg gattaccgct tgcaggaaga caccctctta actcaccttc gttaaaatca   1620
   gtcgctcaag gctattactg gcttgccatg tctgcaaaat catcgcctga taaaacactt   1680
   gcatctattt atcttgcgat tagtgataaa acacaaaatg aatcaactgc tatttttgga   1740
   gaaactatta caccagcgtc tttacctcaa ggtttctatg cctttaatgg cggtgctttt   1800
   ggtattcatc gttggcaaga taaaatggtg acactgaaag cttataacac caatgtttgg   1860
   tcatctgaaa tttataacaa agataaccgt tatggccgtt accaaagtca tggtgtcgct   1920
   caaatagtga gtaatggctc gcagctttca cagggctatc agcaagaagg ttgggattgg   1980
   aatagaatgc caggggcaac cactatccac cttcctctta aagacttaga cagtcctaaa   2040
   cctcatacct taatgcaacg tggagagcgt ggatttagcg gaacatcatc ccttgaaggt   2100
   caatatggca tgatggcatt cgatcttatt tatcccgcca atcttgagcg ttttgatcct   2160
   aatttcactg cgaaaaagag tgtattagcc gctgataatc acttaatttt tattggtagc   2220
   aatataaata gtagtgataa aaataaaaat gttgaaacga ccttattcca acatgccatt   2280
   actccaacat taaataccct ttggattaat ggacaaaaga tagaaaacat gccttatcaa   2340
   acaacacttc aacaaggtga ttggttaatt gatagcaatg gcaatggtta cttaattact   2400
   caagcagaaa aagtaaatgt aagtcgccaa catcaggttt cagcggaaaa taaaaatcgc   2460
   caaccgacag aaggaaactt tagctcggca tggatcgatc acagcactcg ccccaaagat   2520
   gccagttatg agtatatggt ctttttagat gcgacacctg aaaaaatggg agagatggca   2580
   caaaaattcc gtgaaaataa tgggttatat caggttcttc gtaaggataa agacgttcat   2640
   attattctcg ataaactcag caatgtaacg ggatatgcct tttatcagcc agcatcaatt   2700
   gaagacaaat ggatcaaaaa ggttaataaa cctgcaattg tgatgactca tcgacaaaaa   2760
   gacactctta ttgtcagtgc agttacacct gatttaaata tgactcgcca aaaagcagca   2820
   actcctgtca ccatcaatgt cacgattaat ggcaaatggc aatctgctga taaaaatagt   2880
   gaagtgaaat atcaggtttc tggtgataac actgaactga cgtttacgag ttactttggt   2940
   attccacaag aaatcaaact ctcgccactc ccttga                             2976
<210> 42
   <211> 991
   <212> PRT
   <213> Artificial
<220>
   <223> Synthetic polypeptide, HIV-1 TAT chondroitinase ABCI-N(delta)20 fusion polypeptide
<400> 42
   Gly Arg Lys Lys Arg Arg Gln Arg Arg Arg Pro Pro Gln Cys Ala Gln
   1               5                   10                  15
   Asn Asn Pro Leu Ala Asp Phe Ser Ser Asp Lys Asn Ser Ile Leu Thr
   20 25 30
   Leu Ser Asp Lys Arg Ser Ile Met Gly Asn Gln Ser Leu Leu Trp Lys
   35 40 45
   Trp Lys Gly Gly Ser Ser Phe Thr Leu His Lys Lys Leu Ile Val Pro
   50 55 60
   Thr Asp Lys Glu Ala Ser Lys Ala Trp Gly Arg Ser Ser Thr Pro Val
   65 70 75 80
   Phe Ser Phe Trp Leu Tyr Asn Glu Lys Pro Ile Asp Gly Tyr Leu Thr
   85 90 95
   Ile Asp Phe Gly Glu Lys Leu Ile Ser Thr Ser Glu Ala Gln Ala Gly
   100 105 110
   Phe Lys Val Lys Leu Asp Phe Thr Gly Trp Arg Thr Val Gly Val Ser
   115 120 125
   Leu Asn Asn Asp Leu Glu Asn Arg Glu Met Thr Leu Asn Ala Thr Asn
   130 135 140
   Thr Ser Ser Asp Gly Thr Gln Asp Ser Ile Gly Arg Ser Leu Gly Ala
   145 150 155 160
   Lys Val Asp Ser Ile Arg Phe Lys Ala Pro Ser Asn Val Ser Gln Gly
   165 170 175
   Glu Ile Tyr Ile Asp Arg Ile Met Phe Ser Val Asp Asp Ala Arg Tyr
   180 185 190
   Gln Trp Ser Asp Tyr Gln Val Lys Thr Arg Leu Ser Glu Pro Glu Ile
   195 200 205
   Gln Phe His Asn Val Lys Pro Gln Leu Pro Val Thr Pro Glu Asn Leu
   210 215 220
   Ala Ala Ile Asp Leu Ile Arg Gln Arg Leu Ile Asn Glu Phe Val Gly
   225 230 235 240
   Gly Glu Lys Glu Thr Asn Leu Ala Leu Glu Glu Asn Ile Ser Lys Leu
   245 250 255
   Lys Ser Asp Phe Asp Ala Leu Asn Thr His Thr Leu Ala Asn Gly Gly
   260 265 270
   Thr Gln Gly Arg His Leu Ile Thr Asp Lys Gln Ile Ile Ile Tyr Gln
   275 280 285
   Pro Glu Asn Leu Asn Ser Gln Asp Lys Gln Leu Phe Asp Asn Tyr Val
   290 295 300
   Ile Leu Gly Asn Tyr Thr Thr Leu Met Phe Asn Ile Ser Arg Ala Tyr
   305 310 315 320
   Val Leu Glu Lys Asp Pro Thr Gln Lys Ala Gln Leu Lys Gln Met Tyr
   325 330 335
   Leu Leu Met Thr Lys His Leu Leu Asp Gln Gly Phe Val Lys Gly Ser
   340 345 350
   Ala Leu Val Thr Thr His His Trp Gly Tyr Ser Ser Arg Trp Trp Tyr
   355 360 365
   Ile Ser Thr Leu Leu Met Ser Asp Ala Leu Lys Glu Ala Asn Leu Gln
   370 375 380
   Thr Gln Val Tyr Asp Ser Leu Leu Trp Tyr Ser Arg Glu Phe Lys Ser
   385 390 395 400
   Ser Phe Asp Met Lys Val Ser Ala Asp Ser Ser Asp Leu Asp Tyr Phe
   405 410 415
   Asn Thr Leu Ser Arg Gln His Leu Ala Leu Leu Leu Leu Glu Pro Asp
   420 425 430
   Asp Gln Lys Arg Ile Asn Leu Val Asn Thr Phe Ser His Tyr Ile Thr
   435 440 445
   Gly Ala Leu Thr Gln Val Pro Pro Gly Gly Lys Asp Gly Leu Arg Pro
   450 455 460
   Asp Gly Thr Ala Trp Arg His Glu Gly Asn Tyr Pro Gly Tyr Ser Phe
   465 470 475 480
   Pro Ala Phe Lys Asn Ala Ser Gln Leu Ile Tyr Leu Leu Arg Asp Thr
   485 490 495
   Pro Phe Ser Val Gly Glu Ser Gly Trp Asn Ser Leu Lys Lys Ala Met
   500 505 510
   Val Ser Ala Trp Ile Tyr Ser Asn Pro Glu Val Gly Leu Pro Leu Ala
   515 520 525
   Gly Arg His Pro Leu Asn Ser Pro Ser Leu Lys Ser Val Ala Gln Gly
   530 535 540
   Tyr Tyr Trp Leu Ala Met Ser Ala Lys Ser Ser Pro Asp Lys Thr Leu
   545 550 555 560
   Ala Ser Ile Tyr Leu Ala Ile Ser Asp Lys Thr Gln Asn Glu Ser Thr
   565 570 575
   Ala Ile Phe Gly Glu Thr Ile Thr Pro Ala Ser Leu Pro Gln Gly Phe
               580                 585                 590
   Tyr Ala Phe Asn Gly Gly Ala Phe Gly Ile His Arg Trp Gln Asp Lys
           595                 600                 605
   Met Val Thr Leu Lys Ala Tyr Asn Thr Asn Val Trp Ser Ser Glu Ile
       610                 615                 620
   Tyr Asn Lys Asp Asn Arg Tyr Gly Arg Tyr Gln Ser His Gly Val Ala
   625                 630                 635                 640
   Gln Ile Val Ser Asn Gly Ser Gln Leu Ser Gln Gly Tyr Gln Gln Glu
                   645                 650                 655
   Gly Trp Asp Trp Asn Arg Met Pro Gly Ala Thr Thr Ile His Leu Pro
               660                 665                 670
   Leu Lys Asp Leu Asp Ser Pro Lys Pro His Thr Leu Met Gln Arg Gly
           675                 680                 685
   Glu Arg Gly Phe Ser Gly Thr Ser Ser Leu Glu Gly Gln Tyr Gly Met
       690                 695                 700
   Met Ala Phe Asp Leu Ile Tyr Pro Ala Asn Leu Glu Arg Phe Asp Pro
   705                 710                 715                 720
   Asn Phe Thr Ala Lys Lys Ser Val Leu Ala Ala Asp Asn His Leu Ile
                   725                 730                 735
   Phe Ile Gly Ser Asn Ile Asn Ser Ser Asp Lys Asn Lys Asn Val Glu
               740                 745                 750
   Thr Thr Leu Phe Gln His Ala Ile Thr Pro Thr Leu Asn Thr Leu Trp
           755                 760                 765
   Ile Asn Gly Gln Lys Ile Glu Asn Met Pro Tyr Gln Thr Thr Leu Gln
       770                 775                 780
   Gln Gly Asp Trp Leu Ile Asp Ser Asn Gly Asn Gly Tyr Leu Ile Thr
   785                 790                 795                 800
   Gln Ala Glu Lys Val Asn Val Ser Arg Gln His Gln Val Ser Ala Glu
                   805                 810                 815
   Asn Lys Asn Arg Gln Pro Thr Glu Gly Asn Phe Ser Ser Ala Trp Ile
               820                 825                 830
   Asp His Ser Thr Arg Pro Lys Asp Ala Ser Tyr Glu Tyr Met Val Phe
           835                 840                 845
   Leu Asp Ala Thr Pro Glu Lys Met Gly Glu Met Ala Gln Lys Phe Arg
       850                 855                 860
   Glu Asn Asn Gly Leu Tyr Gln Val Leu Arg Lys Asp Lys Asp Val His
   865                 870                 875                 880
   Ile Ile Leu Asp Lys Leu Ser Asn Val Thr Gly Tyr Ala Phe Tyr Gln
                   885                 890                 895
   Pro Ala Ser Ile Glu Asp Lys Trp Ile Lys Lys Val Asn Lys Pro Ala
               900                 905                 910
   Ile Val Met Thr His Arg Gln Lys Asp Thr Leu Ile Val Ser Ala Val
           915                 920                 925
   Thr Pro Asp Leu Asn Met Thr Arg Gln Lys Ala Ala Thr Pro Val Thr
       930                 935                 940
   Ile Asn Val Thr Ile Asn Gly Lys Trp Gln Ser Ala Asp Lys Asn Ser
   945                 950                 955                 960
   Glu Val Lys Tyr Gln Val Ser Gly Asp Asn Thr Glu Leu Thr Phe Thr
                   965                 970                 975
   Ser Tyr Phe Gly Ile Pro Gln Glu Ile Lys Leu Ser Pro Leu Pro
               980                 985                 990
<210> 43
   <211> 2856
   <212> DNA
   <213> Artificial
<220>
   <223> Synthetic polynucleotide, HIV-1 TAT-Chondroitinase ABC I N(delta)60 Nucleic Acid
<220>
   <221> misc_feature
   <222> (948)..(948)
   <223> Unknown nucleotide
<400> 43
   ggtcgtaaaa agcgtcgtca acgtcgtcgt cctcctcaat gctttacttt acataaaaaa      60
   ctgattgtcc ccaccgataa agaagcatct aaagcatggg gacgctcatc cacccccgtt     120
   ttctcatttt ggctttacaa tgaaaaaccg attgatggtt atcttactat cgatttcgga     180
   gaaaaactca tttcaaccag tgaggctcag gcaggcttta aagtaaaatt agatttcact     240
   ggctggcgta ctgtgggagt ctctttaaat aacgatcttg aaaatcgaga gatgacctta     300
   aatgcaacca atacctcctc tgatggtact caagacagca ttgggcgttc tttaggtgct     360
   aaagtcgata gtattcgttt taaagcgcct tctaatgtga gtcagggtga aatctatatc     420
   gaccgtatta tgttttctgt cgatgatgct cgctaccaat ggtctgatta tcaagtaaaa     480
   actcgcttat cagaacctga aattcaattt cacaacgtaa agccacaact acctgtaaca 540
   cctgaaaatt tagcggccat tgatcttatt cgccaacgtc taattaatga atttgtcgga 600
   ggtgaaaaag agacaaacct cgcattagaa gagaatatca gcaaattaaa aagtgatttc 660
   gatgctctta atactcacac tttagcaaat ggtggaacgc aaggcagaca tctgatcact 720
   gataaacaaa tcattattta tcaaccagag aatcttaact ctcaagataa acaactattt 780
   gataattatg ttattttagg taattacacg acattaatgt ttaatattag ccgtgcttat 840
   gtgctggaaa aagatcccac acaaaaggcg caactaaagc agatgtactt attaatgaca 900
   aagcatttat tagatcaagg ctttgttaaa gggagtgctt tagtgacnac ccatcactgg 960
   ggatacagtt ctcgttggtg gtatatttcc acgttattaa tgtctgatgc actaaaagaa 1020
   gcgaacctac aaactcaagt ttatgattca ttactgtggt attcacgtga gtttaaaagt 1080
   agttttgata tgaaagtaag tgctgatagc tctgatctag attatttcaa taccttatct 1140
   cgccaacatt tagccttatt actactagag cctgatgatc aaaagcgtat caacttagtt 1200
   aatactttca gccattatat cactggcgca ttaacgcaag tgccaccggg tggtaaagat 1260
   ggtttacgcc ctgatggtac agcatggcga catgaaggca actatccggg ctactctttc 1320
   ccagccttta aaaatgcctc tcagcttatt tatttattac gcgatacacc attttcagtg 1380
   ggtgaaagtg gttggaatag cctgaaaaaa gcgatggttt cagcgtggat ctacagtaat 1440
   ccagaagttg gattaccgct tgcaggaaga caccctctta actcaccttc gttaaaatca 1500
   gtcgctcaag gctattactg gcttgccatg tctgcaaaat catcgcctga taaaacactt 1560
   gcatctattt atcttgcgat tagtgataaa acacaaaatg aatcaactgc tatttttgga 1620
   gaaactatta caccagcgtc tttacctcaa ggtttctatg cctttaatgg cggtgctttt 1680
   ggtattcatc gttggcaaga taaaatggtg acactgaaag cttataacac caatgtttgg 1740
   tcatctgaaa tttataacaa agataaccgt tatggccgtt accaaagtca tggtgtcgct 1800
   caaatagtga gtaatggctc gcagctttca cagggctatc agcaagaagg ttgggattgg 1860
   aatagaatgc caggggcaac cactatccac cttcctctta aagacttaga cagtcctaaa 1920
   cctcatacct taatgcaacg tggagagcgt ggatttagcg gaacatcatc ccttgaaggt 1980
   caatatggca tgatggcatt cgatcttatt tatcccgcca atcttgagcg ttttgatcct 2040
   aatttcactg cgaaaaagag tgtattagcc gctgataatc acttaatttt tattggtagc 2100
   aatataaata gtagtgataa aaataaaaat gttgaaacga ccttattcca acatgccatt 2160
   actccaacat taaataccct ttggattaat ggacaaaaga tagaaaacat gccttatcaa 2220
   acaacacttc aacaaggtga ttggttaatt gatagcaatg gcaatggtta cttaattact 2280
   caagcagaaa aagtaaatgt aagtcgccaa catcaggttt cagcggaaaa taaaaatcgc 2340
   caaccgacag aaggaaactt tagctcggca tggatcgatc acagcactcg ccccaaagat 2400
   gccagttatg agtatatggt ctttttagat gcgacacctg aaaaaatggg agagatggca 2460
   caaaaattcc gtgaaaataa tgggttatat caggttcttc gtaaggataa agacgttcat 2520
   attattctcg ataaactcag caatgtaacg ggatatgcct tttatcagcc agcatcaatt 2580
   gaagacaaat ggatcaaaaa ggttaataaa cctgcaattg tgatgactca tcgacaaaaa   2640
   gacactctta ttgtcagtgc agttacacct gatttaaata tgactcgcca aaaagcagca   2700
   actcctgtca ccatcaatgt cacgattaat ggcaaatggc aatctgctga taaaaatagt   2760
   gaagtgaaat atcaggtttc tggtgataac actgaactga cgtttacgag ttactttggt   2820
   attccacaag aaatcaaact ctcgccactc ccttga                              2856
<210> 44
   <211> 951
   <212> PRT
   <213> Artificial
<220>
   <223> Synthetic polypeptide, HIV-1 TAT chondroitinase ABCI-N60 fusion polypeptide
<400> 44
   Gly Arg Lys Lys Arg Arg Gln Arg Arg Arg Pro Pro Gln Cys Phe Thr
   1               5                   10                  15
   Leu His Lys Lys Leu Ile Val Pro Thr Asp Lys Glu Ala Ser Lys Ala
               20                  25                  30
   Trp Gly Arg Ser Ser Thr Pro Val Phe Ser Phe Trp Leu Tyr Asn Glu
           35                  40                  45
   Lys Pro Ile Asp Gly Tyr Leu Thr Ile Asp Phe Gly Glu Lys Leu Ile
       50                  55                  60
   Ser Thr Ser Glu Ala Gln Ala Gly Phe Lys Val Lys Leu Asp Phe Thr
   65                  70                  75                  80
   Gly Trp Arg Thr Val Gly Val Ser Leu Asn Asn Asp Leu Glu Asn Arg
                   85                  90                  95
   Glu Met Thr Leu Asn Ala Thr Asn Thr Ser Ser Asp Gly Thr Gln Asp
               100                 105                 110
   Ser Ile Gly Arg Ser Leu Gly Ala Lys Val Asp Ser Ile Arg Phe Lys
           115                 120                 125
   Ala Pro Ser Asn Val Ser Gln Gly Glu Ile Tyr Ile Asp Arg Ile Met
       130                 135                 140
   Phe Ser Val Asp Asp Ala Arg Tyr Gln Trp Ser Asp Tyr Gln Val Lys
   145                 150                 155                 160
   Thr Arg Leu Ser Glu Pro Glu Ile Gln Phe His Asn Val Lys Pro Gln
                   165                 170                 175
   Leu Pro Val Thr Pro Glu Asn Leu Ala Ala Ile Asp Leu Ile Arg Gln
               180                 185                 190
   Arg Leu Ile Asn Glu Phe Val Gly Gly Glu Lys Glu Thr Asn Leu Ala
           195                 200                 205
   Leu Glu Glu Asn Ile Ser Lys Leu Lys Ser Asp Phe Asp Ala Leu Asn
       210                 215                 220
   Thr His Thr Leu Ala Asn Gly Gly Thr Gln Gly Arg His Leu Ile Thr
   225                 230                 235                 240
   Asp Lys Gln Ile Ile Ile Tyr Gln Pro Glu Asn Leu Asn Ser Gln Asp
                   245                 250                 255
   Lys Gln Leu Phe Asp Asn Tyr Val Ile Leu Gly Asn Tyr Thr Thr Leu
               260                 265                 270
   Met Phe Asn Ile Ser Arg Ala Tyr Val Leu Glu Lys Asp Pro Thr Gln
           275                 280                 285
   Lys Ala Gln Leu Lys Gln Met Tyr Leu Leu Met Thr Lys His Leu Leu
       290                 295                 300
   Asp Gln Gly Phe Val Lys Gly Ser Ala Leu Val Thr Thr His His Trp
   305                 310                 315                 320
   Gly Tyr Ser Ser Arg Trp Trp Tyr Ile Ser Thr Leu Leu Met Ser Asp
                   325                 330                 335
   Ala Leu Lys Glu Ala Asn Leu Gln Thr Gln Val Tyr Asp Ser Leu Leu
               340                 345                 350
   Trp Tyr Ser Arg Glu Phe Lys Ser Ser Phe Asp Met Lys Val Ser Ala
           355                 360                 365
   Asp Ser Ser Asp Leu Asp Tyr Phe Asn Thr Leu Ser Arg Gln His Leu
       370                 375                 380
   Ala Leu Leu Leu Leu Glu Pro Asp Asp Gln Lys Arg Ile Asn Leu Val
   385                 390                 395                 400
   Asn Thr Phe Ser His Tyr Ile Thr Gly Ala Leu Thr Gln Val Pro Pro
                   405                 410                 415
   Gly Gly Lys Asp Gly Leu Arg Pro Asp Gly Thr Ala Trp Arg His Glu
               420                 425                 430
   Gly Asn Tyr Pro Gly Tyr Ser Phe Pro Ala Phe Lys Asn Ala Ser Gln
           435                 440                 445
   Leu Ile Tyr Leu Leu Arg Asp Thr Pro Phe Ser Val Gly Glu Ser Gly
       450                 455                 460
   Trp Asn Ser Leu Lys Lys Ala Met Val Ser Ala Trp Ile Tyr Ser Asn
   465 470 475 480
   Pro Glu Val Gly Leu Pro Leu Ala Gly Arg His Pro Leu Asn Ser Pro
   485 490 495
   Ser Leu Lys Ser Val Ala Gln Gly Tyr Tyr Trp Leu Ala Met Ser Ala
   500 505 510
   Lys Ser Ser Pro Asp Lys Thr Leu Ala Ser Ile Tyr Leu Ala Ile Ser
   515 520 525
   Asp Lys Thr Gln Asn Glu Ser Thr Ala Ile Phe Gly Glu Thr Ile Thr
   530 535 540
   Pro Ala Ser Leu Pro Gln Gly Phe Tyr Ala Phe Asn Gly Gly Ala Phe
   545 550 555 560
   Gly Ile His Arg Trp Gln Asp Lys Met Val Thr Leu Lys Ala Tyr Asn
   565 570 575
   Thr Asn Val Trp Ser Ser Glu Ile Tyr Asn Lys Asp Asn Arg Tyr Gly
   580 585 590
   Arg Tyr Gln Ser His Gly Val Ala Gln Ile Val Ser Asn Gly Ser Gln
   595 600 605
   Leu Ser Gln Gly Tyr Gln Gln Glu Gly Trp Asp Trp Asn Arg Met Pro
   610 615 620
   Gly Ala Thr Thr Ile His Leu Pro Leu Lys Asp Leu Asp Ser Pro Lys
   625 630 635 640
   Pro His Thr Leu Met Gln Arg Gly Glu Arg Gly Phe Ser Gly Thr Ser
   645 650 655
   Ser Leu Glu Gly Gln Tyr Gly Met Met Ala Phe Asp Leu Ile Tyr Pro
   660 665 670
   Ala Asn Leu Glu Arg Phe Asp Pro Asn Phe Thr Ala Lys Lys Ser Val
   675 680 685
   Leu Ala Ala Asp Asn His Leu Ile Phe Ile Gly Ser Asn Ile Asn Ser
   690 695 700
   Ser Asp Lys Asn Lys Asn Val Glu Thr Thr Leu Phe Gln His Ala Ile
   705 710 715 720
   Thr Pro Thr Leu Asn Thr Leu Trp Ile Asn Gly Gln Lys Ile Glu Asn
   725 730 735
   Met Pro Tyr Gln Thr Thr Leu Gln Gln Gly Asp Trp Leu Ile Asp Ser
               740                 745                 750
   Asn Gly Asn Gly Tyr Leu Ile Thr Gln Ala Glu Lys Val Asn Val Ser
           755                 760                 765
   Arg Gln His Gln Val Ser Ala Glu Asn Lys Asn Arg Gln Pro Thr Glu
       770                 775                 780
   Gly Asn Phe Ser Ser Ala Trp Ile Asp His Ser Thr Arg Pro Lys Asp
   785                 790                 795                 800
   Ala Ser Tyr Glu Tyr Met Val Phe Leu Asp Ala Thr Pro Glu Lys Met
                   805                 810                 815
   Gly Glu Met Ala Gln Lys Phe Arg Glu Asn Asn Gly Leu Tyr Gln Val
               820                 825                 830
   Leu Arg Lys Asp Lys Asp Val His Ile Ile Leu Asp Lys Leu Ser Asn
           835                 840                 845
   Val Thr Gly Tyr Ala Phe Tyr Gln Pro Ala Ser Ile Glu Asp Lys Trp
       850                 855                 860
   Ile Lys Lys Val Asn Lys Pro Ala Ile Val Met Thr His Arg Gln Lys
   865                 870                 875                 880
   Asp Thr Leu Ile Val Ser Ala Val Thr Pro Asp Leu Asn Met Thr Arg
                   885                 890                 895
   Gln Lys Ala Ala Thr Pro Val Thr Ile Asn Val Thr Ile Asn Gly Lys
               900                 905                 910
   Trp Gln Ser Ala Asp Lys Asn Ser Glu Val Lys Tyr Gln Val Ser Gly
           915                 920                 925
   Asp Asn Thr Glu Leu Thr Phe Thr Ser Tyr Phe Gly Ile Pro Gln Glu
       930                 935                 940
   Ile Lys Leu Ser Pro Leu Pro
   945                 950
<210> 45
   <211> 3036
   <212> DNA
   <213> Artificial
<220>
   <223> Synthetic polynucleotide, C terminal HIV-1 TAT-Chondroitinase ABC I Nucleic acid
<220>
   <221> misc_feature
   <222> (1086)..(1086)
   <223> Unknown nucleotide
<400> 45
   gccaccagca atcctgcatt tgatcctaaa aatctgatgc agtcagaaat ttaccatttt 60
   gcacaaaata acccattagc agacttctca tcagataaaa actcaatact aacgttatct 120
   gataaacgta gcattatggg aaaccaatct cttttatgga aatggaaagg tggtagtagc 180
   tttactttac ataaaaaact gattgtcccc accgataaag aagcatctaa agcatgggga 240
   cgctcatcca cccccgtttt ctcattttgg ctttacaatg aaaaaccgat tgatggttat 300
   cttactatcg atttcggaga aaaactcatt tcaaccagtg aggctcaggc aggctttaaa 360
   gtaaaattag atttcactgg ctggcgtact gtgggagtct ctttaaataa cgatcttgaa 420
   aatcgagaga tgaccttaaa tgcaaccaat acctcctctg atggtactca agacagcatt 480
   gggcgttctt taggtgctaa agtcgatagt attcgtttta aagcgccttc taatgtgagt 540
   cagggtgaaa tctatatcga ccgtattatg ttttctgtcg atgatgctcg ctaccaatgg 600
   tctgattatc aagtaaaaac tcgcttatca gaacctgaaa ttcaatttca caacgtaaag 660
   ccacaactac ctgtaacacc tgaaaattta gcggccattg atcttattcg ccaacgtcta 720
   attaatgaat ttgtcggagg tgaaaaagag acaaacctcg cattagaaga gaatatcagc 780
   aaattaaaaa gtgatttcga tgctcttaat actcacactt tagcaaatgg tggaacgcaa 840
   ggcagacatc tgatcactga taaacaaatc attatttatc aaccagagaa tcttaactct 900
   caagataaac aactatttga taattatgtt attttaggta attacacgac attaatgttt 960
   aatattagcc gtgcttatgt gctggaaaaa gatcccacac aaaaggcgca actaaagcag 1020
   atgtacttat taatgacaaa gcatttatta gatcaaggct ttgttaaagg gagtgcttta 1080
   gtgacnaccc atcactgggg atacagttct cgttggtggt atatttccac gttattaatg 1140
   tctgatgcac taaaagaagc gaacctacaa actcaagttt atgattcatt actgtggtat 1200
   tcacgtgagt ttaaaagtag ttttgatatg aaagtaagtg ctgatagctc tgatctagat 1260
   tatttcaata ccttatctcg ccaacattta gccttattac tactagagcc tgatgatcaa 1320
   aagcgtatca acttagttaa tactttcagc cattatatca ctggcgcatt aacgcaagtg 1380
   ccaccgggtg gtaaagatgg tttacgccct gatggtacag catggcgaca tgaaggcaac 1440
   tatccgggct actctttccc agcctttaaa aatgcctctc agcttattta tttattacgc 1500
   gatacaccat tttcagtggg tgaaagtggt tggaatagcc tgaaaaaagc gatggtttca 1560
   gcgtggatct acagtaatcc agaagttgga ttaccgcttg caggaagaca ccctcttaac 1620
   tcaccttcgt taaaatcagt cgctcaaggc tattactggc ttgccatgtc tgcaaaatca 1680
   tcgcctgata aaacacttgc atctatttat cttgcgatta gtgataaaac acaaaatgaa 1740
   tcaactgcta tttttggaga aactattaca ccagcgtctt tacctcaagg tttctatgcc 1800
   tttaatggcg gtgcttttgg tattcatcgt tggcaagata aaatggtgac actgaaagct 1860
   tataacacca atgtttggtc atctgaaatt tataacaaag ataaccgtta tggccgttac 1920
   caaagtcatg gtgtcgctca aatagtgagt aatggctcgc agctttcaca gggctatcag 1980
   caagaaggtt gggattggaa tagaatgcca ggggcaacca ctatccacct tcctcttaaa   2040
   gacttagaca gtcctaaacc tcatacctta atgcaacgtg gagagcgtgg atttagcgga   2100
   acatcatccc ttgaaggtca atatggcatg atggcattcg atcttattta tcccgccaat   2160
   cttgagcgtt ttgatcctaa tttcactgcg aaaaagagtg tattagccgc tgataatcac   2220
   ttaattttta ttggtagcaa tataaatagt agtgataaaa ataaaaatgt tgaaacgacc   2280
   ttattccaac atgccattac tccaacatta aatacccttt ggattaatgg acaaaagata   2340
   gaaaacatgc cttatcaaac aacacttcaa caaggtgatt ggttaattga tagcaatggc   2400
   aatggttact taattactca agcagaaaaa gtaaatgtaa gtcgccaaca tcaggtttca   2460
   gcggaaaata aaaatcgcca accgacagaa ggaaacttta gctcggcatg gatcgatcac   2520
   agcactcgcc ccaaagatgc cagttatgag tatatggtct ttttagatgc gacacctgaa   2580
   aaaatgggag agatggcaca aaaattccgt gaaaataatg ggttatatca ggttcttcgt   2640
   aaggataaag acgttcatat tattctcgat aaactcagca atgtaacggg atatgccttt   2700
   tatcagccag catcaattga agacaaatgg atcaaaaagg ttaataaacc tgcaattgtg   2760
   atgactcatc gacaaaaaga cactcttatt gtcagtgcag ttacacctga tttaaatatg   2820
   actcgccaaa aagcagcaac tcctgtcacc atcaatgtca cgattaatgg caaatggcaa   2880
   tctgctgata aaaatagtga agtgaaatat caggtttctg gtgataacac tgaactgacg   2940
   tttacgagtt actttggtat tccacaagaa atcaaactct cgccactccc tggtcgtaaa   3000
   aagcgtcgtc aacgtcgtcg tcctcctcaa tgctag                             3036
<210> 46
   <211> 1011
   <212> PRT
   <213> Artificial
<220>
   <223> Synthetic polypeptide, C terminal HIV-1 TAT-Chondroitinase ABC I with gwrt and dalnt sequences
<400> 46
   Ala Thr Ser Asn Pro Ala Phe Asp Pro Lys Asn Leu Met Gln Ser Glu
   1               5                   10                  15
   Ile Tyr His Phe Ala Gln Asn Asn Pro Leu Ala Asp Phe Ser Ser Asp
               20                  25                  30
   Lys Asn Ser Ile Leu Thr Leu Ser Asp Lys Arg Ser Ile Met Gly Asn
           35                  40                  45
   Gln Ser Leu Leu Trp Lys Trp Lys Gly Gly Ser Ser Phe Thr Leu His
       50                  55                  60
   Lys Lys Leu Ile Val Pro Thr Asp Lys Glu Ala Ser Lys Ala Trp Gly
   65                  70                  75                  80
   Arg Ser Ser Thr Pro Val Phe Ser Phe Trp Leu Tyr Asn Glu Lys Pro
   85 90 95
   Ile Asp Gly Tyr Leu Thr Ile Asp Phe Gly Glu Lys Leu Ile Ser Thr
   100 105 110
   Ser Glu Ala Gln Ala Gly Phe Lys Val Lys Leu Asp Phe Thr Gly Trp
   115 120 125
   Arg Thr Val Gly Val Ser Leu Asn Asn Asp Leu Glu Asn Arg Glu Met
   130 135 140
   Thr Leu Asn Ala Thr Asn Thr Ser Ser Asp Gly Thr Gln Asp Ser Ile
   145 150 155 160
   Gly Arg Ser Leu Gly Ala Lys Val Asp Ser Ile Arg Phe Lys Ala Pro
   165 170 175
   Ser Asn Val Ser Gln Gly Glu Ile Tyr Ile Asp Arg Ile Met Phe Ser
   180 185 190
   Val Asp Asp Ala Arg Tyr Gln Trp Ser Asp Tyr Gln Val Lys Thr Arg
   195 200 205
   Leu Ser Glu Pro Glu Ile Gln Phe His Asn Val Lys Pro Gln Leu Pro
   210 215 220
   Val Thr Pro Glu Asn Leu Ala Ala Ile Asp Leu Ile Arg Gln Arg Leu
   225 230 235 240
   Ile Asn Glu Phe Val Gly Gly Glu Lys Glu Thr Asn Leu Ala Leu Glu
   245 250 255
   Glu Asn Ile Ser Lys Leu Lys Ser Asp Phe Asp Ala Leu Asn Thr His
   260 265 270
   Thr Leu Ala Asn Gly Gly Thr Gln Gly Arg His Leu Ile Thr Asp Lys
   275 280 285
   Gln Ile Ile Ile Tyr Gln Pro Glu Asn Leu Asn Ser Gln Asp Lys Gln
   290 295 300
   Leu Phe Asp Asn Tyr Val Ile Leu Gly Asn Tyr Thr Thr Leu Met Phe
   305 310 315 320
   Asn Ile Ser Arg Ala Tyr Val Leu Glu Lys Asp Pro Thr Gln Lys Ala
   325 330 335
   Gln Leu Lys Gln Met Tyr Leu Leu Met Thr Lys His Leu Leu Asp Gln
   340 345 350
   Gly Phe Val Lys Gly Ser Ala Leu Val Thr Thr His His Trp Gly Tyr
   355 360 365
   Ser Ser Arg Trp Trp Tyr Ile Ser Thr Leu Leu Met Ser Asp Ala Leu
   370 375 380
   Lys Glu Ala Asn Leu Gln Thr Gln Val Tyr Asp Ser Leu Leu Trp Tyr
   385 390 395 400
   Ser Arg Glu Phe Lys Ser Ser Phe Asp Met Lys Val Ser Ala Asp Ser
   405 410 415
   Ser Asp Leu Asp Tyr Phe Asn Thr Leu Ser Arg Gln His Leu Ala Leu
   420 425 430
   Leu Leu Leu Glu Pro Asp Asp Gln Lys Arg Ile Asn Leu Val Asn Thr
   435 440 445
   Phe Ser His Tyr Ile Thr Gly Ala Leu Thr Gln Val Pro Pro Gly Gly
   450 455 460
   Lys Asp Gly Leu Arg Pro Asp Gly Thr Ala Trp Arg His Glu Gly Asn
   465 470 475 480
   Tyr Pro Gly Tyr Ser Phe Pro Ala Phe Lys Asn Ala Ser Gln Leu Ile
   485 490 495
   Tyr Leu Leu Arg Asp Thr Pro Phe Ser Val Gly Glu Ser Gly Trp Asn
   500 505 510
   Ser Leu Lys Lys Ala Met Val Ser Ala Trp Ile Tyr Ser Asn Pro Glu
   515 520 525
   Val Gly Leu Pro Leu Ala Gly Arg His Pro Leu Asn Ser Pro Ser Leu
   530 535 540
   Lys Ser Val Ala Gln Gly Tyr Tyr Trp Leu Ala Met Ser Ala Lys Ser
   545 550 555 560
   Ser Pro Asp Lys Thr Leu Ala Ser Ile Tyr Leu Ala Ile Ser Asp Lys
   565 570 575
   Thr Gln Asn Glu Ser Thr Ala Ile Phe Gly Glu Thr Ile Thr Pro Ala
   580 585 590
   Ser Leu Pro Gln Gly Phe Tyr Ala Phe Asn Gly Gly Ala Phe Gly Ile
   595 600 605
   His Arg Trp Gln Asp Lys Met Val Thr Leu Lys Ala Tyr Asn Thr Asn
   610 615 620
   Val Trp Ser Ser Glu Ile Tyr Asn Lys Asp Asn Arg Tyr Gly Arg Tyr
   625 630 635 640
   Gln Ser His Gly Val Ala Gln Ile Val Ser Asn Gly Ser Gln Leu Ser
   645 650 655
   Gln Gly Tyr Gln Gln Glu Gly Trp Asp Trp Asn Arg Met Pro Gly Ala
   660 665 670
   Thr Thr Ile His Leu Pro Leu Lys Asp Leu Asp Ser Pro Lys Pro His
   675 680 685
   Thr Leu Met Gln Arg Gly Glu Arg Gly Phe Ser Gly Thr Ser Ser Leu
   690 695 700
   Glu Gly Gln Tyr Gly Met Met Ala Phe Asp Leu Ile Tyr Pro Ala Asn
   705 710 715 720
   Leu Glu Arg Phe Asp Pro Asn Phe Thr Ala Lys Lys Ser Val Leu Ala
   725 730 735
   Ala Asp Asn His Leu Ile Phe Ile Gly Ser Asn Ile Asn Ser Ser Asp
   740 745 750
   Lys Asn Lys Asn Val Glu Thr Thr Leu Phe Gln His Ala Ile Thr Pro
   755 760 765
   Thr Leu Asn Thr Leu Trp Ile Asn Gly Gln Lys Ile Glu Asn Met Pro
   770 775 780
   Tyr Gln Thr Thr Leu Gln Gln Gly Asp Trp Leu Ile Asp Ser Asn Gly
   785 790 795 800
   Asn Gly Tyr Leu Ile Thr Gln Ala Glu Lys Val Asn Val Ser Arg Gln
   805 810 815
   His Gln Val Ser Ala Glu Asn Lys Asn Arg Gln Pro Thr Glu Gly Asn
   820 825 830
   Phe Ser Ser Ala Trp Ile Asp His Ser Thr Arg Pro Lys Asp Ala Ser
   835 840 845
   Tyr Glu Tyr Met Val Phe Leu Asp Ala Thr Pro Glu Lys Met Gly Glu
   850 855 860
   Met Ala Gln Lys Phe Arg Glu Asn Asn Gly Leu Tyr Gln Val Leu Arg
   865 870 875 880
   Lys Asp Lys Asp Val His Ile Ile Leu Asp Lys Leu Ser Asn Val Thr
   885 890 895
   Gly Tyr Ala Phe Tyr Gln Pro Ala Ser Ile Glu Asp Lys Trp Ile Lys
   900 905 910
   Lys Val Asn Lys Pro Ala Ile Val Met Thr His Arg Gln Lys Asp Thr
           915                 920                 925
   Leu Ile Val Ser Ala Val Thr Pro Asp Leu Asn Met Thr Arg Gln Lys
       930                 935                 940
   Ala Ala Thr Pro Val Thr Ile Asn Val Thr Ile Asn Gly Lys Trp Gln
   945                 950                 955                 960
   Ser Ala Asp Lys Asn Ser Glu Val Lys Tyr Gln Val Ser Gly Asp Asn
                   965                 970                 975
   Thr Glu Leu Thr Phe Thr Ser Tyr Phe Gly Ile Pro Gln Glu Ile Lys
               980                 985                 990
   Leu Ser Pro Leu Pro Gly Arg Lys  Lys Arg Arg Gln Arg  Arg Arg Pro
           995                 1000                 1005
   Pro Gln  Cys
       1010
<210> 47
   <211> 2934
   <212> DNA
   <213> Artificial
<220>
   <223> Synthetic polynucleotide, chondroitinase ABCI-N(delta)20
<220>
   <221> misc_feature
   <222> (1026)..(1026)
   <223> Unknown nucleotide
<400> 47
   gcacaaaata acccattagc agacttctca tcagataaaa actcaatact aacgttatct 60
   gataaacgta gcattatggg aaaccaatct cttttatgga aatggaaagg tggtagtagc 120
   tttactttac ataaaaaact gattgtcccc accgataaag aagcatctaa agcatgggga 180
   cgctcatcca cccccgtttt ctcattttgg ctttacaatg aaaaaccgat tgatggttat 240
   cttactatcg atttcggaga aaaactcatt tcaaccagtg aggctcaggc aggctttaaa 300
   gtaaaattag atttcactgg ctggcgtact gtgggagtct ctttaaataa cgatcttgaa 360
   aatcgagaga tgaccttaaa tgcaaccaat acctcctctg atggtactca agacagcatt 420
   gggcgttctt taggtgctaa agtcgatagt attcgtttta aagcgccttc taatgtgagt 480
   cagggtgaaa tctatatcga ccgtattatg ttttctgtcg atgatgctcg ctaccaatgg 540
   tctgattatc aagtaaaaac tcgcttatca gaacctgaaa ttcaatttca caacgtaaag 600
   ccacaactac ctgtaacacc tgaaaattta gcggccattg atcttattcg ccaacgtcta 660
   attaatgaat ttgtcggagg tgaaaaagag acaaacctcg cattagaaga gaatatcagc 720
   aaattaaaaa gtgatttcga tgctcttaat actcacactt tagcaaatgg tggaacgcaa 780
   ggcagacatc tgatcactga taaacaaatc attatttatc aaccagagaa tcttaactct    840
   caagataaac aactatttga taattatgtt attttaggta attacacgac attaatgttt    900
   aatattagcc gtgcttatgt gctggaaaaa gatcccacac aaaaggcgca actaaagcag    960
   atgtacttat taatgacaaa gcatttatta gatcaaggct ttgttaaagg gagtgcttta   1020
   gtgacnaccc atcactgggg atacagttct cgttggtggt atatttccac gttattaatg   1080
   tctgatgcac taaaagaagc gaacctacaa actcaagttt atgattcatt actgtggtat   1140
   tcacgtgagt ttaaaagtag ttttgatatg aaagtaagtg ctgatagctc tgatctagat   1200
   tatttcaata ccttatctcg ccaacattta gccttattac tactagagcc tgatgatcaa   1260
   aagcgtatca acttagttaa tactttcagc cattatatca ctggcgcatt aacgcaagtg   1320
   ccaccgggtg gtaaagatgg tttacgccct gatggtacag catggcgaca tgaaggcaac   1380
   tatccgggct actctttccc agcctttaaa aatgcctctc agcttattta tttattacgc   1440
   gatacaccat tttcagtggg tgaaagtggt tggaatagcc tgaaaaaagc gatggtttca   1500
   gcgtggatct acagtaatcc agaagttgga ttaccgcttg caggaagaca ccctcttaac   1560
   tcaccttcgt taaaatcagt cgctcaaggc tattactggc ttgccatgtc tgcaaaatca   1620
   tcgcctgata aaacacttgc atctatttat cttgcgatta gtgataaaac acaaaatgaa   1680
   tcaactgcta tttttggaga aactattaca ccagcgtctt tacctcaagg tttctatgcc   1740
   tttaatggcg gtgcttttgg tattcatcgt tggcaagata aaatggtgac actgaaagct   1800
   tataacacca atgtttggtc atctgaaatt tataacaaag ataaccgtta tggccgttac   1860
   caaagtcatg gtgtcgctca aatagtgagt aatggctcgc agctttcaca gggctatcag   1920
   caagaaggtt gggattggaa tagaatgcca ggggcaacca ctatccacct tcctcttaaa   1980
   gacttagaca gtcctaaacc tcatacctta atgcaacgtg gagagcgtgg atttagcgga   2040
   acatcatccc ttgaaggtca atatggcatg atggcattcg atcttattta tcccgccaat   2100
   cttgagcgtt ttgatcctaa tttcactgcg aaaaagagtg tattagccgc tgataatcac   2160
   ttaattttta ttggtagcaa tataaatagt agtgataaaa ataaaaatgt tgaaacgacc   2220
   ttattccaac atgccattac tccaacatta aatacccttt ggattaatgg acaaaagata   2280
   gaaaacatgc cttatcaaac aacacttcaa caaggtgatt ggttaattga tagcaatggc   2340
   aatggttact taattactca agcagaaaaa gtaaatgtaa gtcgccaaca tcaggtttca   2400
   gcggaaaata aaaatcgcca accgacagaa ggaaacttta gctcggcatg gatcgatcac   2460
   agcactcgcc ccaaagatgc cagttatgag tatatggtct ttttagatgc gacacctgaa   2520
   aaaatgggag agatggcaca aaaattccgt gaaaataatg ggttatatca ggttcttcgt   2580
   aaggataaag acgttcatat tattctcgat aaactcagca atgtaacggg atatgccttt   2640
   tatcagccag catcaattga agacaaatgg atcaaaaagg ttaataaacc tgcaattgtg   2700
   atgactcatc gacaaaaaga cactcttatt gtcagtgcag ttacacctga tttaaatatg   2760
   actcgccaaa aagcagcaac tcctgtcacc atcaatgtca cgattaatgg caaatggcaa   2820
   tctgctgata aaaatagtga agtgaaatat caggtttctg gtgataacac tgaactgacg   2880
   tttacgagtt actttggtat tccacaagaa atcaaactct cgccactccc ttga 2934
<210> 48
   <211> 2814
   <212> DNA
   <213> Artificial
<220>
   <223> Synthetic polynucleotide, chondroitinase ABCI-N(delta)60
<220>
   <221> misc_feature
   <222> (906)..(906)
   <223> Unknown nucleotide
<400> 48
   tttactttac ataaaaaact gattgtcccc accgataaag aagcatctaa agcatgggga      60
   cgctcatcca cccccgtttt ctcattttgg ctttacaatg aaaaaccgat tgatggttat     120
   cttactatcg atttcggaga aaaactcatt tcaaccagtg aggctcaggc aggctttaaa     180
   gtaaaattag atttcactgg ctggcgtact gtgggagtct ctttaaataa cgatcttgaa     240
   aatcgagaga tgaccttaaa tgcaaccaat acctcctctg atggtactca agacagcatt     300
   gggcgttctt taggtgctaa agtcgatagt attcgtttta aagcgccttc taatgtgagt     360
   cagggtgaaa tctatatcga ccgtattatg ttttctgtcg atgatgctcg ctaccaatgg     420
   tctgattatc aagtaaaaac tcgcttatca gaacctgaaa ttcaatttca caacgtaaag     480
   ccacaactac ctgtaacacc tgaaaattta gcggccattg atcttattcg ccaacgtcta     540
   attaatgaat ttgtcggagg tgaaaaagag acaaacctcg cattagaaga gaatatcagc     600
   aaattaaaaa gtgatttcga tgctcttaat actcacactt tagcaaatgg tggaacgcaa     660
   ggcagacatc tgatcactga taaacaaatc attatttatc aaccagagaa tcttaactct     720
   caagataaac aactatttga taattatgtt attttaggta attacacgac attaatgttt     780
   aatattagcc gtgcttatgt gctggaaaaa gatcccacac aaaaggcgca actaaagcag     840
   atgtacttat taatgacaaa gcatttatta gatcaaggct ttgttaaagg gagtgcttta     900
   gtgacnaccc atcactgggg atacagttct cgttggtggt atatttccac gttattaatg     960
   tctgatgcac taaaagaagc gaacctacaa actcaagttt atgattcatt actgtggtat    1020
   tcacgtgagt ttaaaagtag ttttgatatg aaagtaagtg ctgatagctc tgatctagat    1080
   tatttcaata ccttatctcg ccaacattta gccttattac tactagagcc tgatgatcaa    1140
   aagcgtatca acttagttaa tactttcagc cattatatca ctggcgcatt aacgcaagtg    1200
   ccaccgggtg gtaaagatgg tttacgccct gatggtacag catggcgaca tgaaggcaac    1260
   tatccgggct actctttccc agcctttaaa aatgcctctc agcttattta tttattacgc    1320
   gatacaccat tttcagtggg tgaaagtggt tggaatagcc tgaaaaaagc gatggtttca    1380
   gcgtggatct acagtaatcc agaagttgga ttaccgcttg caggaagaca ccctcttaac    1440
   tcaccttcgt taaaatcagt cgctcaaggc tattactggc ttgccatgtc tgcaaaatca    1500
   tcgcctgata aaacacttgc atctatttat cttgcgatta gtgataaaac acaaaatgaa    1560
   tcaactgcta tttttggaga aactattaca ccagcgtctt tacctcaagg tttctatgcc    1620
   tttaatggcg gtgcttttgg tattcatcgt tggcaagata aaatggtgac actgaaagct    1680
   tataacacca atgtttggtc atctgaaatt tataacaaag ataaccgtta tggccgttac    1740
   caaagtcatg gtgtcgctca aatagtgagt aatggctcgc agctttcaca gggctatcag    1800
   caagaaggtt gggattggaa tagaatgcca ggggcaacca ctatccacct tcctcttaaa    1860
   gacttagaca gtcctaaacc tcatacctta atgcaacgtg gagagcgtgg atttagcgga    1920
   acatcatccc ttgaaggtca atatggcatg atggcattcg atcttattta tcccgccaat    1980
   cttgagcgtt ttgatcctaa tttcactgcg aaaaagagtg tattagccgc tgataatcac    2040
   ttaattttta ttggtagcaa tataaatagt agtgataaaa ataaaaatgt tgaaacgacc   2100
   ttattccaac atgccattac tccaacatta aatacccttt ggattaatgg acaaaagata   2160
   gaaaacatgc cttatcaaac aacacttcaa caaggtgatt ggttaattga tagcaatggc    2220
   aatggttact taattactca agcagaaaaa gtaaatgtaa gtcgccaaca tcaggtttca    2280
   gcggaaaata aaaatcgcca accgacagaa ggaaacttta gctcggcatg gatcgatcac    2340
   agcactcgcc ccaaagatgc cagttatgag tatatggtct ttttagatgc gacacctgaa    2400
   aaaatgggag agatggcaca aaaattccgt gaaaataatg ggttatatca ggttcttcgt    2460
   aaggataaag acgttcatat tattctcgat aaactcagca atgtaacggg atatgccttt    2520
   tatcagccag catcaattga agacaaatgg atcaaaaagg ttaataaacc tgcaattgtg    2580
   atgactcatc gacaaaaaga cactcttatt gtcagtgcag ttacacctga tttaaatatg    2640
   actcgccaaa aagcagcaac tcctgtcacc atcaatgtca cgattaatgg caaatggcaa    2700
   tctgctgata aaaatagtga agtgaaatat caggtttctg gtgataacac tgaactgacg    2760
   tttacgagtt actttggtat tccacaagaa atcaaactct cgccactccc ttga          2814
<210> 49
   <211> 39
   <212> DNA
   <213> Artificial
<220>
   <223> Nucleotide sequence for TAT
<400> 49
   ggtcgtaaaa agcgtcgtca acgtcgtcct cctcaatgc 39
<210> 50
   <211> 14
   <212> PRT
   <213> Artificial
<220>
   <223> Amino acid sequence for a TAT peptide
<400> 50
   Gly Arg Lys Lys Arg Arg Gln Arg Arg Arg Pro Pro Gln Cys
   1 5 10
<210> 51
   <211> 16
   <212> PRT
   <213> Artificial
<220>
   <223> Synthetic polypeptide, amino acids 35-50 from LOCUS (NP_057854) Rev [Human immunodeficiency virus 1], HIV-1 Rev protein basic motif, amino acids
<400> 51
   Arg Gln Ala Arg Arg Asn Arg Arg Arg Arg Trp Arg Glu Arg Gln Arg
   1 5 10 15
<210> 52
   <211> 19
   <212> PRT
   <213> Artificial
<220>
   <223> Synthetic polypeptide, amino acids 1-19 from LOCUS (NP_057863) [Human T-lymphotropic virus 1]
<400> 52
   Met Pro Lys Thr Arg Arg Arg Pro Arg Arg Ser Gln Arg Lys Arg Pro
   1 5 10 15
   Pro Thr Pro
<210> 53
   <211> 16
   <212> PRT
   <213> Artificial
<220>
   <223> Synthetic polypeptide, the third helix of the homeodomain of Antennapedia (Derossi, et al., J. Biol. Chem. 271:18188-93, 1996)
<400> 53
   Arg Gln Ile Lys Ile Trp Phe Gln Asn Arg Arg Met Lys Trp Lys Lys
   1 5 10 15
<210> 54
   <211> 30
   <212> PRT
   <213> Artificial
<220>
   <223> Synthetic polypeptide, derivable from the heavy chain variable region of an anti-DNA monoclonal antibody LOCUS (AAC40104)
<400> 54
   Val Ala Tyr Ile Ser Arg Gly Gly Val Ser Thr Tyr Tyr Ser Asp Thr
   1               5                   10                  15
   Val Lys Gly Arg Phe Thr Arg Gln Lys Tyr Asn Lys Arg Ala
               20                  25                  30
<210> 55
   <211> 246
   <212> PRT
   <213> human herpesvirus
<220>
   <223> Herpes simplex virus VP22 protein (Elliott and O'Hare, Cell, 88:223-33, 1997)
<400> 55
   Met Thr Ser Arg Arg Ser Val Lys Ser Gly Pro Arg Glu Val Pro Arg
   1               5                   10                  15
   Asp Glu Tyr Glu Asp Leu Tyr Tyr Thr Pro Ser Ser Gly Met Ala Ser
               20                  25                  30
   Pro Asp Ser Pro Pro Asp Thr Ser Arg Arg Gly Ala Leu Gln Thr Arg
           35                  40                  45
   Ser Arg Gln Arg Gly Glu Val Arg Phe Val Gln Tyr Asp Glu Ser Asp
       50                  55                  60
   Tyr Ala Leu Tyr Gly Gly Ser Ser Ser Glu Asp Asp Glu His Pro Glu
   65                  70                  75                  80
   Val Pro Arg Thr Arg Arg Pro Val Ser Gly Ala Val Leu Ser Gly Pro
                   85                  90                  95
   Gly Pro Ala Arg Ala Pro Pro Pro Pro Ala Gly Ser Gly Gly Ala Gly
               100                 105                 110
   Arg Thr Pro Thr Thr Ala Pro Arg Ala Pro Arg Thr Gln Arg Val Ala
           115                 120                 125
   Thr Lys Ala Pro Ala Ala Pro Ala Ala Glu Thr Thr Arg Gly Arg Lys
       130                 135                 140
   Ser Ala Gln Pro Glu Ser Ala Ala Leu Pro Asp Ala Pro Ala Ser Arg
   145                 150                 155                 160
   Ala Pro Thr Val Gln Leu Trp Gln Met Ser Arg Pro Arg Thr Asp Glu
                   165                 170                 175
   Asp Leu Asn Glu Leu Leu Gly Ile Thr His Arg Val Thr Val Cys Glu
               180                 185                 190
   Gly Lys Asn Leu Leu Gln Arg Ala Asn Glu Leu Val Asn Pro Asp Val
           195                 200                 205
   Val Gln Asp Val Asp Ala Ala Thr Ala Thr Arg Gly Arg Ser Ala Ala
       210                 215                 220
   Ser Arg Pro Thr Glu Arg Pro Arg Ala Pro Ala Arg Ser Ala Ser Arg
   225                 230                 235                 240
   Pro Arg Arg Pro Val Glu
                   245
<210> 56
   <211> 36
   <212> PRT
   <213> Artificial
<220>
   <223> Synthetic peptide, sequential residues of the Tat protein basic peptide motif 37-72 (Vives, et al., 1997)
<400> 56
   Cys Phe Ile Thr Lys Ala Leu Gly Ile Ser Tyr Gly Arg Lys Lys Arg
   1               5                   10                  15
   Arg Gln Arg Arg Arg Pro Pro Gln Gly Ser Gln Thr His Gln Val Ser
               20                  25                  30
   Leu Ser Lys Gln
           35
<210> 57
   <211> 10
   <212> PRT
   <213> Artificial
<220>
   <223> Synthetic polypeptide, amino acid residues 48-57 of (HIV-1) Tat protein
<400> 57
   Gly Arg Lys Lys Arg Arg Gln Arg Arg Arg
   1               5                   10
<210> 58
   <211> 40
   <212> PRT
   <213> Artificial
<220>
   <223> Synthetic polypeptide corresponding to amino acids 821-862 from Nogo-A protein short form (homo sapiens) LOCUS (AAG40878)
<400> 58
   Arg Ile Tyr Lys Gly Val Ile Gln Ala Ile Gln Lys Ser Asp Glu Gly
   1               5                   10                  15
   His Pro Phe Arg Ala Tyr Leu Glu Ser Glu Val Ala Ile Ser Glu Glu
               20                  25                  30
   Leu Val Gln Lys Tyr Ser Asn Ser
           35                  40
<210> 59
   <211> 5
   <212> PRT
   <213> Artificial
<220>
   <223> Synthetic polypeptide linker
<400> 59
   Gly Gly Gly Gly Gly
   1 5
<210> 60
   <211> 5
   <212> PRT
   <213> Artificial
<220>
   <223> Synthetic polypeptide linker
<400> 60
   Gly Gly Ala Gly Gly
   1 5
<210> 61
   <211> 51
   <212> DNA
   <213> Artificial
<220>
   <223> chemically synthesised sense oligonucleotide
<400> 61
   tatgtatggt cgtaaaaagc gtcgtcaacg tcgtcgtggt ggtggtggtc a 51
<210> 62
   <211> 54
   <212> DNA
   <213> Artificial
<220>
   <223> chemically synthesised antisense oligonucleotide
<400> 62
   tatgaccacc accaccacca cgacgacgtt gacgacgctt tttacgacca taca 54
<210> 63
   <211> 9
   <212> PRT
   <213> Artificial
<220>
   <223> TAT (sub) 48 - 57 peptide
<400> 63
   Gly Arg Lys Lys Arg Arg Gln Arg Arg
   1 5
<210> 64
   <211> 27
   <212> DNA
   <213> Artificial
<220>
   <223> chemically synthesised sense oligonucleotide with NdeI restriction site
<400> 64
   catatggcca ccagcmtcct gcatttg 27
<210> 65
   <211> 24
   <212> DNA
   <213> Artificial
<220>
   <223> chemically synthesised antisense oligonucleotide with BamHI restriction site
<400> 65
   ggatcctcaa gggagtggcg agag 24

## Description

### BACKGROUND AND SUMMARY

Spinal cord injury (SCI) inflicts trauma to the cells and tissues of the central nervous system (CNS) and causes a severe and debilitating condition in the individual. Following SCI, limited regeneration of injured neurons results in permanent disability characterized by some loss of sensation, paralysis and autonomic dysfunction. One reason that neurons fail to regenerate is their inability to traverse the glial scar that develops following SCI. This glial scar contains extracellular matrix molecules including chondroitin sulfate proteoglycans (CSPGs). In vitro studies show that neurons fail to extend processes over CSPG coated surfaces, while in vivo data correlate failure of regeneration with areas of CSPG expression. Within the adult central nervous system (CNS) myelin there are also several identified axon growth inhibitor compounds like myelin associated glycoprotein (MAG), OMgp, and Reticulon 4 or Nogo that have been shown to be inhibitory for the growth of neurons.

The proteglycan degrading enzyme chondroitinase ABC type I (SEQ ID NO: X) has been used to enhance neuronal growth in a dorsal column lesion model of spinal cord injury. It has also been reported that treating a spinal chord injury with NOGO receptor antagonist promotes a certain limited degree of neuronal regeneration. It has further been reported that creating a NOGO knock out mouse resulted in certain limited and inconsistent degrees of neuronal regeneration following dorsal hemisection of the spinal cord.

Experimental treatments for injury to the CNS have utilized the application of chondroitinase to the extracellular space, however the enzyme digests CSPG in the extracellular matrix and not intracellular stores. Furthermore, diffusion of chondroitinase within the parenchyma the essential and distinctive tissue of an organ or an abnormal growth as distinguished from its supportive framework) or between anatomical compartments is limited. The limited access of drugs, imaging agents, and anesthetics, etc. to target cells and/or tissues of the Central Nervous System (CNS) may reduce the usefulness or effectiveness of any of these substances.

The delivery of theraprutic and diagnostic molecules into cells and tissues is, in part, dependent upon extracellular matrices as well as carbohydrates and proteins linked to cell membranes. The extracellular matrix is composed in part of proteoglycans, among them are the chondroitin sulfated proteoglycans (CSPGs). CSPGs are a family of proteoglycans composed of a core protein and covalently linked sulfated glycosaminoglycans. Each proteoglycan is determined by the glycosaminoglycan side chains. For CSPGs these side chains are made up of approximately 40 to 100 sulfated disaccharides composed of chondroitin 4, 6 and dermatan sulfates. The protein component of the CSPG is ribosomally synthesized and the glycosylation occurs in the endoplasmic reticulum and Golgi apparatus. The sugar chains are then sulfated at the 4 or 6 positions by several glycosaminoglycan sulfotransferases.

Transduction proteins may be used to transport polypeptides and polynucleotides cargo across anatomical barriers and into cells. For example, the TAT protein from the human immunodeficiency virus (shiv) contains a protein transduction domain (PTD) that is involved in the transduction of HIV into cells. The PTD contains an 11 amino acid domain (TAT Peptide) that is responsible for the activity of the PTD. The TAT Peptide can be linked to proteins and facilitate the transduction of the proteins into cells. The mechanism of transduction is independent of the molecular weight or chemical properties of the proteins that are linked to the TAT Peptide. In vivo studies show that if a fusion protein consisting of the TAT Peptide linked to the 120 kd enzyme, beta-galactosidase (β-Gal), is injected into mice, then a robust delivery of β-Gal into a wide variety of cells is observed. When linked to the TAT transduction peptide, β-Gal activity was observed in the brain; without the TAT Peptide, β-Gal was not observed in the brain. Transport across the blood brain barrier is normally restricted to certain hydrophobic small molecules and particular low molecular weight lipophilic peptides. Transport of proteins as large as β -Gal into the brain is usually not possible without substantial disruption of the blood brain barrier, but the TAT Peptide facilitates transport while leaving the blood brain barrier intact.

Chimeric proteins, also called fusion proteins, are hybrid proteins which combine at least parts of two or more precursor proteins or polypeptides. Chimeric proteins may be produced by recombinant technology, i.e. by fusing at least a part of the coding sequence of one gene to at least a part of the coding sequence of another gene. The fused gene may then be used to transform a suitable organism which then expresses the fusion protein.

Tat Peptide Complexes Frankel et al. (U.S. Pat. Nos. 5,804,604; 5,747,641; 5,674,980; 5,670,617; 5,652,122) discloses the use of Tat peptides to transport covalently linked biologically active cargo molecules into the cytoplasm and nuclei of cells. Frankel only discloses covalently linked cargo moieties that are (therapeutic, diagnostic or prophylactic), and does not teach or suggest the attachment of molecules that facilitate diffusion, plasticity, neurite growth, and axon regeneration. These molecules can include but are not limited to molecules that over come neurite out growth inhibition, or promote nerve growth such as soluble NOGO antagonists like NgR_{27-311,} neural cell adhesion molecules like L1, neurotrophic factors, growth factors, phosphodiesterase inhibitors, and inhibitors of MAG or MOG. Additionally, deletion mutants may be combined with other compounds that promote remyelination such as neuregulins . (GGF2) and antibodies that promote remyelination. or proteoglycan degrading molecules to Tat peptides.

Regeneration following SCI is limited because of a variety of obstacles that include the deposition of CSPG at the glial scar, demyelination of axons, lack of trophic support and lack of axonal guidance. A single therapy directed against one aspect of SCI may not be as effective as a combinatorial approach. Fusion proteins with chondroitinase will allow combinatorial therapy with a single protein. Fusion partners for chondroitinase that will be constructed in this proposal were chosen from among proteins that have evidence for efficacy in SCI.

The use of a molecule that has the ability to both degrade extracellular matrix glycoproteins and to block or overcome the inhibitory nature of myelin components may be used to improve the ability of damaged neurons to grow or regenerate compared with either treatment alone. The proteoglycan degrading molecules may also be used advantageously to provide a method of facilitating access and diffusion of substances into cells or tissues through the use of at least one enzyme capable of cleaving proteoglycans and preferably degrading chondroitin sulfate proteoglycans (CSPG).

According to the first aspect of the present invention, there are provided compositions comprising a polypeptide comprising a TAT protein transduction domain and a deletion mutant of chondroitinase ABC 1, wherein the TAT protein transduction domain is attached to the N-terminus of the chondroitinase ABC 1 deletion mutant and the chondroitinase ABC 1 deletion mutant is selected from SEQ ID NO: 2 and SEQ ID NO: 3. Embodiments of the present invention therefore include compositions that comprise polypeptides which cleave proteoglycans, optionally the compositions can additionally comprise polypeptides that block and/or over come the activity of neuronal growth inhibitory molecules. The compositions also include molecules for transduction of the polypeptides across cell membranes and the blood brain barrier. The compositions may be used in the treatment of spinal cord injuries and related disorders of the central nervous system (CNS). The compositions can be used in the regeneration of damaged neurological tissue and facilitate the diffusion and transport of therapeutic molecules capable of blocking and/or over coming the activity of neuronal growth inhibitory molecules into damaged or diseased tissue.

Embodiments of the present invention include compositions that facilitate delivery and diffusion of therapeutics or diagnostic agents, and preferably agents that promote regeneration of nerves and axons, into cells or tissues. The composition includes the use of an enzyme capable of cleaving chondroitin sulfate proteoglycans (CSPG) to increase the diffusion of these agents into cells or tissues of the central nervous system.

Compositions of the present invention comprise chimeric or fusion proteins that may be capable of systemic use in the treatment of spinal cord injuries and related disorders of the central nervous system (CNS), and in particular, fusion proteins capable of crossing the blood brain barrier. The fusion protein includes a polypeptide transduction domain, a polypeptide domain capable of cleaving chondroitin sulfate proteoglycan (CSPG), and optionally a polypeptide domain that blocks and or over comes the activity of neuronal growth inhibitory molecules, all of which may be used in the treatment of spinal cord injuries and related disorders of the central nervous system (CNS). The various polypeptide domains may be linked or chemically bonded together by polypeptide linkers.

Polynucleotides which encode for the chimeric or fusion proteins capable of systemic use in the treatment of spinal cord injuries and related disorders of the central nervous system (CNS) are also contemplated, and in particular, they encode for fusion proteins capable of crossing the blood brain barrier. The polynucleotides which encode for these chimeric or fusion proteins may include a polynucleotide domain encoding for a polypeptide transduction domain, a polynucleotide domain encoding for a polypeptide domain capable of degrading a proteoglucan, preferably cleaving chondroitin sulfate proteoglycan (CSPG), a polynucleotide domain encoding for a polypeptide domain that blocks and or over comes the activity of neuronal growth inhibitory molecules, or any combination of these domains that may be used in the treatment of spinal cord injuries and related disorders of the central nervous system (CNS). The polynucleotide also includes one or more polynucleotide domains that encode for polypeptides that link the domains of the polypeptide together to form the fusion protein.

One embodiment of the present invention is a composition according to the first aspect that facilitates the access and distribution of therapeutic and diagnostic agent in the composition into cells, through membranes or into tissues by the use of the enzyme capable of cleaving CSPG's. The molecules or agents in the composition may include one or more of Growth factors including, Brain Derived Neurotrophic Factor, Insulin-like Growth Factor, Fibroblast Growth Factor, Ciliary Neurotrophic Factor, Glial Derived Neurotrophic Factor, Transforming Growth Factor, Glial Growth Factor 2, L1, GM1, Vascular Endothelial Growth Factor, Nerve Growth Factor, Immunophilins. Molecules in the composition can include fluorescent or contrast agents for imaging. The agents may include cells for transplant-stem cells, neurons, others, cells as delivery agents, chemotherapeutic agents, antibiotics, antibody therapies, Nogo receptor antagonists, other chondroitinase enzymes. The fusion protein can facilitate the transport or modifies transport of such agents into cells, tissues, and/or otherwise inaccessible locations; and/or to enhance penetration rates, distance of penetration; or provide more even concentration distribution. The modified transport occurs through the use of at least one enzyme capable of cleaving CSPGs. The compositions can be used for treating a CNS injury, preferably the composition is used in the treatment of neuronal damage from a contusion injury.

Contemplated are chimeric proteins of a proteoglycan degrading domain linked to a polypeptide that blocks the action of neuronal growth inhibitors such as but not limited to a Nogo-receptor antagonist (NgR27311) domain or variant linked to a chondroitinase like chondroitinase ABC I or a variant of chondroitinase having one or more N terminal amino acids deleted. The compound may include chimeric proteins of a proteoglycan degrading domain linked to a polypeptide that is a neural cell adhesion promoter such as an LI neural cell adhesion promoter domain or variant linked to chondroitinase ABC I or a variant of chondroitinase having one or more N terminal amino acids deleted. The chimeric proteins may include chimeric proteins of a proteoglycan degrading domain linked to a polypeptide that is a glial cell stimulator, such as but not limited to a GGF2 glial cell stimulator or variant linked to chondroitinase ABCI or a variant of chondroitinase having one or more N terminal amino acids deleted.

An E. Coli recombinant expression system and purification process can be used to produce essentially pure and catalytically active chondroitinase ABCI. These methods may be modified for producing chimeras of proteoglycan degrading molecules and other agents.

The chimera may be assayed for chondroitinase enzymatic activity and the specific biological activity of each fusion partner. Methods to measure the activities of the chimera may be modified for those used to measure chondroitinase activity including a spectrophotometric assay, zymography, an HPLC assay to detect CSPG disaccharide digestion products and an in vito neurite outgrowth assay. A neuron growth cone collapse assay can be used to evaluate NOGO receptor antagonists and a neurite outgrowth assay can be used measure LI activity. GGF2 activity may be measured using a Schwann cell proliferation assay.

The compositions of the present invention can be used for the treatment of spinal cord injuries and in the promotion of regeneration of axons. The compositions of the present invention can also be used to promote plasticity, regrowth, repair, and/or regeneration of dysfunctional neurons in the CNS that have been damaged as a result of disease, such as degenerative diseases including Alzheimer's and Parkinson's disease. Advantageously, the use of proteoglycan degrading polypeptides and membrane transducing polypeptides in the compositions of the present invention also promote diffusion and access of damage or diseased tissue to other therapeutic agents promoting the regeneration of neurons.

### DESCRIPTION OF THE DRAWINGS

The file of this patent contains at least one drawing/photograph executed in color. Copies of this patent with color drawing (s) /photograph (s) will be provided by the Office upon request and payment of the necessary fee.

In part, other aspects, features, benefits and advantages of the embodiments of the present invention will be apparent with regard to the following description, appended claims and accompanying drawings where:

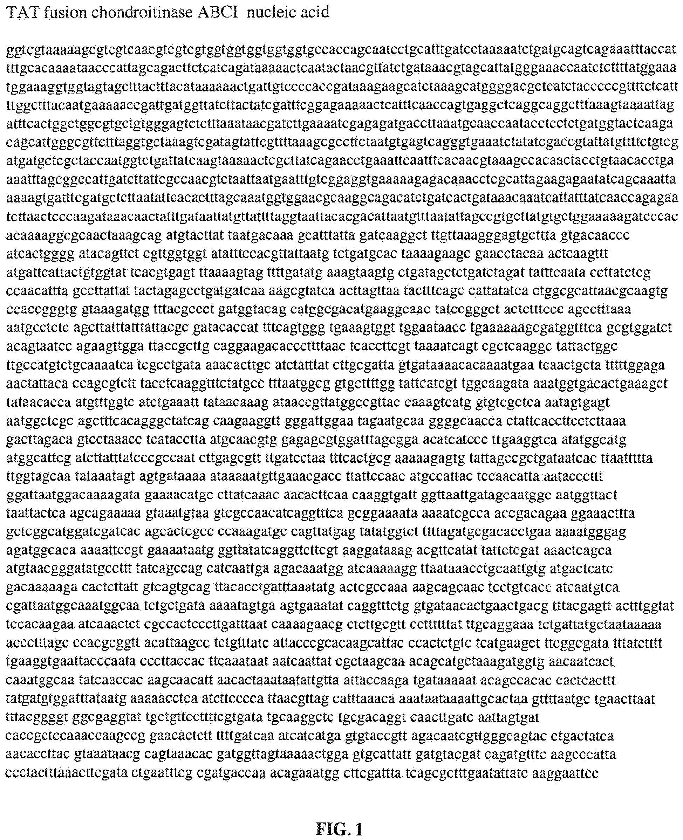

FIG. 1 is an illustrative example of a TAT-chondroitinase ABCI construct; the DNA sequence for the entire gene fragment fused with the Tat sequence followed by the 5 glycine linker ; the sense and antisense oligonucleotides having the sequences as 5'-tatgtatggtc gtaaaaagcgtc gtcaacgtcgtcgtgg tggtggtggtca-3'and 5'-tatgaccaccaccaccaccacgac gacgttgacgac gcttttt acgaccataca-3'were annealed and ligated at the NdeI site shich is at the 5'end fo ABCI gene cloned in pET15b (Novagen) at the NdeI and BamHI sites. The Tat48 s7 peptide sequence (GRKKRRQRR) is joined with the ABCI sequence by a pentaglycine linker.

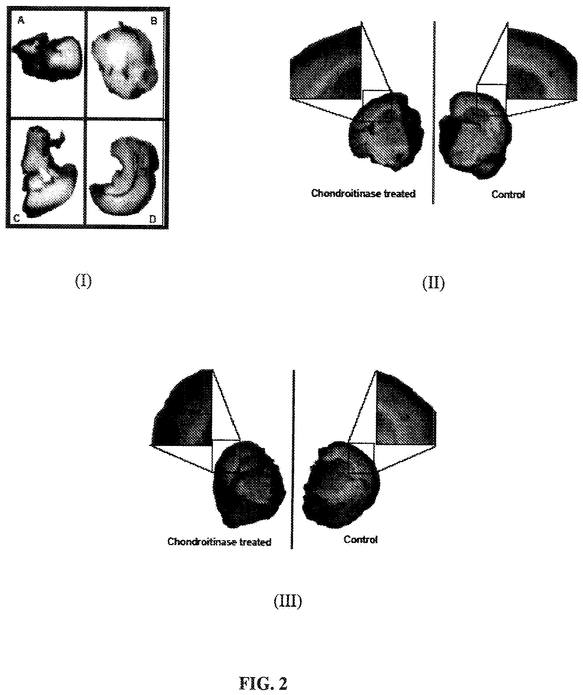

FIG. 2 are Brain section images; (I) illustrating lobes from adult rat which were incubated in beta-galactodidase alone (B & D), or with the addition of Choindroitinase ABCI (A, 0.5 U/ml or C, 0.005 U/ml); (II) Eosin Y penetration through the cortex of a Choindroitinase treated brain hemisphere and control showing approximately the same penetration, Eosin Y is a zwitterionic, having an overall negative charge at the low pH it was used at, and it is 692 kDa; (III)A saturate solution of Congo Red demonstrates greater penetration through the cortex of a Chondroitinase treated brain hemisphere as compared to untreated brain, Congo red is a negatively charged dye of 697 kDa;

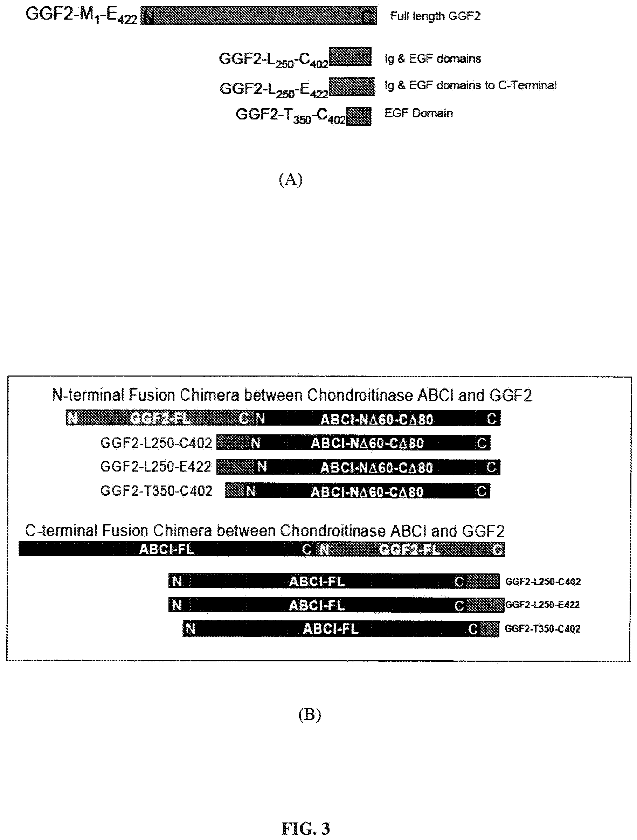

FIG. 3 (A) is a diagram representing the full length GGF protein (GGF2-M₁-E₄₂₂) and the three GGF2 fragments containing the Ig and EFG domains alone and in combination; (B) a schematic presentation of chimeric proteins of a proteoglycan degrading molecule like chondroitinase ABCI and a neuregulin 1 gene isoform GGF2;

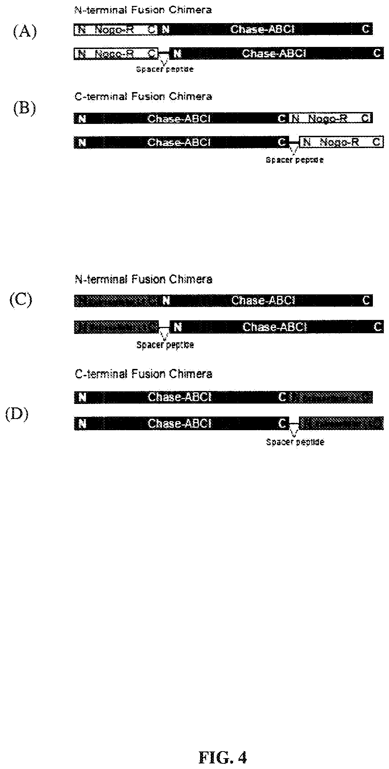

FIG. 4 illustrate the structure of (A) an NgR₂₇₋₃₁₁- N terminal chondroitinase ABCI chimeric protein with and without a peptide spacer or linking group; (B) an NgR₂₇₋₃₁₁- C terminal chondroitinase ABCI chimeric protein with and without a peptide spacer or linking group; (C) an extracellular domain L1 N-terminal chondroitinase ABCI chimeric protein with and without a spacer or linking peptide; (D) an extracellular domain L1 C-terminal chondroitinase ABCI chimeric protein with and without a spacer or linking peptide.

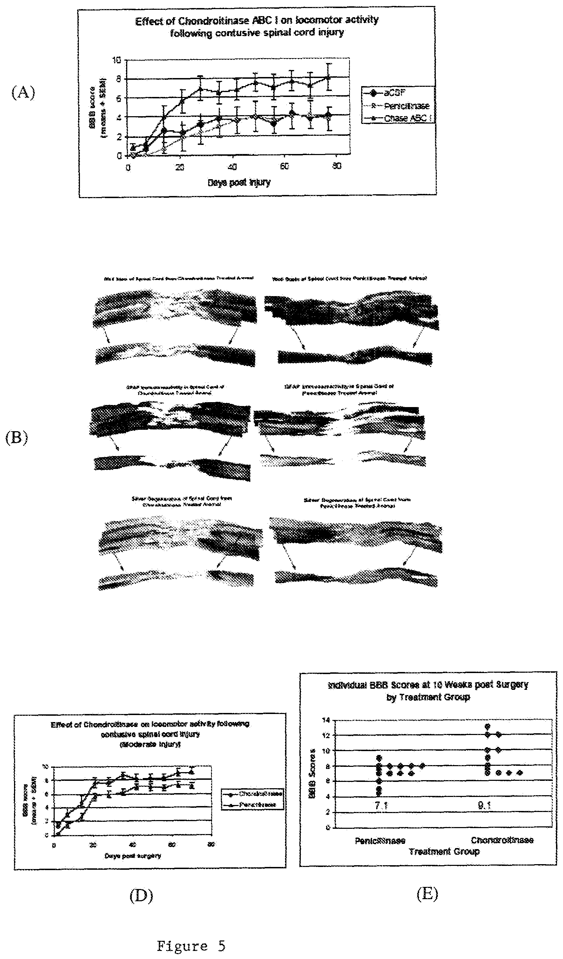

FIG. 5 (A)BBB scores from spinal cord injured animals treated with chondroitinase (Chondroitinase ABC I), penicillinase or artificial cerebrospinal fluid (aCSF); (B) Parasagittal sections of spinal cords from injured animals treated with chondroitinase (left column) or penicillinase (right column). Sections were cut at 30 microns. Each set of images contains four parasagittal sections realigned with the rostral cord to the left and the caudal cord t the right. The most central section of each set has been removed and replaced below to aid visualization. Pairs of images are Weil stained, anti-GFAP and an amino cupric stain of neuronal degeneration (top to bottom). (C) and (D) Distribution of individual BBB scores according to treatment group. Scores are BBB scores at ten weeks post surgery. Group averages are shown below the data points.

### DETAILED DESCRIPTION

Before the present compositions and methods are described, it is to be understood that this invention is not limited to the particular, compositions and associated molecules, methodologies or protocols described, as these may vary. It is also to be understood that the terminology used in the description is for the purpose of describing the particular versions or embodiments only, and is not intended to limit the scope of the present invention which will be limited only by the appended claims.

It must also be noted that as used herein and in the appended claims, the singular forms"a", "an", and"the"include plural reference unless the context clearly dictates otherwise. Thus, for example, reference to a"cell"is a reference to one or more cells and equivalents thereof known to those skilled in the art, and so forth. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described.

"Optional"or"optionally"means that the subsequently described event or circumstance may or may not occur, and that the description includes instances where the event occurs and instances where it does not.

Enzymes that cleave CSPGs include chondroitinase ABC type I, chondroitinase ABC Type II, chondroitinase AC and Chondroitinase B or mammalian enzymes with chondroitinase-like activity such as hyaluronidasel, hyaluronidase 2, hyaluronidase 3, hyaluronidase 4, cathepsins, ADAMTs and PH-20 or mixtures thereof.

CSPGs are a family of proteoglycans composed of a core protein and covalently linked sulfated glycosaminoglycans. The polysaccharide chains are cleaved by several enzymes, including a family of chondroitinases. To date, this enzyme family contains at least four members, Chondroitinase ABC I, ABC II, AC and B. Chondroitinase ABC I is an exo-lyase which cleaves both chondroitin and dermatan sulfates. It has been used extensively in the study of in vitro neuronal regeneration over CSPG-laden substrates and more recently in in vivo studies following CNS injury.

Proteins and polypeptide that may be used in the compositions and fusion proteins of the present invention are those which promote plasticity as well as the regeneration of injured or diseased neurons and axons. These regenerating proteins and polypeptides may include cell adhesion proteins, those which stimulate glial cells, and polypeptide which block the inhibitory effect of proteins that act as axon growth inhibitors.

Plasticity of the nervous system refers to any type of functional reorganization. This reorganization occurs with development, learning and memory and brain repair. The structural changes that occur with plasticity may include synapse formation, synapse removal, neurite sprouting and may even include strengthening or weakening existing synapses. Regeneration is generally differentiated from plasticity by the long range growth of axons in disrupted tracts that is characteristic of regeneration.

Proteins and polypeptides that are capable of blocking the activity of neuronal growth inhibitory molecules may include peptides and polypeptides that block the inhibitory properties of peptide such as but not limited Nogo, MAG, OMgp. Suitable compositions that over come the activity of neuronal growth inhibitory molecules include but are not limited to Protein Kinase C family inhibitors, Rho Kinase family inhibitors and agents, such as phosphodiesterase inhibitors, that increase intracellular cyclic AMP, and L1. The (NgR27311) peptide has been shown to inhibit binding of Nogo66, OMgp, MAG, and MOG to membrane-bound NogoR and overcome the inhibitory effects of Nogo on the regeneration of nerve processes.

Proteins and polypeptides that affect cell adhesion or stimulate cells may include but are not limited to poly peptides such as L1 and GGF2. L1 is a neural cell adhesion protein that is a potent stimulator of neurite outgrowth in vitro for which has been found that treatment of acute SCI in rodents using a soluble form of LI leads to an increase in the recovery of neurological function. GGF2 stimulates glial cells and has been shown to improve clinical outcome measures in a murine model of experimental allergic encephalomyelitis (EAE) likely as a result of the stimulation of oligodendrocytes to promote remyelination.

Cell membrane-permeant peptide sequences useful in the present invention are derivable from the human immunodeficiency virus type 1 (HIV-1) Tat protein (Fawell et al. , Proc. Natl. Acad. Sci. , 91: 664-68,1994). In particular, the Tat peptide can comprise any sequential residues of the Tat protein basic peptide motif 37-72 (Vives et al. 1997) (37-CFITKALGISYGRKKRRQRRRPPQGSQTHQVSLSKQ-72 (SEQ ID NO : 56)). The minimum number of amino acid residues can be in the range of from about three to about six, preferably from about three to about five, and most preferably about four, i. e. , the minimal requirement for one alpha helical turn. A preferred embodiment comprises Tat protein residues 48-57 (GRKKRRQRRR) (SEQ ID NO : 57).

Tat Proteins and Peptides Tat is an 86-amino acid protein involved in the replication of human immunodeficiency virus type 1 (HIV-1). The HIV-1 Tat transactivation protein is efficiently taken up by cells (Mann and Frankel 1991; Vives et al. 1994), and low concentrations (nM) are sufficient to transactivate a reporter gene expressed from the HIV-1 promoter (Mann and Frankel 1991). Exogenous Tat protein is able to translocate through the plasma membrane and reach the nucleus to transactivate the viral genome.

A region of the Tat protein centered on a cluster of basic amino acids is believed to be responsible for this translocation activity (Vives et al. 1997). Tat peptide-mediated cellular uptake and nuclear translocation have been demonstrated in several systems (Vives, et al., J Biol Chem 272:16010-16017, 1997; Jones, Genes Dev 11:2593-2599,1997). Chemically coupling a Tat-derived peptide (residues 37-72) to several proteins results in their internalization in several cell lines or tissues (Fawell, et al., Proc Natl Acad Sci USA 91:664-668, 1994; Anderson, et al., Biochem Biophys Res Commun 194:876-8884, 1993; Fahraeus, et al., Curr Biol 6:84-91, 1996; Nagahara, et al., Nat Med 4:1449-1452, 1998). A synthetic peptide consisting of the Tat basic amino acids 48-60 with a cysteine residue at the C-terminus coupled to fluorescein maleimide translocates to the cell nucleus as determined by fluorescence microscopy (Vives et al. 1997). In addition, a fusion protein (Tat-NLS-.beta.-Gal) consisting of Tat amino acids 48-59 fused by their amino-terminus to .beta.-galactosidase amino acids 9-1023 translocates to the cell nucleus in an ATP-dependent, cytosolic factor-independent manner (Efthymiadis et al. 1998).

Chimeric proteins, also referred to in the art as fusion proteins, are hybrid proteins which combine at least parts of two or more precursor proteins or peptides. Chimeric proteins may be produced by recombinant technology, i.e. by fusing at least a part of the coding sequence of one gene to at least a part of the coding sequence of another gene. Where desirable, one or more genes for linker peptides may be fused to the coding sequence of genes for the other polypeptide domains in the fusion protein. The fused gene may then be used to transform a suitable organism such as but not limited to *E. coli* or CHO cells which then expresses the fusion protein.

Genes encoding either N or C terminal deletion mutants of polypeptide domains of the fusion proteins can be used in constructs for expression of the fusion proteins. Preferably the generated deletion mutants maintain their catalytic proteoglycan degrading activity, blocking activity, growth activity, or transduction activity. Generated deletion mutants of the proteoglycan degrading molecules like chondroitinase ABCI enzyme where the mutant is missing a certain number of amino acids from the N and or C-terminal are those that retain some proteoglycan degrading activity. N-terminal deletions of chondriotinase like chondroitinase ABC I maintain a histidine-tag that is attached to the N-terminus. It is expected that a TAT- deletion mutant chondroitinase ABC I fusion DNA construct can be expressed without removal of the TAT polypeptide during expression. For example Fuse TAT peptide at the N-terminus of either ABCI-NA20 or ABCI-NA60 deletion mutant. Fragments of polypeptides, such as those shown in FIG. 3 for GGF2, may be used in the construction of chimeric fusion proteins.

Catalytically active deletion mutants of chondroitinase ABCI can be prepared by deleting 20or 60 amino acids respectively from the N-terminus of the mature ABCI protein. These deletion mutants may used for construction of N-terminal fusion chimeric protein. Detailed comparative biochemical studies can be done to determine the efficacy of mature chondroitinase ABCI versus various deletion mutant in compositions and fusion proteins with respect to the substrate specificity, substrate binding and tissue penetration.

A mutant of chondroitinase ABCI that has native protein structure, but lacks catalytic activity may be prepared as a null or a negative control for bioassays and SCI studies. Based on the crystal structure of chondroitinase ABCI a site-specific mutant designated H501a and Y508a to knock out catalytic activity in the putative active site can be prepared. Such mutants can be tested for inactivation of catalytic activity and SEC to compare to the wild-type enzyme. If the null activity mutant is successfully created it will provide a negative control for the various fusion proteins for use in bioassays and ultimately in SCI animal studies.

An E. Coli expression system may be to make chondroitinase using PET expression vectors (Novagen). The GGF2-chondroitinase ABCI fusion protein may be expressed in E. coli. Constructs for Tat-chondroitinase deletion mutant fusion proteins, Tat-GGF2 fusion proteins, or Tat-chondroitinase-GGF2 fusion proteins may be expressed from E. coli. Other fusion proteins can be expressed in CHO cell lines.

Table 1 illustrates various non-limiting components which may be used in compositions described herein as a fusion or chimeric molecule. The composition or chimeric molecule described may include one or more of the molecules in Table 1 and all features of claim 1. In the case of chimeric molecules one or more linker segments, preferably polypeptides are used.

**Table 1 Components of Compositions**

| Transduction molecules | Proteoglycan degrading molecules | Therapeutic, diagnostic, receptor antagonist molecules |
|---|---|---|
| Tat protein residues 48-57 HIV-1 Rev protein basic motif | Any agent that degrades extracellular matrix glycoproteins including: | Any peptide that blocks the inhibitory properties of Nogo, |
| HIV-1 Rev protein basic motif | Chrondroitinase ABC I, | MAG, OMgp including: |
| | Chondroitinase ABC II, | Nogo peptide 1-40* |
| Herpes simplex virus VP22 protein | Chondroitinase AC, | Any component of Nogo |
| | Chondroitinase B, | peptide 1-40 that maintains |
| | Hyaluronidase | the ability to block the |
| | 1, Hyaluronidase 2, | inhibitory properties of |
| | Hyaluronidase 3, | Nogo |
| | Hyaluronidase 4, PH20 | Other blocking peptides of |
| | deletion and or substitution | Nogo |
| | mutants of the above listed molecules that maintains | Antibodies that recognize Nogo |
| | enzymatic activity. | Blocking peptides of MAG |
| | | Antibodies that recognize MAG |
| | | Blocking peptides of OMgp |
| | | Antibodies that recognize OMgp |
| | | Antibodies that recognize the |
| | | Nogo-66 receptor Blocking peptides of the P75 receptor |
| | | Antibodies that recognize the p75 neurotrophin receptor Peptides or antibodies that block other receptors of Nogo, |
| | | MAG and/or OMgp |
| | | Peptide that overcomes the inhibitory properties of Myeline, Nogo, MAG, OMgp including: |
| | | Protein Kinase C family inhibitors* |
| | | Rho Kinase family inhibitors Agents that increase intracellular cAMP concentration L1 |

| | | |
|---|---|---|
| *Nogo peptide 1-40: The human 40-aa NEP1-40 aa (Acetyl-RIY KGV IQA IQK SDE GHP FRA YLE SEV AIS EEL VQK YSN S-amide corresponding to 1-40 aa of Nogo-66 | | |

A schematic illustration of chimeric fusion proteins are illustrated in FIG. 4A and FIG. 4B.

A peptide component from any column in Table 1 could be linked by an oligopeptide linker well known in the art such as a glycine rich peptide, for example Gly-Gly-Gly-Gly-Gly, or linkers prepared including an of the naturally occurring amino acids as well as substituted or beta or gamma amino acids like 4-aminobutyric acid or 6-aminocaproic acid can be used. Other linkers including but not limited to alkyl diamines, amino, or alkyl diols may also be used. Preferably the Transduction component of the fusion protein is in a terminal position in the chimeric protein. Other examples of common linkers may include but would not be limited to Gly-Gly-Ala-Gly-Gly, Gly/Ser rich linkers (for example Gly4Ser3), or Gly/Ala rich linkers. Additionally, linkers may be of any length and design to promote or restrict the mobility of components in the fusion protein.

The incorporation of non-natural amino acids, including synthetic non-native amino acids, substituted amino acids, or one or more D-amino acids into the peptides (or other components of the complexes) described herein (subsequently referred to herein as "D-peptides") is advantageous in a number of different ways. D-amino acid-containing peptides exhibit increased stability in vitro or in vivo compared to L-amino acid-containing counterparts. Thus, the construction of peptides incorporating D-amino acids can be particularly useful when greater intracellular stability is desired or required. More specifically, D-peptides are resistant to endogenous peptidases and proteases, thereby providing better oral transepithelial and transdermal delivery of linked drugs and conjugates, improved bioavailability of membrane-permeant complexes, and prolonged intravascular and interstitial lifetimes when such properties are desirable. The use of D-peptides can also enhance transdermal and oral transepithelial delivery of linked drugs and other cargo molecules.

In a spinal cord injury, the axons of ascending sensory and descending motor neurons are disrupted, that can result in the loss of sensation and paralysis. These axons fail to regenerate successfully leading to permanent disability. A scar envelopes the site of the injury which is believed to wall off the area of fragile tissue, stabilize the blood brain barrier, and prevent an overwhelming cascade of uncontrolled tissue damage. This scar is composed of hypertrophic glial cells and an extracellular matrix (ECM). Chondroitin sulfate proteoglycans (CSPGs) are one important component of the scar. They are expressed by glial cells and deposited in the ECM in regions of blood brain barrier breakdown. *In vitro* evidence demonstrates that these CSPGs are potently inhibitory for the growth of axons and without wishing to be bound by theory, are believed to contribute to the failure of the spinal cord axons to regenerate and reform functional synapses. *In vivo* studies have demonstrated that regenerating axons are able to grow into and even beyond the scar.

CSPGs and white matter components are generally accepted as molecular barriers that neurons must overcome in order to regenerate and reestablish functional connections after injury. Transplanted adult sensory neurons placed distal to a forming scar can regenerate robustly even along degenerating white matter pathways, however, regeneration ceases abruptly as axons enter the proteoglycan containing glial scar. Treatment of CNS white matter pathways with chondroitinase enhances the ability of neurons to grow in these substrates.

Central nervous system tissues are tightly compacted with cells and have limited extracelluar space. The proteins and carbohydrates of the extracellular matrix provide charge and osmotic forces as well as specific and non-specific binding sites,which may prevent the penetration of therapeutic agents. The enzymatic cleavage of these matrix and cellular components may later or facilitate the access of compounds or cells through tissues. A proteoglycan degrading molecule like Chondroitinase ABC I that is an enzyme that digests chondroitin sulfate proteoglycans can be used to promote diffusion of therapeutic molecules into the CNS. Tat peptides to transport covalently linked biologically active cargo molecules into the cytoplasm and nuclei of cells. In the case of a fusion protein having a protein transduction polypeptide domain like the HIV tat protein, therapeutic molecules for axon regeneration may be delivered across the blood brain barrier.

Treatment of SCI model injuries, preferably contusion model injury, can be used to determine the degree of regeneration and functional recovery achieved by compositions and method described herein. The degree of functional recovery can be demonstrated by improved corticospinal tract conduction, improved tape removal, beam walking, grid walking and paw placement following chondroitinase treatment of a dorsal column lesion. Motor skill improvement as well as autonomic function: bowel, bladder, sensor and sexual function may also be used as measures of function improvement and related to molecular structure and components in the compositions of the present invention.

In addition to the ability of chondroitinase to enhance regeneration, chondroitinase digests components of the perineuronal network (PNN). The density of the PNN is variable within the CNS and is particularly dense in the somatosensory, auditory, visual cortices and the hippocampus. PNN has also been demonstrated to be dense around spinal motor neurons. The inventors have discovered the dense PNN within the dorsal horn of the cord. Digestion of the PNN can enhance plasticity within the hippocampus and visual cortex. Plasticity within intact systems in incomplete SCI, especially in the region of the central pattern generators or in the reticular core, may support the function of damaged or destroyed systems. Promotion of plasticity in these systems may be one mechanism other than or in addition to regeneration by which chondroitinase can improve function following CNS injury. Furthermore, regeneration and plasticity may work in concert to affect recovery following injury; indeed, the corticospinal tract has been shown to be critical for modulation of spinal cord plasticity.

Recovery of neurological function following contusion injury in the CNS or a disease state may be promoted by administering the fusion proteins to cells, a tissue, or a subject having damaged or diseased neurons whether the injury or disease is immediate or long-standing.

The fusion proteins herein are administered in an amount effective to degrade CSPGs and thereby promote the recovery of neurological function. Once the proteins or polypeptides in the compositions have been purified to the extent desired, they may be suspended or diluted in an appropriate physiological carrier or excipient for SCI treatment. In models of SCI, effective intrathecal doses in rats have been about 0.06 units on alternate days for 14 days. A dose for a 70 kilogram human may be about 17 Units. At about 100 Units/ milligram, this would equal about 170 micrograms. Doses of up to 20 Units appear safe in the rat. Compositions including a proteoglycan degrading molecule as part of a fusion protein diluted in a carrier or pharmaceutically acceptable excipient can be injected, generally at concentrations in the range of 1 ug to 500 mg/kg of host. Administering the agent can be by bolus injection, intravenous delivery, continuous infusion, sustained release from implants, or sustained release pharmaceuticals. Administration may be by injection, such as intramuscularly, peritoneally, subcutaneously, intravenously. Oral administration may include tablets or capsules, preferably the oral dosage is a sustained release formulation for once or twice daily administration. Percutneous administration can be once per day, and is preferably less than once per day administration. Administration to the human patient or other mammalian subject may be continued until a measurable improvement in autonomic or motor function in the patient is achieved.

The chondroitinase PTD fusion proteins can be administered with a suitable pharmaceutical carrier. The administration of the compositions of the present invention chimeric proteins can be topical, local or systemic. The chimeric fusion proteins may also be secreted by genetically cells, preferably a chimeric fusion protein can be secreted by genetically modified cells that are implanted, either free or in a capsule, at or near the site of CNS injury.

Once the compositions are administered, degradation. of CSPGs removes the inhibitory molecules that block neurite outgrowth, and allow the regeneration of neurites into the affected area. Chondroitinase ABCI is an exo and endo lyase that cleave both CS and DS. The removal of CS and DS from the glial scar permits the regeneration of neurite outgrowths into the injured area.

Mixtures of any of these fusion polypeptides may be used to provide a therapeutic treatment for CNS injuries and disorders which may include but not limited to contusion injury, traumatic brain injury, stroke, multiple sclerosis, brachial plexus injury, amblioplia, spinal cord injuries. Spinal cord injuries includes disease and traumatic injuries, such as the crushing of neurons brought about by an auto accident, fall, contusion, or bullet wound, as well as other injuries. Practice of the present methods can confer clinical benefits to the treated mammal, providing clinically relevant improvements in at least one of the subject's motor coordination functions and sensory perception. Clinically relevant improvements can range from a detectable improvement to a complete restoration of an impaired or lost function of the CNS.

The regeneration of the nerve cells in to the affected CNS area allows the return of motor and sensory function. Clinically relevant improvement will range from a detectable improvement to a complete restoration of an impaired or lost nervous function, varying with the individual patients and injuries.

A variant of a protein or fragments thereof refer to a molecule substantially similar to either the entire protein or a fragment, which possesses biological activity that is substantially similar to a biological activity of the complement protein or fragments. A molecule is substantially similar to another molecule if both molecules have substantially similar structures or if both molecules possess a similar biological activity.

Variants of complement protein or fragments thereof are produced by chemical or recombinant means. Variants of the polynucleotides constructed to express fusion proteins may also be made. The variants may include, for example, deletions from, or insertions or substitutions of, amino acid residues within the amino acid sequence, or deletion, substitution, or insertion of nucleic acids from a sequence encoding for a particular fusion protein or polypeptide domain in the fusion protein. For example, in some cases the removal of one or more amino acid residues from a chondroitinase polypeptide can be made without significant change in its CSPG degradation activity. Substantial changes in functional properties like proteoglycan degradation or blocking activity against axon growth inhibitors are made by selecting substitutions that are less conservative, ie. that differ more significantly in their effect on maintaining the structure, charge or hydrophobicity of the peptide backbone in the area of the substitution.

Most deletions, insertions, and substitutions are not expected to produce radical changes in the characteristics of the protein molecule; however, when it is difficult to predict the exact effect of the substitution, deletion, or insertion in advance of doing so, one skilled in the art will appreciate that the effect will be evaluated by routine screening assays. For example, a change in the axon regeneration character of the polypeptide molecule, through more or less proteoglycan degradation can be measured with functional recovery tests as well as HPLC assays to detect CSPG disaccharide digestion products.

The use of a proteoglycan degrading molecule and an HIV tat protein, or a tat-derived polypeptide, as a fusion protein can deliver a molecule of interest into the CNS or a site in the CNS where neuronal tissue damage has occurred by disease or trauma. In particular the damage site in the CNS is where scaring has occurred as a result of a contusion injury. The molecule of interest, optionally referred to as an agent or cargo molecule can be a therapeutic molecule which promotesplasticity, axon growth, a diagnostic molecule, or a proteoglycan degrading molecule. The fusion protein having a protein transduction HIV tat protein permits, therapeutic molecules for axon regeneration to be delivered across the blood brain barrier or proteoglycan degrading molecule to be delivered into cells to degrade cellular stores of proteoclycans and promote axon regeneration.

Transport polypeptides of this invention may be advantageously attached to cargo molecules by chemical cross-linking or by genetic fusion. A unique terminal cysteine residue is a preferred means of chemical cross-linking. According to some preferred embodiments of this invention, the carboxy terminus of the transport moiety is genetically fused to the amino terminus of the cargo moiety. Embodiment of the present invention consists of an amino- terminal methionine followed by tat residues 47-58, followed by a chondroitinase polypeptide.

It will be appreciated that the entire 86 amino acids which make up the tat protein may not be required for the uptake activity of tat. For example, a protein fragment or a peptide which has fewer than the 86 amino acids, **but which exhibits uptake into cells and or can cross the blood brain barrier,** can be used (a functionally effective fragment or portion of tat). For example, tat protein containing residues 1-72 can be sufficient for uptake activity and tat residues 1-67 can mediate the entry of a heterologous protein into cells. Synthetic peptide containing tat residues 1-58 can have uptake activity.

The tat peptide can be a single (i.e., continuous) amino acid sequence present in tat protein or it can be two or more amino acid sequences which are present in tat protein, but in the naturally-occurring protein are separated by other amino acid sequences. As used herein, tat protein includes a naturally-occurring amino acid sequence which is the same as that of naturally-occurring tat protein, its functional equivalent or functionally equivalent fragments thereof (peptides). Such functional equivalents or functionally equivalent fragments can possess uptake activity into the cell or across the blood brain barrier that is substantially similar to that of naturally-occurring tat protein. Tat protein can be obtained from naturally-occurring sources or can be produced using genetic engineering techniques or chemical synthesis.

The amino acid sequence of naturally-occurring HIV tat protein can be modified, by addition, deletion and/or substitution of at least one amino acid present in the naturally-occurring tat protein, to produce modified tat protein (also referred to herein as tat protein or polypeptide). Modified tat protein or tat peptide analogs with increased stability can thus be produced using known techniques. Therefore, tat proteins or peptides may have amino acid sequences which are substantially similar, although not identical, to that of naturally-occurring tat protein or portions thereof. In addition, cholesterol or other lipid derivatives can be added to tat protein to produce a modified tat having increased membrane solubility.

Naturally-occurring HTV-1 tat protein has a region (amino acids 22-37) wherein 7 out of 16 amino acids are cysteine. Those cysteine residues are capable of forming disulfide bonds with each other, with cysteine residues in the cysteine-rich region of other tat protein molecules and with cysteine residues in a cargo protein or the cargo moiety of a conjugate. Such disulfide bond formation can cause loss of the cargo's biological activity. Furthermore, even if there is no potential for disulfide bonding to the cargo moiety (for example, when the cargo protein has no cysteine residues), disulfide bond formation between transport polypeptides can lead to aggregation and insolubility of the transport polypeptide, the transport polypeptide-cargo conjugate, or both. The tat cysteine-rich region may be deleted to avoid disulfide bond formation and prevent aggregation and insolubility of the transport polypeptide, the transport polypeptide-cargo conjugate, or both.

Chondroitinase is also able to promote plasticity in regions of the CNS with significantly dense PNN, including cortex, tectum, hippocampus and spinal cord. It is reasonable that some combination of effects, including regeneration, sprouting and plasticity are responsible for the improvement in function following SCI with chondroitinase treatment or treatment with fusion molecules including chondroitinase or other proteoglycan degrading molecule.

NbR₂₇₋₃₁₁: Nogo is a high molecular weight myelin component that inhibits neurite outgrowth. The amino terminal region (Nogo66) is the part of the molecule that is specifically associated with the inhibition of neurite outgrowth. Expression cloning methods revealed the receptor for Nogo66 (NgR) is a GPI anchored glycoprotein expressed mainly on neurons. NgR interacts not only with Nogo, but also with other myelin associated inhibitors such as MAG and MOG. Due to its central role in the inhibitory properties of myelin on neurons, NgR has been the target for approaches to antagonize its interactions with its ligands. The soluble segment of NgR interacts with Nogo66, MAG and MOG. The region spans residues 27-311 and this NgR fragment can therefore act as a decoy receptor that will interfere with myelin associated inhibition of neurons. If NgR₂₇₋₃₁₁ is linked to proteoglycan degrading molecule like chondroitinase ABCI to form a chimeric fusion protein, the NgR₂₇₋₃₁₁ polypeptide domain of the fusion protein would be expect to limit the myelin-associated inhibition of neurite outgrowth and promote axonal regeneration in chondroitinase digested regions of a spinal cord injury.

For cloned (NgR₂₇₋₃₁₁), the precise region of interest, or fragments can be derived from the initial clone. The biological activity of NgR₂₇₋₃₁₁, its fragments, and fusion polypeptides including NgR₂₇₋₃₁₁ may be confirmed using an in vitro assay for the collapse of growth cones on neurons. The growth cone collapse assay has been established and the addition of MAG or Nogo66 causes the collapse of growth cones in a dose dependent manner. If NgR₂₇₋₃₁₁, its fragments, and fusion polypeptides including NgR₂₇₋₃₁₁ are active is biologically active then they should inhibit the MAG and Nogo66 mediated growth cone collapse. Growth cone collapse data may be collected by the inspection of photomicrographs of DRG neurons in response to Nogo66 and MAG.

The L1 polypeptide is a member of the immunoglobulin superfamily of cell adhesion molecules and is expressed in growing axons, glial progenitor cells and Schwann cells throughout life, but has only limited expression in the CNS. L1 interacts with itself and with other extracellular molecules, such as the FGF receptor to promote neurite fasciculation and growth. Expression of L1 is generally associated with a permissive environment for axonal regeneration the, precise region of interest, or fragments of L1 can be derived from the initial clone. For example, Schwann cells that express L1 support peripheral nerve regeneration and axonal growth is observed in the optic nerves from transgenic mice that express L1 in astrocytes but not in wild-type optic nerves. Fibroblasts engineered to express L1 support axonal growth when transplanted into spinal cord. Finally, a soluble form of L1 linked to Fc promoted functional recovery following acute SCL If L1 is linked to proteoglycan degrading molecule like chondroitinase ABCI in a fusion protein it is reasonable to expect the fusion protein to promote axonal regeneration in chondroitinase digested regions of a spinal chord injury.

The neuregulins and their receptors comprise a diverse growth factor and receptor tyrosine kinase system that has been demonstrated to be essential for organogenesis in the CNS, muscle epithelial and other tissues. GGF2 is a soluble isoform of the neuregulin 1 gene. It was initially characterized as a Schwann cell mitogen 32, but subsequent studies have demonstrated direct actions on oligodendrocytes, neurons and other cell types. GGF2 diminishes demyelination and inflammation and enhances remyelination in a mouse model for multiple sclerosis. Based on these results, it is reasonable to expect that a recombinant human GGF2 is a potential treatment for demyelination associated with SCI. With GGF2 is linked to a proteoglycan degrading molecule like chondroitinase ABCI it is reasonable to expect to promote remyelination of axons in chondroitinase-digested regions of a SCL

**Table 2. Examples of non-limiting GGF2 fragments for expression in E. coli for use in compositions and fusion polypeptide compositions.**

| Construct | Domain | Nucleotide Seq | Amino Acid Seq | Mol. Wt. |
|---|---|---|---|---|
| GGF2-FL | Full length GGF2 | | M1-E422 | 46kDa |
| GGF2/1 | Ig domain +EGF domain | 748-1206 | L250-C402 | 16.8 kDa |
| GGF2/2 | Ig domain to C-terminus | 748-1266 | L250-E422 | 19 kDa |
| GGF2/3 | EGF domain | 1048-1206 | T350-C402 | 5.7 kDa |

The efficacy of a composition of the present invention can be evaluated using a validated rat SCI model at three different levels of SCI severity.

A proteoglycan degrading molecule like Chondroitinase ABCI is commercially available in small quantities as a naturally derived enzyme (Seikagaku Corporation) and can be made by a recombinant production system to have essentially the same activity as the enzyme purified from Proteus vulgaris. Genomic DNA can be isolated from Proteus vulgaris using DNeasy Tissue kit (Qiagen). PCR primers can be synthesized with an Ndel restriction site at the 5'end and a BamHI site at the 3'end having sequences 5'-CAT ATG GCC ACC AGC MT CCT GCA TTT G-3' (F2) and 5'-GGA TCC TCA AGG GAG TGG CGA GAG-3' (R) respectively, to synthesize the mature protein. The 3.0 kb PCR products can be ligated into pCR 2.1 vector (TOPO cloning kit, Invitrogen) and transformed into DH5a competent cells (Invitrogen). Plasmid DNA can be isolated from a number of clones screened by digestion with EcoRI restriction enzyme. The integrity of a gene prepared in this way can be confirmed by repeated DNA sequencing.

The chondroitinase ABCI sequence can cloned into a PET vector (Novogen) for expression in E. Coli After induction of gene expression with IPTG the bacteria can lysed by sonication with the concomitant extraction of chondroitinase ABCI with Triton X-114/PBS. The inventors discovered that the majority of recombinant chondroitinase ABCI was found in the cytosolic fraction of the bacterial cell lysate that enabled the develop a chondroitinase ABCI purification protocol that yields an enzyme with high activity at high yields. The protocol includes cation-exchange chromatography as a capture step and gel filtration as a polishing step. After these steps chondroitinase ABCI reaches a purity of ~95%. Anion exchange membrane filtration (Intercept Q, Millipore) can be used for endotoxin and host DNA removal. This step is expected to remove approximately 75% of the endotoxin. Following filtration, chondroitinase ABCI can be dialyzed into volatile buffer, pH 8.0 and lyophilized to dryness. The final product is stable at -70°C for long term storage. The purified cABCI is a highly basic protein with pI∼9.5 as determined by IEF-PAGE analysis of the samples from the crude cell lysate.

A variety of analytical methods can be used to compare the enzymatic activity of the recombinant version of chondroitinase ABCI to that of a commercially available form of the enzyme (Seikagaku Corporation) purified from *Proteus vulgaris.* The methods may be adapted to evaluate the activity of fusion proteins including proteoglycan degrading polypeptides like chondroitinase. Specific activity measurements were obtained using an accepted spectrophotometric assay that measures the change in absorbance due to the production of reaction products from the degradation of proteoglycans. The recombinant form of chondroitinase ABCI had approximately 25% higher specific activity than the Seikagaku chondroitinase ABCI. Size exclusion chromatography can be used to compare the hydrodynamic properties the enzymes. The elution profiles for recombinant enzyme was identical to that of the naturally derived enzyme.

A form of zymography can used to further characterize the enzyme and may be adapted for characterization of the fusion proteins. Polyacrylamide gels can be polymerized in the presence of aggrecan, a substrate for chondroitinase ABCI. Enzyme samples may be resolved on the aggrecan-impregnated gels by electrophoresis in the presence of SDS. The gels can then be subjected to a renaturation step wherein the SDS was extracted and the enzymes allowed to refold. Enzyme refolds and regains activity then digests aggrecan within the gel and the resulting loss of carbohydrate in that region of the gel can be visualized by a carbohydrate-specific stain. A similar loss of carbohydrate in the gel would be expected for active forms of a fusion protein including a proteoglycan degrading polypeptide portion. In the case of recombinant Chondroitinase ABCI, its activity can be visualized as a clear spot in the zymogram. The zymography results were consistent with the spectrophotometric analysis that demonstrates that the recombinant form of chondroitinase ABCI has the same or greater specific activity as the naturally occurring form.

HPLC methods may be used for detecting the four and six sulphated disaccharides (Δ4DS and A6DS, respectively) liberated as a result of chondroitinase ABCI digestion of CSPG. The two disaccharides can be effectively resolved by anion exchange chromatography. The HPLC assay has been validated by showing that the quantitation of Δ4DS and Δ6DS from chromatograms yields a linear relationship to the amounts injected into the HPLC. Production of Δ4DS and Δ6DS from CSPG digestion is directly related to the amount of chondroitinase specific activity as determined by the spectrophotometric assay described above. This assay may be used as a sensitive and accurate means to independently quantitate Δ4DS and Δ4DS released by chondroitinase digestion of a variety of substrates and may also be used to determine the activity of chondroitinase polypeptides in a fusion protein.

Another functional assay that can be performed to characterize proteoglycan polypeptide activity is where dorsal root ganglian (DRG) neurons are plated on aggrecan or aggrecan treated with a proteoglycan like chondroitinase ABCI. It is expected that neurons plated on aggrecan will failed to adhere to the plate and extend axons. In contrast, neurons plated on aggrecan treated with a proteoglycan degrading polypeptide like chondroitinase ABCI in a composition or as part of a fusion polypeptide would be expected to adhere to the surface and extend axons. The extensive axon growth, which is observed for chondroitinase ABCI is believed to be due to the digestion of the carbohydrates on the aggrecan core protein which creates a more permissive substrate for axon growth.

The rat contusion model of SCI is a clinically relevant model and may be used to evaluate the efficacy of fusion proteins and other compositions of the present invention for promoting axon regeneration. With a contusion SCI, cells are destroyed, hemorrhage ensues and inflammation begins. Destroyed cells are removed by macrophages and a reactive gliosis begins. A cystic cavity is formed and the gliosis matures into a glial scar. Myelin in the area is destroyed and many local neurons that are not destroyed are left in a demyelinated state.

The forceps compression model of SCI is a contusion model developed and characterized. This model has been validated and results in injuries that are very similar to the more widely used impactor models. The model can involves a forceps compression at vertebral level T9/T10. The forceps compress the cord to a width of 0.9,1.3 or 1.7 mm for 15 seconds. These three levels of compression allow a severe, mild or moderate injury. This model has been validated using the open field locomotor testing and the Basso, Bresnahan and Beattie (BBB) scoring system. It was also characterized histologically. Behavioral testing and BBB scoring demonstrated that the forceps produce a highly reproducible injury, with recovery similar to that seen with impactor models. These testing and scoring data demonstrate that the forceps compression model is comparable to other contusion models and sufficiently reproducible to be used as an experimental model of SCI and may be used for the evaluation of compositions of the present invention..

Tissue from forceps compression injured animals can be processed histologically to examine white matter sparing, glial scar and cyst formation. As with other contusion SCI models, a central cyst is formed after injury, with a size that increases with increased injury severity (decrease forceps gap). Around the cyst a glial scar forms that is characterized by astrogliosis (GFAP), macrophage activation and myelin wasting.

Various experimental procedures will be illustrated with reference to the following non-limiting examples.

### EXAMPLE 1

This example illustrates how a proteoglycan degrading molecule may be administered, studied, and demonstrated to show a functional improvement in animals having a model contusion injury.

The forceps compression model of SCI is a contusion model developed and characterized. This model has been validated and results in injuries that are very similar to the more widely used impactor models. The model can involves a forceps compression at vertebral level T9/T10. The forceps compress the cord to a width of 0.9, 1.3 or 1.7 mm for 15 seconds. These three levels of compression allow a severe, mild or moderate injury.

Rats were injured with the forceps compression model at vertebral T9/T10/At the time of surgery an intrathecal catheter was placed for delivery of chondroitinase. Animals were treated every day for one week and then on alternating days for one week with 0.06 U/dose chondroitinase ABC I (Seikagaku), penicillinase or artificial cerebrospinal fluid (aCSF). Doses and controls were derived from Bradbury et al. , 2002. Behavior was assessed using open-field locomotor testing and the BBB scoring system at day 2 and then weekly post injury for ten weeks.

FIG. 5A shown are mean BBB scores for animals treated with chondroitinase ABC I, penicillinase and aCSF. Rats treated with aCSF or penicillinase recovered to a mean BBB score of about 4. Rats treated with chondroitinase recovered to a mean BBB score of about 8. Multiple animals recovered to scores above 10, indicating supra-spinal input. The chondroitinase scores were significantly different from both control groups by ANOVA and post hoc Tukey.

Tissue from these animals was processed immunohistochemically for glial fibrillary acidic protein (GFAP) to asses general scar architecture. Tissue was also stained with a Weil stain and a silver degeneration stain to assess myelin and neuron degeneration, respectively. Interestingly no obvious differences in these parameters were noted between experimental and control treated tissues.

In FIG. 5B the large lesion comprising several segments and the sparing in the ventral cord. Weil staining revealed extensive demyelination in both the treated and untreated animals. The GFAP images (middle set) demonstrate the extent of the scar that is formed following forceps injury. The bottom amino cupric silver degeneration stain demonstrates the vast neural degeneration extending both rostrally and caudally after injury. Again, no obvious differences were noted between chondroitinase treated and control tissues.

Additional preliminary experiments have been performed at moderate injury levels and significant improvement in open-field locomotor activity was observed with chondroitinase treatment. Animals receiving chondroitinase ABCI recovered to a mean BBB score of 9.1 at ten weeks following injury, compared with 7.1 for the penicillinase controls. The consistency of the data (SEM's of 0.6 and 0.3, respectively) and the region of scores on the BBB scale make this 2 point change not only statistically significant, but also clinically meaningful. A score of 9 indicates plantar placement with weight support or frequent to consistent weight support with dorsal stepping while a score of 7 indicates movement of each joint in hind limb but with no weight support and no consistent sweeping of limbs. An examination of the individual animal scores at ten weeks shows that 6 of 12 animals in the chondroitinase group recovered to scores of 9 or above, while only one of 12 animals in the penicillinase group recovered to a score of 9. One animal from each group was removed from analysis because its score failed to ever rise above 2.5, indicating an injury severity outside the model norms. FIG. 5C and FIG. 5D includes a scatter plot of scores at 10 weeks for each animal in the penicillinase and chondroitinase treatment groups for the moderate injury. Group means are shown below.

The results show in this well controlled study, that chondroitinase improves open-field locomotor function in Rats that were injured with the forceps compression model at vertebral T9/T10. Animals recovered to mean BBB scores of 9.7 and 9.9 for the penicillinase and chondroitinase groups, respectively. This study demonstrates a significant effect at severe and moderate injury levels, but not mild injury levels. No significant differences were noted between any groups in the mild injury study (1.3 mm forceps). It is unclear if chondroitinase is not effective with mild injury, if chondroitinase effected changes not adequately assayed by the open-field locomotor testing such as stride length, paw placement, sensory or autonomic functions. Preliminary analysis of histology from animals in this study confirmed placement of the catheters and injuries in each animal. Ongoing experiments sacrificing animals at time points after injury with and without chondroitinase treatment will characterize the CSPG content of the scar and the effects of chondroitinase digestion on stub-antigen expression. Future experiments will expand the battery of behavior tests and examine the cord for evidence of regeneration with tract tracing.

Two acute toxicity studies were conducted in rats. The first was an intravenous (IV) study wherein rats were injected with 0,0.2,0.775 or 7.775 mg/kg chondroitinase ABCI. In the second study, intrathecal (IT) catheters were placed over the spinal cords using the same methods employed in the SCI animal studies and 0.06, 0.6 and 6.0 units of chondroitinase ABCI was delivered through the IT catheters. These doses were 1, 10, and 100-fold greater than the estimated local concentrations of chondroitinase ABCI achieved with IT catheters in the SCI experiments. Animals were monitored for pain and distress and body weights were acquired daily. No overt reactions were observed during or immediately after chondroitinase ABCI dosing. No swelling, inflammation, bruising or necrosis was noted at the injection site for the IV experiment. No changes in body temperature were observed in animals treated via the IT route. No alterations in feeding, grooming or vocalizations were noted. Animals were assessed for motor behavior in an open pool. No abnormalities were noted by the animal care staff or behavioral specialists. Animals displayed no signs of joint tenderness or swelling. There were no significant differences in weight change between the treatment groups. The results demonstrate that chondroitinase ABCI treatment is not associated with acute toxicity using IV and IT doses substantially greater than the efficacious IT doses.

### EXAMPLE 2

This example describes the preparation of Nogo-receptor agonist, L1 neural cell adhesion protein, and GGF2 polypeptide domains which can be used for compositions and fusion proteins described herein.

A soluble portion of the human Nogo receptor spanning amino acids 27 to 311 (NgR₂₇₋₃₁₁) was selected as it has been shown to inhibit the binding of Nogo66, MAG, and MOG to membrane-bound NgR. Primers were designed flanking this region, and RT-PCR was performed using human hippocampal RNA (BD Biosciences). The 1.05kb region was successfully amplified and purified.

L1 was prepared through a CHO cell line from the laboratory of Dr. Melitta Schachner that secretes human L1 as a fusion protein with human Fc (L1-Fc). The cells were grown in roller bottles and then L1-Fc was purified from the conditioned media using protein A affinity column chromatography. The purity of L1-Fc was assessed by SDS-PAGE and a single band at the appropriate molecular weight was observed. The biological activity of L1-Fc was confirmed using a neurite outgrowth assay. Tissue culture plates were coated with either poly L-Lysine or L1-Fc and then cerebellar granule cells from postnatal day 10 rats were isolated and placed into culture on the substrates. It was observed that neurons plated on the L1-Fc substrate exhibited a substantial number of long neuritis compared to the polylysine substrate controls. These results demonstrate that the L1-Fc produced is biologically active in promoting neurite outgrowth. This neurite outgrowth assay will be used to assess the biological activity related to the L1 portion of the chondroitinase ABCI fusion protein..

A CHO cell line that secretes a soluble form of full length GGF2 glycoprotein was obtained from CeNes. Extensive optimization of media and purification methods was completed to obtain essentially pure and biologically active GGF2. A number of analytical methods were developed to characterize the GGF2 including SDS-PAGE, isoelectric focusing, peptide mapping and carbohydrate analysis. For example an SDS-PAGE gel of reduced and non-reduced GGP2; the isolate is essentially free of contaminating proteins and shows the expected molecular weight and monomeric structure. The biological activity of GGF2 was assayed using a primary rat Schwann cell proliferation method and the expected effect was reproducibly obtained with four independent batches of GGF2. Another functional assay was developed that measures the phosphorylation of Akt kinase, a downstream cell signaling component of the erbB receptor pathway. It was observed that there is a dose dependent phosphorylation of Akt kinase

### EXAMPLE 3

This example illustrates the manipulation of chondroitinase ABCI for construction of chimeras.

Chondroitinase ABCI was cloned from *P.vulgans* and expressed in *E.coli.* Surprisingly repeated attempts to create N-terminal fusion proteins were not successful because the N-Terminal fusion portion was cleaved from chondroitinase ABCI during synthesis. However, catalytically active deletion mutants with N-terminal His fusion tags designated ABC I-NΔ2O, ABC I-NΔ4O and ABCI-NΔ6O were prepared by deleting 20,40 and 60 amino acids respectively from the N-terminus of the mature ABCI protein. Unlike the full length chondroitinase ABCI, the N-terminal deletion mutants are capable of synthesizing a 6xHis tag as an N-terminal fusion protein. It was also observed that deletion of 80 amino acids from the C-terminal end formed a mutant with proteoglycan degrading activity of chondroitinase ABCI as tested in a zymography assay.

Fusion proteins with NgR₂₇₋₃₁₁, or L1 with a proteoglycan degrading molecule such as chondroitinase ABCI will utilize mammalian expression and can be performed in CHO cells. The cDNA for chondroitinase ABCI has been cloned in pSECTag vector (Invitrogen) in the proper reading frame. A CHO cell line having secretary chondroitinase ABCI can be developed and the conditioned medium can be tested for catalytic activity by zymography assay to confirm that chondroitinase ABCI expressed in mammalian cells is functional. A mammalian cell codon optimized version of chondroitinase ABCI can be synthesized by methods known in the art for use for CHO cell expression.

GGF2 is a spliced variant of the NRG1 gene expressed in brain and spinal cord of adult humans. It is a glycosylated protein of molecular mass between 66-90 kDa. The inventors have discovered that recombinant GGF2 expressed in CHO cells is highly glycosylated and promotes Schwann cell proliferation in vitro and further that an EGF-like domain of NRG1 expressed in E.coli is fully functional in promoting myocyte proliferation and survival.

The inventors have expressed fragments of GGF2 in *E.coli* as described in the preliminary data section. The specific GGF2 domain responsible for Schwann cell proliferation and thereby remyelination can be determined. If the Ig and EGF domains together or separately show biological activity in vitro, then they can be used to form chimeric fusion proteins.

Cloning and expression of NgR₂₇₋₃₁₁ in CHO cells. An NgR fragment corresponding to residues 1-359 was isolated by RT-PCR from human hippocampus poly K RNA (BD Biosciences) and its structure can be confirmed by DNA sequencing. The gene fragment corresponding to residues 27-311 can be cloned from the larger fragment and then subcloned in pSECTag vector (Invitrogen) in the proper reading frame to express the fragment as a secretary protein in CHO cells. The plasmid DNA containing the NgR₂₇₋₃₁₁ gene can be transfected in CHO cells and the cell line producing NgR₂₇₋₃₁₁ can be selected under the selection pressure of hygromycine B. An NgR₂₇₋₃₁₁ -ABCI chimera expression plasmid can be constructed for expression in a CHO cell expression system using methods known in the art.

### EXAMPLE 4

This example illustrates methods which may be used to purify and isolate expressed chondroitinase ABCI, and GGF2 domains expressed in *E. coli.* These method may be applied to purification of chimeric fusion proteins of the present invention.

An efficient *E.coli* recombinant expression system and a purification process for chondroitinase ABCI have been developed by the inventors that could be applied for the purification of chondroitinase ABCI chimeric derivatives.

For example, the expression of various GGF2 domains in the chondroitinase ABCI expression host, *E.Coli.* has been preformed and the following peptides have been tested: aa250-402, aa250-422 and aa350-402. These expressed peptides were found in the soluble fraction of the *E.coli* lysates. It is reasonable to expect that final chimeric products of these peptides with chondroitinase ABCI in *E.coli* will also be soluble. In addition, it is expected that the charge characteristics of the chimeric products when compared to the recombinant chondroitinase ABCI will be similar. Thus, the theoretical isoelectric point (p1) values for the GGF2 peptides are 9.3 for aa250-402, 9.18 for aa250-422 and 7.55 for aa350-402. Fusing the first two peptides with chondroitinase ABCI is expected to result in chimeric proteins with p1 values still above 9. In this case SP chromatography is expected to perform well as the capturing step. The smallest GGF2 peptide, aa 350-402, will reduce the p1 of the final chondroitinase ABCI chimera to approximately 8.4. This change may require optimization of the capture step conditions.

The chimeric products of chondroitinase ABCI with NgR₂₇₋₃₁₁ and L1 peptides can be expressed in a CHO cell system. Prior to purification of the expressed chimeric proteins, the growth conditions for the cell line producing either the NgR₂₇₋₃₁₁ or L1 chimeric proteins will be optimized in serum-free medium. A detailed media optimization study can be performed to determine the highest production conditions. Scale-up volume can be decided based on the rate of production of the chimeric proteins (pg/cell/day). Conditioned media from the various chimera-producing cell lines can be collected and subjected to tangential flow filtration. Ion-exchange chromatography can be used for capturing the secreted proteins from conditioned media, gel filtration chromatography can be used as a polishing purification step and then anion-exchange membrane filtration can be used for endotoxin and DNA removal. At each step the efficiency of the purification will be analyzed by SDS-PAGE, and spectrophotometric quantitation of protein concentration.

### EXAMPLE 5

This prophetic example describes in vitro assessment of chimera biological activity: Each chimera can be assayed for chondroitinase enzymatic activity and the specific biological activity of each fusion partner.

The first step in analysis may employ conventional protein biochemical methodologies to confirm the fidelity of gene expression. These include SDSPAGE, 1EE, mass spectrometry and size exclusion chromatography.

Chondroitinase chimera specific activity can be determined using a standard and uniformly accepted spectrophotometric assay. The production of reaction products from the catalytic activity of a chondroitinase chimeric polypeptide can be determined by a measurement of the absorbance of the product at a wavelength of 232 nm. A typical reaction mixture consists of 120 microliters of reaction mixture (40mM Tris, pH 8.0, 40mM NaAcetate, 0.002% casein) combined with substrate (5 microliters of 50 mM chondroitin C) and 1.5 microliters of chondroitinase ABCI chimeric fusion polypeptide or test sample. The change in absorbance units is the initial rate which can be converted to a unit activity measurement.

Disaccharide HPLC assay: The catalytic activity of chondroitinase chimeric polypeptide on a CSPG substrate is expected to releases two species of sulphated disaccharides including α-Δ AUA-[1→3]-GaINAc-4S (Δ 4DS) and α-Δ AUA-[1→3]-GaINAc-6S (Δ 6DS). These species can be resolved by HPLC and the quantitation from the resulting chromatograms is a sensitive and accurate measure of chondroitinase activity. To perform the assay, samples from chondroitinase digestion reactions carried out with wild-type chondroitinase and chondroitinase fusion proteins can be clarified by centrifugation and then subjected to a validated anion exchange HPLC method as follows. A Dionex CarboPac PA-10 analytical column (4 x 250mm) fitted with a Dionex CarboPac PA-10 (4x 50mm) guard column can be used with a mobile phase consisting of a gradient of water at pH 3.5 (Buffer A) and 2M NaCl, at pH 3.5 (Buffer B). Detection may be set at a wavelength of 232 nm. A flow rate of 1 mL/minute and a 45 minute continuous gradient of 100% A to 100% B affords acceptable resolution of Δ4DS and Δ 6DS. Standard curves can be generated using known amounts of Δ4DS and Δ6DS.

Zymography allows resolution of proteins by molecular weight with a concomitant assessment of chondroitinase activity. A 10% polyacrylamide gel may be polymerized in the presence of 85pg/ml of aggrecan. Samples can be boiled in an SDS-loading buffer and then the analytes can be resolved by electrophoresis. After separation the gel can be incubated for 1 hour at room temperature in 2.5% Triton X100 then 16 hours at 37°C in fresh 2.5% Triton X-100. During these incubations, the SOS can be extracted from the gel and the chondroitinase refolds and digests the aggrecan in its immediate vicinity. After the refolding process the gel can be stained for carbohydrate: The gel can be first incubated in 0.2% Cetylpyridinium for 90 minutes at room temperature and then transferred into 0.2% Toludine Blue in 49:50:1 H20, Ethanol, and Acetic Acid for 30 minutes. The gel can then be fully destained. Following destaining the gel can be incubated overnight in a 50 microgram/ml solution of Stains-All (Sigma) in 50% ethanol. Chondroitinase activity can be detected as a clear spot in the gel that is coincident with the molecular weight of the enzyme. The size of the clearing has been shown to be almost linearly related to Unit activity.

The NgR₂₇₋₃₁₁-chondroitinase chimera can be assessed for the activity of the decoy Nogo receptor. The assay can be used to measures the collapse of growth cones: Dorsal root ganglia (DRG) are dissected from postnatal day 1 (P1) Sprague Dawley rat pups and dissociated in 200 U/ml collagenase I (Worthington) and 2.5 U/ml dispase (Boehringer/Roche) 2 times for 30 min. at 37°C. Enzymes are removed and DNAse (0.5mg/ml) can be added to the ganglia. Trituration may be done with a pipette tip attached to a 1000 µl Pipetman. The resulting cell suspension can be filtered through a 40 micron cell filter and centrifuged at 70xg for 5 min. Cells can be re-suspended in DMEM/10% FBS and pre-plated for 2 hours on a non-coated tissue culture plate (1 00mm diameter). Non-adherent neurons are removed and plated at 10,000 cells/well in a Poly-lysine/laminin-coated 24 well plate in serum-free Neurobasal/B27 with 50ng/ml NGF. After 20 to 24 hours, MAG or Nogo66 can be added at varying concentrations for 1 hour at 37°C to induce growth cone collapse. NgR₂₇₋₃₁₁-chondroitinase chimera can be added at various concentrations to compete with MAG and Nogo66 and thus protect the neurons from growth cone collapse. Cultures may be fixed by adding an equal volume of pre-warmed 8% paraformaldehyde/0.6 M sucrose to the medium for 20 min. while the cells are kept on a 37°C hot plate. Growth cones may be labeled with AlexaS68 phalloidin (Molecular Probes). Briefly, cells can be permeabilized with 0.1% Triton-X 100 for 5 min. at RT, blocked for 20min. in 1% BSA in PBS and incubated in phalloidin, diluted 1:40 in 1% BSA, in PBS for 20mm at RT. Cells can be washed in PBS and mounted in Fluorescent Mounting Medium (DAKO). The percentage of collapsed growth cones is determined by analyzing a minimum of 100 growth cones per well under a 40x objective.

The biological activity L1 fusion proteins may be determined using a standardized neurite outgrowth assay. After etching a 25mm circle in the center of a tissue culture-treated 35mm dish, 1 ml of 10 µg/ml poly-lysine can be added to each etched circle and incubated at 37°Cfor 60 minutes. The poly-lysine provides a negative control for neurite outgrowth. Etched circles may be washed 2 times with Hank's balanced salt solution plus calcium and magnesium (HBSS⁺⁺) after the last rinse; 1.2 µl of L1-Fc (0.6uM, 0.3µM, 0.15 µM, 0.075 µM) is spotted onto the petri dishes to serve as a positive control and various concentrations of the L1-chondroitinase fusion protein can be applied to the plates as test samples. The plates can be incubated for 1hour at room temperature. The spots are lightly aspirated, so as not to dry them out, and immediately washed 2 times with 1 ml HBSS⁺⁺ and then 1 ml of 1% BSA in PBS is added to each etched circle. After about 15 minutes at room temperature the etched circles can be washed 2 times with HBSS⁺⁺, and once with bioassay medium (NeuralBasal(Gibco) + B27 supplements (Gibco) + L-glutamine + L- glutamic acid, penicillin and streptomycin (100 U/ml) + 1% fetal bovine serum which will remain on the petri dishes until the time of the assay. To provide neurons to plate on the substrates Cerebellar granule cells from postnatal day (PND) 9 or 10 are harvested. The brain is removed from the cranium of 1 pup, and the cerebellum separated from the rest of the tissue. Meninges is removed, and the cerebellum is placed in ice cold HBSS⁺⁺. The tissue is minced and trypsinized using 0.25% trypsin for 15 minutes at 37°C. Trypsin action is inhibited by adding 0.5mg/ml soybean trypsin inhibitor (Gibco). The tissue is rinsed with HBSS⁺⁺ and triturated using a flame narrowed Pasteur pipette coated with EBS. Dissociated cells are pelleted at 500xg for 3 minutes, the supernatant is decanted and the cells are resuspended in 2ml of growth medium. After lightly triturating, the cell suspension is carefully layered on top of a 3.5%BSA (in PBS) cushion, and spun at 500xg for 3 minutes. Supernatant is aspirated and the pellet is resuspended in 2 ml growth media. A cell count is performed and the cells are diluted to a final working concentration of 1.5x10⁵ cell/ml. A 300 µl aliquot of diluted cells is added to the center of each plate, resulting in the even distribution of 6x10⁴ cells across the entire surface area of the etched circle. The cells are allowed to grow for 16 hours at 37°C in a humidified environment supplemented with 5% CO₂. The next day, plates are removed from the 37°C incubator, cells are fixed with 4% paraformaldehyde and the outgrowth of neurites is recorded by photomicroscopy.

Biological activity assays for GGF2 may be performed using Schwann cell proliferation. Sciatic nerves are dissected from three day old Sprague Dawley rat pups and dissociated in L-15 medium (Invitrogen) containing 0.25% trypsin and 0.03% collagenase type I (SIGMA) for 15 minutes at 37°C. Nerves are centrifuged at 400xg for 5 minutes and dissociation medium is replaced by DMEM/10% FBS. Nerves are triturated using a 10 ml syringe with a 21g needle and subsequently a 23g needle. The cell suspension is filtered through a 40 µm mesh placed over the opening of a 50ml conical tube. Cells can be centrifuged at 400xg for 5min, plated in a poly-D-Iysine (PDL)coated T-75 flask at a density of approximately 5 million cells in 15 ml of DMEM/10% FBS/PenStrep and incubated in a 37°C incubator regulated with 10% CO₂. After a 24 hour incubation the cells can be washed twice with DMEM/10% FBS and re-fed with fibroblast inhibition medium consisting of DMEM/10%FBS and 10 µl/ml of 1 mM cytosine arabinoside (Ara-C). After an incubation time of 2 to 3 days the fibroblast inhibition medium is replaced with Schwann cell growth medium (DMEM/10% FBS/ 150 ng/ml of GGF2/5 µM forskolin). Cells can be expanded and aliquots of 2x10⁶ cells/ml are frozen in DMEM/10%DMSO, 54% FBS in liquid nitrogen. At the time of use, Schwann cells are thawed and plated at a density of about 16,000 cells/well in a PDL-coated 96 well tissue culture plate in DMEM/5% FBS. After about 24 hours GGF2 is added in serial dilution ranging from 100 ng/ml to 0.78ng/ml containing 5 µM forskolin to establish a standard curve. Samples of GGF2 fusion proteins can also be added in serial dilution together with 5 µM forskolin. BrdU is added at 10 µM. Cells are incubated for 48 hours in a 10% CO₂ incubator. A BrdU ELISA kit from Roche Applied Science can be used (Cat. No. 1 647 229) to detect Schwann cell proliferation. Medium is poured out of the plate and plate is tapped on tissue paper. A 200 µl/well aliquot of Fix/Denat is added and incubated for 30 min at 15-25°C. A 100 µl/well aliquot of anti-BrdU-POD working solution (lyophilized antibody is dissolved in 1.1 ml double distilled water and diluted 1:100 with antibody dilution solution) can be added and incubated for 90 min at room temperature. Wells may be washed 3 times with 200-300 µl/well of washing solution. A 100 µl aliquot of substrate solution is added and incubated for 15 minutes or until color development is sufficient for photometric detection. The Plates are read on a SpectraMax plate reader at 450nm either before addition of stop solution at 370nm or after addition of 50 ml 1N sulfuric acid to each well.

Akt [pS473] ELISA: C6 glioma cells, obtained from ATCC, are grown in DMEM/10%FBS in a T-75 flask to confluence. After trypsinization, cells are plated in a 24 well plate at a density of 500,000 cells/well in 0.5 m1 of medium. One day after plating the cells are treated with GGF2 (batch: Glu from Lonza Biologics) at serial dilutions ranging from 0.78 to 100 ng/ml for 30 minutes at 37°C to establish a standard curve. As a negative control, wortmannin, a PI3-kinase inhibitor, is added to cells at 10 nnM for 30 minutes before the addition of GGF2. Samples of GGF2 fusion proteins will also be added in serial dilution. The cells are washed with PBS and then extracted with 100µl of cell extraction buffer (10 mM Tris, pH 7.4,100 mM NaCl,1 mM EDTA, 1 mM EGTA, 1 mM NaF, 20 mM Na₄P₂O₇, 2 mM Na₃VO₄, 1% Triton X-100, 109b glycerol, 0.1% SDS, 0.5% deoxycholate, 1 mM PMSF, protease inhibitor cocktail (Pierce)). Cell extracts are kept at -80°C until further use. An ELISA kit from Biosource (Cat. #KHOO1 II) can be used to measure phosphorylated Akt kinase levels. Briefly, 100 µl of a 1:50 dilution of samples and a serial dilution of Akt [pS473] standards are loaded into wells pre-coated with anti-Akt antibody for 2 h at room temperature (RT) or overnight at 4°C. Wells are washed and anti-Akt [pS473] antibody is added and incubated for 1 hour. After washing, HRP-conjugated anti-rabbit antiserum is added to wells and incubated for 30 min. After washing, TMB chromogen is added to wells and incubated for 30 min at RT before the reaction is stopped with stop solution. The plate is read on a SpectraMax plate reader at 450 nm. The optical densities are plotted against the Akt [pS473] standards and the concentration of pAkt in the C6 samples are deduced from the standard curve.

### EXAMPLE 6

This example illustrates construction of a fusion protein of chondroitinase polypeptide and a TAT cellular transduction peptide.

The gene sequence encoding a chondroitinase enzyme can be functionally linked to the protein transduction domain from HIV called the TAT Peptide. The resultant chimeric genes TAT-chondroitinase ABCI fusion DNA construct are shown in FIG. 1. It was observed that during bacterial expression of this construct, the TAT peptide was removed from the chondroitinase enzyme at some processing point during the bacterial growth. The removal of n-terminal linked peptides was also observed during expression of an n-terminal histidine-tagged chondroitinase ABCI enzyme.

Deletion mutants of the chondroitinase ABCI enzyme were generated where the mutant was missing a certain number of amino acids from the n-terminal portion of the polypeptide but maintained it proteoglycan degrading activity. It was observed that these n-terminal deletion maintained a histidine-tag that was attached to the n-terminus. It is expected that various TAT-deletion mutant chondroitinase ABCI fusion DNA construct can be expressed without removal of the TAT polypepdde during expression. For example, the TAT peptide may be fused at the N-terminus of deletion mutants like ABCI-NΔ20 or ABCI-NΔ60 deletion mutant. Without wishing to be bound by theory, the inventors believe that the native proteoglycan degrading enzyme contains a signal sequence that is attached to the n-terminus. This signal sequence is removed during the natural production in bacteria and in production of the cloned enzyme in E. coli. It is thought that some signal within the n-terminal amino acids instructs the bacteria to remove anything attached to this end.

A DNA construct with the TAT-peptide attached to the N-terminus of one of the chondroitinase deletion mutants can be made and Western blot and protein gel showing this expressed protein and activity.

### EXAMPLE 7

This example illustrates the diffusion of molecules into cells and tissue using a proteoglycan degrading composition.

A brain from an adult Sprague Dawley rat was removed from the skull and quartered into Right frontal, left frontal, right rear and left rear sections, corresponding roughly to the frontal (front) and occipito-parietal (rear) lobes. (A) Right frontal quarter was placed in artificial cerebrospinal fluid (Catalog # 59-7316; Harvard Apparatus, Holliston, MA) containing the beta-galactosidase enzyme (Catalog #, G 5160; Sigma, St. Louis, MO) and chondroitinase ABC I at 0.5 U/ml (Catalog# C 3667; Sigma, St. Louis, MO) for 2 hours at 37°C. Brain quarter was rinsed several times in phosphate buffered saline (PBS) and then processed with a Beta-Gal staining kit (Catalog # Gal-S; Sigma, St. Louis, MO). The substrate-enzyme reaction (blue product) was allowed to develop for 1 hour, and the brain was rinsed several times in PBS and slabs from the middle of each brain block cut using parallel straight razors. (B) Left frontal quarter was placed in artificial cerebrospinal fluid (Catalog # 59-7316; Harvard Apparatus, Holliston, MA) containing the beta-galactosidase enzyme (Catalog #, G 5160; Sigma, St. Louis, MO) for 2 hours at 37°C. Brain quarter was rinsed several times in phosphate buffered saline (PBS) and then processed with a Beta-Gal staining kit (Catalog # Gal-S; Sigma, St. Louis, MO). The substrate-enzyme reaction (blue product) was allowed to develop for 1 hour, and the brain was rinsed several times in PBS and slabs from the middle of each brain block cut using parallel straight razors. (C) Right frontal quarter was placed in artificial cerebrospinal fluid (Catalog # 59-7316; Harvard Apparatus, Holliston, MA) containing the beta-galactosidase enzyme (Catalog #, G 5160; Sigma, St. Louis, MO) and chondroitinase ABC I at 0.5 U/mi (Catalog# C 3667; Sigma, St. Louis, MO) for 2 hours at 37°C. Brain quarter was rinsed several times in phosphate buffered saline (PBS) and then processed with a Beta-Gal staining kit (Catalog # Gal-S; Sigma, St. Louis, MO). The substrate-enzyme reaction (blue product) was allowed to develop for 1 hour, and the brain was rinsed several times in PBS and slabs from the middle of each brain block cut using parallel straight razors. **(D)** Left rear quarter was placed in artificial cerebrospinal fluid (Catalog # 59-7316; Harvard Apparatus, Holliston, MA) containing the beta-galactosidase enzyme (Catalog #, G 5160; Sigma, St. Louis, MO) for 2 hours at 37°C. Brain quarter was rinsed several times in phosphate buffered saline (PBS) and then processed with a Beta-Gal staining kit (Catalog # Gal-S; Sigma, St. Louis, MO). The substrate-enzyme reaction (blue product) was allowed to develop for 1 hour, and the brain was rinsed several times in PBS and slabs from the middle of each brain block cut using parallel straight razors. Images of brains were acquired on a scanner and are shown in FIG. 2(I)(A-D).

Adult rat brain hemispheres were soaked in buffer alone or containing 33U/ml Chondroitinase ABCI (Acorda) for 2 hours at 37 degrees C. Hemispheres were rinsed and immediately placed in Eosin Y (Sigma) or a saturated solution of Congo Red (Sigma) in 70% ethanol. Slabs of tissue were cut and images were acquired on a scanner. See FIG. 2(II) Eosin and FIG. 2(III) Congo red.

FIG. 2(III) saturated solution of Congo Red demonstrates greater penetration through the cortex of a Chondroitinase treated brain hemisphere as compared to untreated brain. Congo Red is a negatively charged dye of 697 kDA. FIG. 2(II) Eosin Y penetration through the cortex of a Chondroitinase treated brain hemisphere looks slightly more diffuse, but penetration does not seem to be any deeper as compared to untreated brain. Eosin Y is zwitterionic, having an overall negative charged at the low pH it was used at, and it is 692 kDa. FIG. 2(I) Lobes from adult rat were incubated in beta-galactosidease alone (B&D), or with the addition of Chondroitinase ABCI (A, 0.5U/ml or C, 0.005U/m).

The results showed that chondroitinase treated tissue affected penetration of beta-galactosidase into CNS tissue. Chondroitinse had dramatic effects on the rate of Congo Red dye penetration but apparently not Eosin.

### EXAMPLE 8

This example illustrates a Chondroitinase ABCI Assay Protocol which may be modified to measure the activity of Chondroitinase ABCI deletion mutant-fusion proteins or the activity of other proteoglycan degrading polypeptide-fusion proteins described herein.

The production of reaction products from the catalytic activity of a proteoglycan degrading molecule or fusion protein is determined by a measurement of the absorbance of the product at a wavelength of 232 nm. A typical reaction mixture consisteo f 120 p, 1 of reaction mixture (40mM Tris, pH 8. 0,40mM NaAcetate, 0.002% casein) combined with a substrate (5 ; il of 50 mM chondroitin C) and 1.5 pLi of chondroitinase ABCI or a fusion protein of a deletion mutant of chondroitinase ABCI-TAT. Seikagaku's cABCI reference: frozen at-20 C in 5ml aliquots, use 1ml per reaction, 50 mM chondroitin C (MW 521) in water: frozen at-20 C in 100m1 aliquots. Reaction mixture aliquots of about 120 ul can be prepared at 37 C for 3 min or longer. A wavelength of 232 nm, is used with the spectrometer.

Knowing the extinction coefficient for the reaction product, measuring the change in the absorbance of the reaction product at 232 nm reading over time upon addition of a known amount of the Chondroitinase ABCI or other other proteoglycan degrading polypeptide- fusion proteins to the 120 ul reaction mixture with 0.002% casein and chondroitin substrate added, the specific activity in mol/min/mg of the proteoglycan degrading fusion protein can be determined. Seikagaku Chondroitinase ABCI has a specific activity under these assay conditions of about 450, umole/min/mg.
**SEQ ID NO: 1** Chondroitinase ABC I protein ORIGIN
**SEQ ID NO: 2** NΔ20 ABCI (A₄₅-N₁₀₂₃), protein
**SEQ ID NO: 3** NΔ60 ABCI (F₈₅-N₁₀₂₃) protein
**SEQ ID No. 4:** NΔ60 CΔ80 ABCI (F₈₅-A₉₄₂) protein
**SEQ ID NO: 5** Chondroitinase AC protein Locus 1HMW_A
   ORIGIN
**SEQ ID NO: 6** CΔ200 AC (Q₂₃-T₅₀₀) protein
**SEQ ID NO: 7 CΔ220** AC (Q₂₃-A₄₈₀) protein
**SEQ ID NO: 8** NΔ20 CΔ200 AC (L₄₃-T₅₀₀) protein
SEQ ID NO: 9 NΔ50 CΔ200 AC (T₇₄-T₅₀₀) protein
**SEQ ID NO: 10** NΔ100 CΔ200 AC (S₁₂₃₋T₅₀₀) protein
SEQ ID NO: 11 NΔ50 CΔ275 AC (T₇₄-L₄₂₆) protein
**SEQ ID NO: 12** Chondroitinase B Locus Q46079 protein
   ORIGIN
**SEQ ID NO: 13** NΔ80 Chase B (G₁₀₆-H₅₀₆) protein
**SEQ ID NO: 14** NΔ120 Chase B (I₁₄₆-H₅₀₆) protein
**SEQ ID NO: 15** CΔ19 Chase B (Q₂₆-L₄₈₈) protein
**SEQ ID NO: 16** CΔ120 Chase B (Q₂₆-K₃₉₀) protein
**SEQ ID NO: 17** NΔ120 CΔ120 Chase B (I₁₄₆-K₃₉₀) protein
**SEQ ID NO: 18** Choindroitinase AC nucleotide locus CHU27583
   ORIGIN //
**SEQ ID NO: 19** Chondriotinase AC nucleic acid deletion NΔ50 C Δ275 (a₂₂₀ - t₁₂₇₈)
**SEQ ID NO: 20** Chondroitinase B nuclei acid Locus CHU27584
   ORIGIN
**SEQ ID NO: 21** Chondriotinase B nucleic acid deletion NΔ120 CΔ120 (a₄₃₆ - g₁₁₇₀)
**SEQ ID NO: 22** Chondroitinase ABCI nucleic acid Locus I29953
   ORIGIN //
**SEQ ID NO: 23** TAT fusion chondroitinase ABCI nucleic acid
**SEQ ID NO: 24** An HIV TAT sequence and Gly penta linker protein
   G R K K R R Q R R R G G G G G
**SEQ ID NO: 25** Tat sequence and Gly penta linker nucleic acid
   ggt cgt aaa aag cgt cgt caa cgt cgt cgt ggt ggt ggt ggt ggt
**SEQ ID NO: 26 Present Invention Chondroitinase ABC II Nucleic acid**
**SEQ ID NO: 27 Present Invention Chondroitinase ABC II protein**
**SEQ ID NO: 28 Present Invention Chondroitinase ABC I nucleic acid**
**SEQ ID NO: 29 Present Invention Choindroitinase ABC I protein**
hyaluronidase 1 protein, **SEQ ID NO: 30**, Locus HSU96078
hyaluronidase 2 protein, **SEQ ID NO: 31**,
hyaluronidase 3 protein, **SEQ ID NO: 32**, Locus BC012892
hyaluronidase 4 protein, **SEQ ID NO: 33**, AF009010
PH-20 protein, **SEQ ID NO: 34**,
Tat peptide **SEQ ID NO: 35**
ABCI a site-specific mutant designated H501a and Y508a **SEQ ID NO: 36**
**SEQ ID NO: 37** Chondroitinase ABCI protein Locus P59807
   ORIGIN
**SEQ ID NO: 38** NΔ20 ABCI (A₄₅-N₁₀₂₃), protein
**SEQ ID NO: 39** NΔ60 ABCI (F₈₅-N₁₀₂₃) protein
**SEQ ID NO. 40:** NΔ60 CΔ80 ABCI (F₈₅-A₉₄₂) protein
SEQ ID NO. 41: I. Nucleotide sequence for TAT-ABCI-nΔ20:
**SEQ ID NO. 42: *II. Amino acid sequence for TAT-ABCI-nΔ20:***
**SEQ ID NO. 43: II. Nucleotide sequence for TAT-ABCI-nΔ60:**
**SEQ ID NO. 44: IV. Amino acid sequence for TAT-ABCI-nΔ60**
**SEQ ID NO. 45: V. Nucleotide sequence for ABCI-TAT-C:**
**SEQ ID NO. 46: V. Amino acid sequence for ABCI-TAT-C:**
SEQ ID NO. 47 Nucleotide sequence for chondroitinase ABCI-nΔ20
**SEQ ID NO. 48: II. Nucleotide sequence for ABCI-nΔ60:**
**SEQ ID NO. 49: Nucleotide sequence for TAT**
   ggtcgtaaaaagcgtcgtcaacgtcgtcgtcctcctcaatgc
(**SEQ ID NO. 50**) Amino acid sequence for a TAT peptide
   grkkrrqrrrppqc

## Claims

1. A composition comprising:
a polypeptide comprising a TAT protein transduction domain and a deletion mutant of chondroitinase ABC I, wherein the TAT protein transduction domain is attached to the N-terminus of the chondroitinase ABC 1 deletion mutant and the chondroitinase ABC I deletion mutant is selected from SEQ ID NO: 2 and SEQ ID NO: 3.

2. The composition of claim 1, wherein the protein transduction domain is a TAT domain represented by SEQ ID NO: 57.

3. The composition of claim 1, wherein the TAT domain and the chondroitinase ABC I deletion mutant are bonded together by a linker polypeptide domain.

4. The composition of claim 3, wherein the linker polypeptide domain comprises a glycine rich peptide.

5. The composition of claim 3, wherein the linker polypeptide domain comprises a sequence selected from the group consisting of Gly-Gly-Gly-Gly-Gly, Gly-Gly-Ala-Gly-Gly, Gly/Ser rich linkers, or Gly/Ala rich linkers.

6. The composition of claim 3, wherein the linker polypeptide domain comprises a non-naturally occurring amino acid, a substituted amino acid, a beta amino acid, or a gamma amino acid.

7. The composition of claim 1, wherein the polypeptide is selected from SEQ ID NO: 42, or SEQ ID NO: 44.

## Patentansprüche

1. Eine Zusammensetzung, die Folgendes beinhaltet:
ein Polypeptid, das eine TAT-Proteintransduktionsdomäne und eine Deletionsmutante von Chondroitinase ABC I beinhaltet, wobei die TAT-Proteintransduktionsdomäne an dem N-Terminus der Chondroitinase-ABC-I-Deletionsmutante angebracht ist und die Chondroitinase-ABC-I-Deletionsmutante aus SEQ ID NO: 2 und SEQ ID NO: 3 ausgewählt ist.

2. Zusammensetzung gemäß Anspruch 1, wobei die Proteintransduktionsdomäne eine TAT-Domäne ist, die durch SEQ ID NO: 57 dargestellt wird.

3. Zusammensetzung gemäß Anspruch 1, wobei die TAT-Domäne und die Chondroitinase-ABC-I-Deletionsmutante durch eine Linker-Polypeptiddomäne aneinander gebunden sind.

4. Zusammensetzung gemäß Anspruch 3, wobei die Linker-Polypeptiddomäne ein glycinreiches Peptid beinhaltet.

5. Zusammensetzung gemäß Anspruch 3, wobei die Linker-Polypeptiddomäne eine Sequenz beinhaltet, die aus der Gruppe, bestehend aus Gly-Gly-Gly-Gly-Gly, Gly-Gly-Ala-Gly-Gly, Gly/Ser-reichen Linkern oder Gly/Ala-reichen Linkern, ausgewählt ist.

6. Zusammensetzung gemäß Anspruch 3, wobei die Linker-Polypeptiddomäne eine nicht natürlich vorkommende Aminosäure, eine substituierte Aminosäure, eine beta-Aminosäure oder eine gamma-Aminosäure beinhaltet.

7. Zusammensetzung gemäß Anspruch 1, wobei das Polypeptid aus SEQ ID NO: 42 oder SEQ ID NO: 44 ausgewählt ist.

## Revendications

1. Une composition comprenant :
un polypeptide comprenant un domaine de transduction protéique de TAT et un mutant de délétion de chondroïtinase ABC I, le domaine de transduction protéique de TAT étant attaché à l'extrémité N-terminale du mutant de délétion de chondroïtinase ABC I et le mutant de délétion de chondroïtinase ABC I étant sélectionné parmi SEQ ID N° : 2 et SEQ ID N° : 3.

2. La composition de la revendication 1, dans laquelle le domaine de transduction protéique est un domaine de TAT représenté par SEQ ID N° : 57.

3. La composition de la revendication 1, dans laquelle le domaine de TAT et le mutant de délétion de chondroïtinase ABC I sont liés ensemble par un domaine polypeptidique lieur.

4. La composition de la revendication 3, dans laquelle le domaine polypeptidique lieur comprend un peptide riche en glycine.

5. La composition de la revendication 3, dans laquelle le domaine polypeptidique lieur comprend une séquence sélectionnée dans le groupe consistant en des lieurs riches en Gly-Gly-Gly-Gly-Gly, Gly-Gly-Ala-Gly-Gly, des lieurs riches en Gly/Ser ou des lieurs riches en Gly/Ala.

6. La composition de la revendication 3, dans laquelle le domaine polypeptidique lieur comprend un acide aminé d'origine non naturelle, un acide aminé substitué, un acide aminé bêta, ou un acide aminé gamma.

7. La composition de la revendication 1, dans laquelle le polypeptide est sélectionné parmi SEQ ID N° : 42, ou SEQ ID N° : 44.
